# NOUVEAU FASCICULE DE BREVET EUROPEEN

(11) **EP 1 902 077 B2**
(45) Date de publication et mention de la décision concernant l'opposition: **18.07.2018**
(45) Mention de la délivrance du brevet: 16.11.2011
(21) Numéro de dépôt: 06778737.4
(22) Date de dépôt: 30.06.2006
(51) Int. Cl.: C08F 26/00

(54) **POLYMERE CATIONIQUE NEUTRALISE, COMPOSITION LE COMPRENANT ET PROCEDE DE TRAITEMENT COSMETIQUE**
NEUTRALISIERTES KATIONISCHES POLYMER, ZUSAMMENSETZUNG MIT DEM BESAGTEN POLYMER UND KOSMETISCHES BEHANDLUNGSVERFAHREN
NEUTRALISED CATIONIC POLYMER, COMPOSITION CONTAINING SAID POLYMER AND A COSMETIC TREATMENT METHOD

(30) Priorité: 01.07.2005 FR 0552011; 07.07.2005 US 696789 P; 26.07.2005 FR 0507980
(43) Date de publication de la demande: 26.03.2008
(73) Titulaire: L'Oréal, 75008 Paris (FR)
(72) Inventeur: MOUGIN, Nathalie, F-75011 Paris (FR); JEGOU, Gwenaëlle, F-93190 Livry Gargan (FR)
(74) Mandataire: Kromer, Christophe
(86) Numéro de dépôt international: PCT/FR2006/001547
(87) Numéro de publication internationale: WO 2007/003784

(56) Documents cités:
- EP-A1- 1 031 342
- EP-A1- 1 180 527
- EP-A1- 1 440 680
- EP-A2- 1 110 536
- EP-A2- 1 319 389
- WO-A1-2005/004821
- WO-A1-2006/045510
- JP-A- 9 040 996
- US-A1- 2003 086 895
- US-A1- 2004 142 828
- US-B1- 6 300 381
- US-B1- 6 300 381
- KIM S J ET AL: "Electrical sensitive behavior of a polyelectrolyte complex composed of chitosan/hyaluronic acid" SOLID STATE IONICS, NORTH HOLLAND PUB. COMPANY. AMSTERDAM, NL, vol. 164, no. 3-4, novembre 2003 (2003-11), pages 199-204, XP004474007 ISSN: 0167-2738
- ANONYMOUS: "Structure of Chitin / Chitosan and Cellulose" DALWOO.COM, [Online] 12 avril 2002 (2002-04-12), XP002372029 Extrait de l'Internet: URL:http://www.dalwoo.com/chitosan/structu re.htm> [extrait le 2006-03-14]
- LARS KELD NIELSEN: "Metabolic engineering of hyaluronic acid production" BIOENGINEERING, [Online] 13 juin 2003 (2003-06-13), XP002372030 Extrait de l'Internet: URL:http://www.cheque.uq.edu.au/research/b ioengineering/research/Metabolic_Engineeri ng/HA.html> [extrait le 2006-03-14]
- Technical Information Luviflex® Soft
- HÖSSEL P. ET AL: 'VP/Methacrylamide/Vinyl Imidazole Copolymer: Ein neuer Standard für Haarstyling' SÖFW-JOURNAL vol. 129, no. 12, 2003, pages 65 - 70
- IP.com number IPCOM0C0097478D (2005)

## Description

La présente invention a trait à de nouveaux polymères, à leur utilisation notamment en cosmétique, ainsi qu'aux compositions les comprenant.

Il est connu d'employer des polymères dans le domaine cosmétique, et notamment en capillaire, par exemple pour apporter de la tenue ou du coiffant à la chevelure.
Dans le domaine des compositions capillaires dites "rincées", telles que les shampooings ou après-shampooings, les compositions de coloration ou de permanente, on utilise notamment des polymères cationiques fonctionnalisés, comprenant des fonctions amines, essentiellement tertiaire ou quaternaire, sous forme de chlorhydrate, d'acétate, ou d'alkylsulfonate, par exemple. Ces polymères cationiques, solubles dans l'eau, sont connus pour apporter une bonne cosmétique aux cheveux; toutefois, ils n'apportent généralement aucun effet de mise en forme des cheveux. En outre, les propriétés cosmétiques, telles que le toucher, le démêlage ou la douceur, peuvent être améliorées.
Il n'est pas connu de polymères apportant des propriétés coiffantes avec une corsmétique améliorée, en mode rincé.

Dans le domaine des compositions capillaires dites "non rincée", telles que les produits de coiffage de type laques, gels ou sprays coiffants, on est constamment à la recherche de polymères apportant des effets coiffants, et de la tenue à la chevelure, tout en ayant une cosmétique acceptable: démêlage et toucher.
D'une manière générale, on connaît dans les produits capillaires, des polymères à motifs amines, tels que les polymères à base de vinylpyrrolidone et de méthacrylate de diméthylaminoéthyle (polymères Gaffix). Les compositions obtenues présentent toutefois une tenue insuffisante dans le temps. On connaît également des polymères de type Luviquat, à base de vinylimidazole quaternisée et de vinylpyrrolidone, qui apportent de la douceur mais épaississent les compositions.
Dans WO200209656, il est décrit des compositions capillaires comprenant des polymères hydrophobes à base d'acrylate de butyle, apportant un effet coiffant repositionnable.
Dans EP1201223, on décrit des compositions capillaires comprenant des copolymères à base de (méth)acrylate d'alkyles permettant de coiffer et recoiffer la chevelure à volonté.
WO9830196 décrit des homopolymères acrylamides à amines quaternaires dont les contre-ions sont des groupements alkyle sulfate ou aryle sulfonate.
DE 2446449 décrit des sels de (co)polymères amines comportant des comonomères hydrophobes dont la reprise en eau est inférieure à 20%. Sont décrits des polymères (méth)acrylate à motifs amines neutralisés par l'acide acétique. JP09040996 décrit l'association d'un polymère cationique et d'un polymère anionique neutralisant sélectionné parmi les copolymères acrylate/acrylamide, vinyl acétate/crotonique acide, vinyl acétate/crotonique acide/vinyl néodécanoate. EP1038891 décrit l'utilisation d'un polymère comprenant des groupes amines et d'un neutralisant di ou polyvalent acide, ainsi que l'utilisation de polymère acide et de neutralisant amine.
Toutefois, dans l'ensemble des documents de l'art antérieur, les compositions cosmétiques comprenant des polymères cationiques permettent d'obtenir de bonnes propriétés cosmétiques, mais avec des effets coiffants insuffisants.

Le but de la présente invention est donc de pallier les inconvénients de l'art antérieur et de proposer des compositions cosmétiques comprenant des polymères cationiques, qui présentent un bon effet coiffant tout en conservant, voire en améliorant, leurs propriétés cosmétiques, notamment le toucher, la douceur, le volume et le démêlage, que cela soit en milieu sec (après séchage des cheveux), ou en milieu humide (avant séchage).
On a constaté qu'avec les compositions selon l'invention, l'effet de mise en forme et/ou de discipline du cheveu était particulièrement amélioré (gain en effet coiffant). Par ailleurs, les compositions selon l'invention possèdent un bon effet conditionneur.
Les compositions selon l'invention possèdent des propriétés cosmétiques intéressantes, par exemple lors de l'application dans une formulation de type shampooing; on a en effet constaté que les cheveux se démêlent facilement lors du shampooing, et qu'ils présentent de la douceur; après séchage, les compositions selon l'invention permettent également, une fois la chevelure séchée, une mise en forme des cheveux particulièrement intéressante.

Un objet de l'invention est donc une composition cosmétique ou pharmaceutique comprenant, dans un milieu physiologiquement acceptable, notamment cosmétiquement ou pharmaceutiquement acceptable, un polymère éthylénique cationique comprenant au moins une fonction amine primaire, secondaire ou tertiaire, protonable, ladite fonction étant au moins partiellement neutralisée par un agent neutralisant, caractérisée en ce que ledit agent neutralisant est un acide organique polymérique comprenant au moins une fonction acide carboxylique, sulfonique et/ou phosphonique; et est choisi parmi :
- les polymères comportant des unités répétitives de dextran comprenant au moins une fonction acide, notamment carboxylique, en bout de chaîne,
- les dendrimères ou molécules hyperbranchées fonctionnalisées en surface par des groupements acides, notamment carboxyliques;
- les polymères comprenant au moins trois unités répétitives de type alkylènes glycols pour lesquels le groupe alkyle comprend 2 à 4 atomes de carbone, et leurs combinaisons; étant entendu qu'ils comportent en outre au moins une fonction acide carboxylique, sulfonique et/ou phosphonique
choisis parmi les polymères de structures suivantes :

CH₃-O-(CH₂-CH₂-O-)n-co-(CH₂-CHR-O)m-CH₂-R₁₀

CH₃-O-(CH₂-CH₂-O-)n-b-(CH₂-CHR-O)m-CH₂-R₁₀

CH₃-O-(CH₂-CH₂-O-)n-co-(CH₂-CHR-O)m-CH₂-CH₂ R₁₀

CH₃-O-(CH₂-CH₂-O-)n-b-(CH₂-CHR-O)m-CH₂-CH₂ R₁₀

CH₃-O-(CH₂-CH₂-O-)n-co-(CH₂-CHR-O)m-CH₂-CH₂CH₂ R₁₀

CH₃-O-(CH₂-CH₂-O-)n-b-(CH₂-CHR-O)m-CH₂-CH₂CH₂ R₁₀

CH₃-O-(CH₂-CH₂-O-)n-CO-(CH₂-CHR-O)m-CH₂-CH₂OCO-CH₂-CH₂COOH CH₃-O-(CH₂-CH₂-O)n-b-(CH₂-CHR-O)m-CH₂-CH₂OCO-CH₂-CH₂COOH

R₁₀-(CH₂-CH₂-O-)n-co-(CH₂-CHR-O)m-CH₂-CH₂R₁₀

R₁₀-(CH₂-CH₂-O-)n-b-(CH₂-CHR-O)m-CH₂-CH₂ R₁₀

R₁₀-CH₂-O-(CH₂-CH₂-O-)n-co-(CH₂-CHR-O)m-CH₂-R₁₀

R₁₀-CH₂O-(CH₂-CH₂-O-)n-b-(CH₂-CHR-O)m-CH₂-R₁₀

R₁₀-CH₂-CH₂-CH₂-O-(CH₂-CH₂-O-)n-co-(CH₂-CHR-O)m-CH₂-CH₂-CH₂-R₁₀

R₁₀-CH₂-CH₂-CH₂-O-(CH₂-CH₂-O-)n-b-(CH₂-CHR-O)m-CH₂-CH₂-CH₂-R₁₀

R₁₀-CH₂-CH₂-COO-(CH₂-CH₂-O-)n-co-(CH₂-CHR-O)m-CH₂-CH₂-O-CO-CH₂-CH₂-R₁₀

R₁₀-CH₂-CH₂-COO-(CH₂-CH₂-O-)n-b-(CH₂-CHR-O)m-CH₂-CH₂-O-CO-CH₂-CH₂-R₁₀

R₁₀-CH₂-CH₂-CONH-(CH₂CH₂-O-)n-co-(CH₂-CHR-O)m-CH₂-CH₂-NH-CO-CH₂-CH₂-R₁₀

R₁₀-CH₂-CH₂-CONH-(CH₂-CH₂-O)n-b-(CH₂-CHR-O)m-CH₂-CH₂-NH-CO-CH₂-CH₂-R₁₀

dans lesquelles:
- le radical R, identique ou différent, représente -CH₃, -(CH₂)x-COOH, -(CH₂)x-SO3H; (CH₂)x-PO₃H₂H₂; avec x = 1 à 8;
- le radical R₁₀, identique ou différent, est -COOH, -SO₃H ou -PO₃H₂;
- n est un entier compris entre 3 et 1000;
- m est un entier compris entre 0 et 200.
étant donné que le ratio n:m est supérieur à 5, quand m est non nul.

Un autre objet de la présente invention est un polymère éthylénique cationique comprenant au moins une fonction amine primaire, secondaire ou tertiaire, protonable, ladite fonction étant au moins partiellement neutralisée par un agent neutralisant choisi parmi les acides organiques polymériques comprenant au moins une fonction acide carboxylique, sulfonique et/ou phosphonique, ledit polymère au moins partiellement neutralisé étant véhiculable en milieu aqueux, c'est-à-dire hydrosoluble ou hydrodispersible.

La présente invention a pour avantage de proposer des polymères généralement véhiculables dans l'eau, c'est-à-dire solubles ou dispersibles dans l'eau, ce qui permet de les employer de manière avantageuse dans des compostions cosmétiques, notamment de soin de la peau, ou capillaires, généralement à base aqueuse.
Par hydrosoluble ou soluble dans l'eau, on entend que le polymère forme une solution limpide dans l'eau, à raison d'au moins 5% en poids, à 25°C.
Par hydrodispersible ou dispersible dans l'eau, on entend que le polymère forme dans l'eau, à une concentration de 5 % en poids, à 25°C, une suspension ou dispersion stable de fines particules, généralement sphériques. La taille moyenne des particules constituant ladite dispersion est inférieure à 1 µm et, plus généralement, varie entre 5 et 400 nm, de préférence de 10 à 250 nm. Ces tailles de particules sont mesurées par diffusion de lumière.

Dans la suite de la présente description, on entendra par 'radical cyclique' un radical monocyclique ou polycyclique, qui peut se présenter lui-même sous forme d'un ou plusieurs cycles, saturés et/ou insaturés, éventuellement substitués (par exemple cyclohexyle, cyclodécyle, benzyle ou fluorényle), mais également un radical qui comprend un ou plusieurs desdits cycles (par exemple p-tertbutylcyclohexyle ou 4-hydroxybenzyle).
On entendra par 'radical saturé et/ou insaturé', les radicaux totalement saturés, les radicaux totalement insaturés, y compris aromatiques, ainsi que les radicaux comportant une ou plusieurs doubles et/ou triples liaisons, le reste des liaisons étant des liaisons simples.

Le polymère cationique selon l'invention est un polymère éthylénique comprenant au moins une fonction amine, qui peut être primaire, secondaire ou tertiaire, ladite fonction amine devant être protonable à un pH choisi entre pH 1 et pH 12.
Par "protonable", on entend que ladite fonction amine peut être neutralisée au moins partiellement par un agent neutralisant selon l'invention.

Il peut bien évidemment comprendre plusieurs fonctions amines primaires, secondaires et/ou tertiaires, ou un mélange de telles fonctions; il peut en outre optionnellement comprendre des fonctions anioniques et/ou amines quaternaires, et donc être un polymère amphotère qui sera considéré comme un polymère cationique au sens de la présente invention.

Par polymère "éthylénique", on entend notamment un polymère obtenu par polymérisation de monomères comprenant une insaturation éthylénique. On entend également les polymères obtenus par métathèse comme le poly(norbornène) et ses dérivés. Ces polymères peuvent être obtenus par polymérisation radicalaire, anionique ou cationique ou par catalyse de métaux de transition ou par ouverture de cycle.

Le polymère cationique selon l'invention comprend de préférence au moins un monomère dit'cationique' de formule (I), de formule (II) et/ou de formule (III), qui peuvent être présents seul ou en mélange.

Les monomères de formule (I), susceptibles d'être présents, seul ou en mélange, dans le polymère cationique, sont : 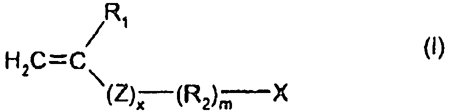 dans laquelle:
- R1 est un atome d'hydrogène ou un radical hydrocarboné, linéaire ou ramifié, de type CₚH₂ₚ₊₁, avec p étant un entier compris entre 1 et 12, inclus;
   Notamment, R1 peut représenter un radical méthyle. éthyle, propyle, butyle. De préférence, R1 représente l'hydrogène ou un radical méthyle.
- Z est un groupement divalent choisi parmi -COO-, -CONH-, -CONCH₃-, -OCO- ou -O-, -SO₂- -CO-O-CO- ou -CO-CH₂-CO-;
   De préférence, Z est choisi parmi COO et CONH.
- x est 0 ou 1, de préférence 1.
- R2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Cl, Br, Si et P;
   Dans le radical R2, le ou les hétéroatomes, quand ils sont présents, peuvent être intercalés dans la chaîne dudit radical R2, ou bien ledit radical R2 peut être substitué par un ou plusieurs groupes les comprenant tels que hydroxy, -CF₃, CN, epoxy ou amino (NH2, NHR' ou NR'R" avec R' et R" identiques ou différents représentant un alkyle linéaire ou ramifié en C1-C22, notamment méthyle ou éthyle).
   Notamment R2 peut être ou peut comprendre :
   - un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
   - un radical phénylène -C₆H₄-(ortho, méta ou para) éventuellement substitué, par un radical alkyle en C1-C12 comprenant éventuellement 1 à 25 hétéroatomes choisis parmi N, O S, F, Si et/ou P; ou bien un radical benzylène -C₆H₄-CH₂- éventuellement substitué, par un radical alkyle en C1-C12 comprenant éventuellement 1 à 25 hétéroatomes choisis parmi O N, S, F, Si et P;
   - un radical de formule -CO-, -O-CO-O-, -CO-O-, -O-, -O-CO-NH-, -CO-NH-, - NHCO-, -N(R')CO-, -NH-CO-NH-, -NR'-, epoxy, -N-CO- avec R' représentant un alkyle linéaire ou ramifié en C1-C22 comprenant éventuellement 1 à 12 hétéroatomes choisis parmi O N, S, F, Si et P;
   - ou un mélange de ces radicaux;
   - m est 0 ou 1;
   - X est
      (a) un groupe guanidino, amidino, ou bien
      (b) un groupe de formule -N(R₆)(R₇) avec R6 et R7 représentant, indépendamment l'un de l'autre, (i) un atome d'hydrogène, (ii) un groupement alkyle linéaire, ramifié ou cyclique, saturé ou insaturé, éventuellement aromatique, comprenant de 1 à 18 atomes de carbone, pouvant comprendre 1 à 10 hétéroatomes choisis parmi O, N, S, F, Si et P; ou (iii) R6 et R7 forment avec l'atome d'azote un cycle de formule : 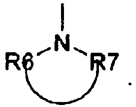 saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit premier cycle pouvant être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 4 hétéroatomes choisi parmi O, S et N. Par exemple, R6 et R7 peuvent être choisis parmi l'hydrogène, un groupement méthyle, éthyle, propyle, isopropyle, n-butyle, t-butyle, isobutyle, octyle, lauryle, stéaryle.
         De préférence R6 et R7 sont choisis, indépendamment l'un de l'autre, parmi H, CH3 et C2H5. ou bien
      (c) X peut représenter un cycle : 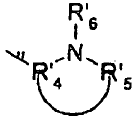 dans lequel R'4 et R'5 forment avec l'atome d'azote un cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant être fusionné avec un ou plusieurs autres cycles, saturés ou insaturés, éventuellement aromatiques, comprenant chacun 5, 6 ou 7 atomes, et notamment 4, 5, 6, 7 ou 8 atomes de carbone et/ou 2 à 4 hétéroatomes choisi parmi O, S et N;
         et R'6 est choisi parmi H, -CH₃ et -C₂H₅.

Par exemple, X peut représenter un hétérocycle, aromatique ou non, contenant un azote secondaire ou tertiaire. Parmi ces radicaux X préférés, on peut citer les radicaux de type indolyle, isoindolinyle, imidazolyle, imidazolinyle, piperidinyl, pyrazolynyl, pyrazolyl, quinoline, pyridinyl, piperazinyl, pyrrolidinyl, quinidinyl, thiazolinyl, morpholine, guanidino, amidino, et leurs mélanges.

Il est bien entendu que le monomère de formule (1) doit comprendre au moins une fonction amine, primaire, secondaire ou tertiaire, protonable à un pH choisi entre pH 1 et pH 12; cette fonction pouvant être, ou être portée par, les radicaux R2 et/ou X.

Parmi les monomères de formule (I) préférés, on peut citer, seul ou en mélange, le (méth)acrylamide de diméthylaminopropyle, le (méth)acrylamide de diméthylaminoéthyle, le (méth)acrylate de diéthylaminoéthyle, le (méth)acrylate de diméthylaminoéthyle, le (méth)acrylate de tert-butylaminoéthyle, le (méth)acrylate de morpholinoéthyle, la vinylimidazole, la vinylpyridine, la vinylamine, l'altylamine, et les monomères ci-après dans lesquels R = H ou méthyl :

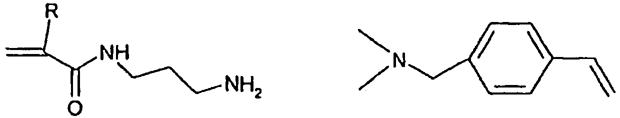

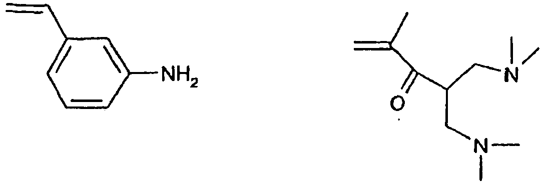

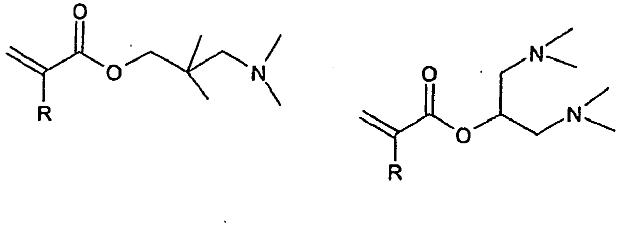

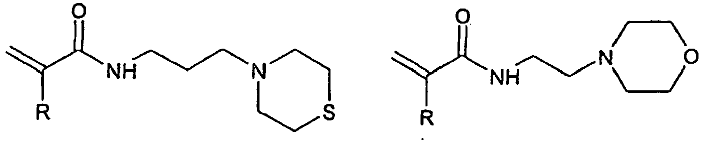

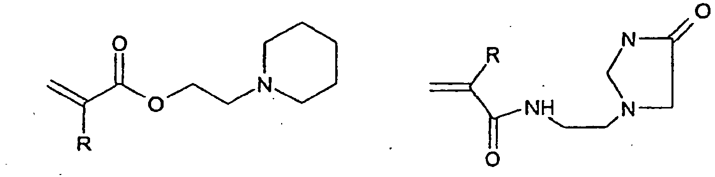

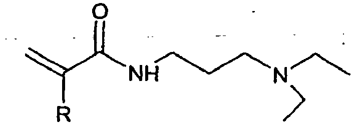

Les monomères de formule (II), susceptibles d'être présents, seul ou en mélange, dans le polymère cationique, sont du type diallyle tel que défini ci-après : 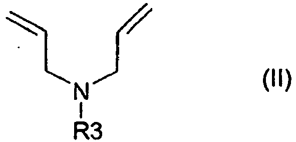 dans laquelle R3 est l'hydrogène ou un radical carboné, notamment hydrocarboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Cl, Br, Si et P.

Dans le radical R3, le ou les hétéroatomes, quand ils sont présents, peuvent être intercalés dans la chaîne dudit radical R3, ou bien ledit radical R3 peut être substitué par un ou plusieurs groupes les comprenant; notamment R3 peut comprendre un groupement choisi parmi hydroxy -OH, CF3, CN, amino (-NH₂, -NHR' ou - NR'R" avec R' et R" identiques ou différents représentant un alkyle linéaire ou ramifié en C1-C22, notamment méthyle ou éthyle); -O-CO-O-, -CO-O-, -O-, -O-CO-NH-, -NH-CO-NH-, -CO-NH-, -NR'-, -N-CO- avec R' représentant un alkyle linéaire ou ramifié en C1-C22 comprenant éventuellement 1 à 12 hétéroatomes choisis parmi O, N, S, F, Si et P.

Notamment R3 peut être :
- un radical alkyle tel que méthyle, éthyle, propyle, n-butyle, isobutyle, tertiobutyle, n-hexyle, n-octyle, n-dodécyle, n-octadécyle, n-tétradécyle, n-docosanyle; comprenant éventuellement 1 à 25 hétéroatomes choisis parmi O, N, S, F, Si et P et/ou éventuellement substitué par au moins un des groupements cités ci-dessus;
- un radical phényle -C₆H₅ éventuellement substitué par un radical alkyle en Cl-C12 comprenant éventuellement 1 à 25 hétéroatomes choisis parmi N, O S, F, Cl, Br, Si et/ou P; et/ou éventuellement substitué par au moins un des groupements cités ci-dessus
- un radical benzyle -C₆H₄-CH₃ éventuellement substitué par un radical alkyle en C1-C12 comprenant éventuellement 1 à 25 hétéroatomes choisis parmi O N, S, F, Cl, Br, Si et P; et/ou éventuellement substitué par au moins un des groupements cités ci-dessus;
- ou un mélange de ces radicaux.

Il est bien entendu que le monomère de formule (II) doit comprendre au moins une fonction amine, primaire, secondaire ou tertiaire, protonable à un pH choisi entre pH 1 et pH 12.

De préférence, R3 est H ou méthyle.

Parmi les monomères de formule (II), on peut notamment citer la N-méthyldiallylamine (R3 = méthyle) et la diallylamine (R3=H).

lets monomères de formule (III), susceptibles d'être présents, seul ou en mélange, dans le polymère cationique, sont : 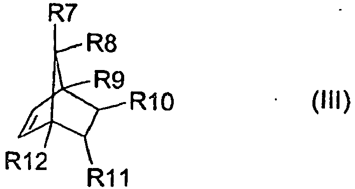 dans laquelle R7 à R12, indépendamment les uns des autres, représentent :
- un atome d'hydrogène,
- un groupement.-NR13R'13 avec R13 et R'13, indépendamment l'un de l'autre, représentant l'hydrogène ou un alkyle linéaire, ramifié ou cyclique, saturé ou insaturé, éventuellement aromatique, comprenant de 1 à 18 atomes de carbone, pouvant comprendre 1 à 10 hétéroatomes choisis parmi O, N, S, F, Si et P; et notamment R13 et R'13 peuvent être choisis parmi l'hydrogène, un groupement méthyle, éthyle, propyle, isopropyle, n-butyle, t-butyle, isobutyle, octyle, lauryle, stéaryle.
   De préférence, -NR13R'13 représente -NH₂, -N(CH₃)₂, -N(C₂H₅)₂, -N(isopropyle)₂ ou -N(butyl)₂.
- un radical carboné, notamment hydrocarboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P.

Dans ces radicaux, le ou les hétéroatomes, quand ils sont présents, peuvent être intercalés dans la chaîne du radical, ou bien le radical peut être substitué par un ou plusieurs groupes les comprenant; notamment le radical peut comprendre un groupement choisi parmi hydroxy -OH, CF3, CN, epoxy, amino (-NH₂, -NHR' ou - NR'R" avec R' et R" identiques ou différents représentant un alkyle linéaire ou ramifié en C1-C22, notamment méthyle ou éthyle); -C(O)-, -O-CO-O-, -CO-O-, -O-,-O-CO-NH-, -NH-CO-NH-, -CO-NH-, -NHCO-, N(R')CO-, -NR'-, -N-CO- avec R' représentant un alkyle linéaire ou ramifié en C1-C22 comprenant éventuellement 1 à 12 hétéroatomes choisis parmi O, N, S, F, Si et P. Il est bien entendu que le monomère de formule (III) doit comprendre au moins une fonction amine, primaire, secondaire ou tertiaire, protonable à un pH choisi entre pH 1 et pH 12.

Notamment R7 à R12 peuvent être :
- H
- NH2
- un radical alkyle tel que méthyle, éthyle, propyle, n-butyle, isobutyle, tertiobutyle, n-hexyle, n-octyle, n-dodécyle, n-octadécyle, n-tétradécyle, n-docosanyle; comprenant éventuellement 1 à 25 hétéroatomes choisis parmi O, N, S, F, Si et P, et/ou éventuellement substitué par au moins un des groupements cités ci-dessus
- un radical phényle -C₆H₅ éventuellement substitué par un radical alkyle en Cl-C12 comprenant éventuellement 1 à 25 hétéroatomes choisis parmi N, O, S, F, Si et/ou P; et/ou éventuellement substitué par au moins un des groupements cités ci-dessus
- un radical benzyle -C₆H₄-CH₃ éventuellement substitué par un radical alkyle en C1-C12 comprenant éventuellement 1 à 25 hétéroatomes choisis parmi ON, S, F, Si et P; et/ou éventuellement substitué par au moins un des groupements cités ci-dessus
- ou un mélange de ces radicaux.

Préférentiellement, au moins un des radicaux R7 à R12 est de forme -NR13R'13. très préférentiellement, il s'agit du radical R11.

De préférence, R11 =NH2
De préférence, R7= H ou méthyle.
De préférence, R8= H ou méthyle
De préférence, R9= H ou méthyle
De préférence, R10= -CH₂OH ou -C(O)OEt
De préférence, R12= H ou méthyle

Parmi les monomères de formule (III), on peut notamment citer les monomères suivants :

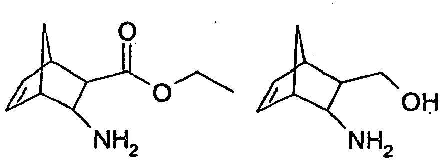

Les monomères de formule (I), (II) et/ou (III), peuvent représenter la totalité des monomères présents dans le polymère (soit 100% en poids par rapport au poids du polymère final), ou bien peuvent être présents à raison de 1 à 99,9% en poids par rapport au poids du polymère final, notamment de 30 à 95% en poids, de préférence de 35 à 90% en poids, voire à raison de 40 à 85% en poids.

Il est donc possible que, de manière optionnelle, le polymère cationique selon l'invention, comprenne des monomères additionnels, non cationiques, qui peuvent être présents à raison de 0,01 à 99% en poids par rapport au poids du polymère final, notamment de 5 à 70% en poids, de préférence de 10 à 65% en poids, voire à raison de 15 à 60% en poids.

Ces monomères additionnels peuvent être choisis parmi les monomères suivants, seuls ou en mélange, ainsi que leurs seins :
- (i) les esters de l'acide (méth)acrylique de formule CH₂=CHCOOR'1 ou CH₂=C(CH₃)COOR'1 avec R'1 représentant une chaîne carbonée, notamment hydrocarbonée (alkyle), ayant 1 à 30 atomes de carbone, linéaire, ramifiée ou cyclique, saturée ou insaturée, éventuellement aromatique (aryle, aralkyle ou alkylaryle), comprenant éventuellement 0 à 2 fonctions éthers (-O-) et éventuellement 0 à 12, notamment 1 à 8, fonctions choisies parmi -OH (hydroxy), -OR' avec R' alkyle en C1-C6 (alcoxy), -CN, -X (halogène, notamment Cl, F, Br ou I), -CO-, - S03H, -COOH, -OCOO-, -COO-, -OCONR-, -OCONH-, -NH-CONH-, -NR-CONR-, -CF3, -CN, epoxy, -NHCO-, -N(R)CO- avec R=alkyle linéaire ou ramifié en C1-C22 comprenant éventuellement 1-12 hétéroatomes;
   On peut ainsi citer les (méth)acrylates de méthyle, d'éthyle, de propyle, d'isopropyle, de n-butyle, d'isobutyle, de tertiobutyle, d'hexyle, de cyclohexyle de 2-éthylhexyle, d'octyle, d'isooctyle, d'isodécyle, de décyle, de dodécyle, de myristyle, de cétyle, de palmityle, de stéaryle, de béhényle, d'oléyle, de tridécyle, d'hexadécyle, d'isobornyle; d'hydroxyéthyle, d'hydroxypropyle; de phényle, de benzyle, de furfuryle; de tétrahydrofurfuryle, d'éthoxyéthyle, de méthoxyéthyle; de glycérol, de 2,2,2-trifluoroéthyle, de poly(éthylène-isobutylène); les (méth)acrylonitriles.
- (ii) les amides de l'acide (méth)acrylique de formule CH₂=CHCONR'2R"2 ou CH₂=C(CH₃)CONR'2R"2 avec R'2, R"2, identiques ou différents, représentant l'hydrogène ou une chaîne carbonée, notamment hydrocarbonée (alkyle), ayant 1 à 30 atomes de carbone, linéaire, ramifiée ou cyclique, saturée ou insaturée, éventuellement aromatique, comprenant éventuellement 0 à 2 fonctions éthers (-O-) et éventuellement 0 à 12, notamment 1 à 8, fonctions choisies parmi -OH (hydroxy), -OR' avec R' alkyle en C1-C6 (alcoxy), -CN, -X (halogène, notamment Cl, F, Br ou 1), -CO-, -S03H, -COOH, -OCOO-, -COO-, -OCONR-, -OCONH-, -NH-CONH-, - NR-CONR-, -CF3, -CN, epoxy, -NHCO-, -N(R)CO- avec R=alkyle linéaire ou ramifié en C1-C22 comprenant éventuellement 1-12 hétéroatomes; On peut ainsi citer la (méth)acrylamide, la N-méthyl (méth)acrylamide, la N-isopropyle (méth)acrylamide, la N-tertbutyl (méth)acrylamide, la N-octyl (méth)acrylamide, la N-undécyl(méth)acrylamide, la N,N-diméthyl (méth) acrylamide, la N,N-dibutyl (méth)acrylamide;
- (iii) les esters de vinyle de formule CH₂=CH-OCO-R'3 ou CH₂=C(CH₃)-OCO-R'3 avec R'3 représentant une chaîne carbonée, notamment hydrocarbonée, ayant 1 à 30 atomes de carbone, linéaire, ramifiée ou cyclique, saturée ou insaturée, éventuellement aromatique, comprenant éventuellement 0 à 2 fonctions éthers (-O-) et éventuellement 0 à 12, notamment 1 à 8, fonctions choisies parmi -OH (hydroxy), -OR' avec R' alkyle en C1-C6 (alcoxy), -CN, -X (halogène, notamment Cl, F, Br ou I), -CO-, -SO3H, -COOH, -OCOO-, -COO-, -OCONR-, -OCONH-, -NH-CONH-, - NR-CONR-, -CF3, -CN, epoxy, -NHCO-, -N(R)CO- avec R=alkyle linéaire ou ramifié en C1-C22 comprenant éventuellement 1-12 hétéroatomes;
   On peut notamment citer l'acétate de vinyle, le propionate de vinyle, le butyrate (ou butanoate) de vinyle, l'éthylhexanoate de vinyle, le néononanoate de vinyle et le néododécanoate de vinyle, le néodécanoate de vinyle, le pivalate de vinyle, le cyclohexanoate de vinyle, le benzoate de vinyle, le 4-tert-butylbenzoate de vinyle, le trifluoroacétate de vinyle.
- (iv) les éthers de vinyle de formule CH₂=CHOR'4 ou CH₂=C(CH₃)OR'4 avec R'4 représentant une chaîne carbonée, notamment hydrocarbonée, ayant 1 à 30 atomes de carbone, linéaire, ramifiée ou cyclique, saturée ou insaturée, éventuellement aromatique, comprenant éventuellement 0 à 2 fonctions éthers (-O-) et éventuellement 0 à 12, notamment 1 à 8, fonctions choisies parmi -OH (hydroxy), -OR' avec R' alkyle en C1-C6 (alcoxy), -CN, -X (halogène, notamment Cl, F, Br ou 1), - CO-, -SO3H, -COOH, -OCOO-, -COO-, -OCONR-, -OCONH-, -NH-CONH-, -NR-CONR-, -CF3, -CN, epoxy, -NHCO-, -N(R)CO- avec R=alkyle linéaire ou ramifié en C1-C22 comprenant éventuellement 1-12 hétéroatomes;
   On peut citer le méthylvinyléther, féthylvinyléther, l'éthylhexylvinyléther et le butylvinyléther, le cyclohexylvinyléther, l'isobutylvinyléther.
- (v) les composés vinyliques de formule CHF"5=CR5R'5 dans laquelle :
   R"5 est H ou COOH, et
   R5 est H, CN ou COOH, et
   R'5 est choisi parmi :
      - un atome d'hydrogène, ou un groupe choisi parmi -OH, -CH=O, halogène (Cl, Br, 1 Notamment), -COOH, -CH2COOH, -NHC(O)H, -N(CH3)-C(O)H, -NHC(O)CH₃,
      - N(CH3)-C(O)CH₃;
      - un cycle : 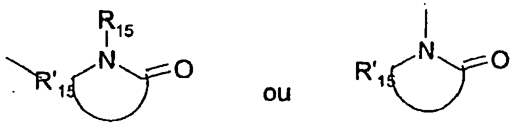 où R'15 et R15 représentent, indépendamment l'un de l'autre, H, un groupe alkyle linéaire ou ramifié, saturé ou insaturé, éventuellement aromatique, cyclique ou non, comprenant 1 à 25 atomes de carbone, dans lequel se trouve(nt) éventuellement intercalé(s) un ou plusieurs hétéroatomes choisis parmi O N, S et P; ledit groupe alkyle pouvant, en outre, être éventuellement substitué par un ou plusieurs substituants choisis parmi -OH et les atomes d'halogène (Cl, Br, I et F);
      - un groupe alkyle linéaire ou ramifié, comprenant 1 à 25 atomes de carbone,
      - un groupe cycloalkyle en C₃ à C₈ tel que cyclohexane,
      - un groupe aryle en C₆ à C₂₀ tel que phényle,
      - un groupe aralkyle en C₇ à C₃₀ (groupe alkyle en C₁ à C₄) tel que 2-phényléthyle ou benzyle,
      - un groupe hétérocyclique de 4 à 12 chaînons contenant un ou plusieurs hétéroatomes choisis parmi O N, et S,
      - un groupe hétérocycloalkyle (alkyle de 1 à 4 carbones), tel que furfuryle, furfurylméthyle ou tétrahydrofurfurylméthyle,
   lesdits groupes alkyles, cycloalkyle, aryle, aralkyle, hétérocyclique ou hétérocycloalkyle pouvant comprenant éventuellement 0 à 2 fonctions éthers (-O-) et éventuellement 0 à 12, notamment 1 à 8, fonctions choisies parmi -OH (hydroxy), -OR' avec R' alkyle en C1-C6 (alcoxy), -CN, -X (halogène, notamment Cl, F, Br ou I),-CO-, -S03H, -COOH, -OCOO-, -COO-, -OCONR-, -OCONH-, -NH-CONH-, -NR-CONR-, -CF₃, -CN, epoxy, -NHCO-, -N(R)CO- avec R=alkyle linéaire ou ramifié en C1-C22 comprenant éventuellement 1-12 hétéroatomes; et/ou pouvant être éventuellement substitué par un ou plusieurs groupes alkyles en C1-C4, linéaires ou ramifiés, eux-mêmes comprenant éventuellement 0 à 2 fonctions éthers (-O-) et éventuellement 0 à 12, notamment 1 à 8, fonctions choisies parmi -OH (hydroxy), -OR' avec R' alkyle en C1-C6 (alcoxy), -CN, -X (halogène, notamment Cl, F, Br ou 1), -CO-, -S03H, -COOH, -OCOO-, -COO-, - OCONH-, -NH-CONH-, -CF₃, -CN, epoxy, -NHCO-, -N(R)CO- avec R=alkyle linéaire ou ramifié en C1-C22 comprenant éventuellement 1-12 hétéroatomes. Des exemples de tels monomères vinyliques sont l'alcool vinylique, le vinylcyclohexane; la vinylpyrrolidone, la vinylcaprolactame, la N-vinyl acétamide, la N-vinylformamide, le N-méthyl-N-vinylformamide, la N-vinylacétamide ; la N-méthyl-N-vinylacétamide; le styrène, le méthylstyrène, le 4-tert-butylstyrene, le 4-acétoxystyrène, le4-méthoxystyrene, le 3-méthylstyrene; le 4-méthylstyrene, le 2-chlorostyrene, le 3-chlorostyrene, le 4-chlorostyrene, le diméthylstyrène, le 2,6-dichlorostyrène, le 2,4-diméthylstyrène; le 2,5-diméthylstyrène, le 3,5-éthoxystyrène, le 2,4-éthoxystyrène, le 4-fluorostyrène; le vinyl butyral; le vinyl carbazole; le vinyl chlorure; le vinyl formal; le chlorure de vinylidène, le fluorure de vinylidène, le 2-vinyl naphtalène; le N-méthyl maléimide; le 1-octene, le 1-butene, le cis-chlorobutadiene, le trans-chlorobutadiene, le chlorotrifluoroethylene; le cis-isoprene, le trans-isoprene, le 1-octadecene, le butadiène, l'hexadécène et l'eicosène.
- (vii) les monomères anioniques suivants, et leurs sels :
   l'anhydride maléique, l'acide acrylique, l'acide méthacrylique, l'acide crotonique, l'acide itaconique, l'acide fumarique, l'acide maléique, l'acrylate de 2-carboxyéthyle (CH2=CH-C(O)-O-(CH₂)₂-COOH); l'acide styrènesulfonique, l'acide 2-acrylamido-2-methylpropanesulfonique, l'acide vinylbenzoïque, l'acide vinylphosphonique, le (méth)acrylate de sulfopropyle, et parmi les sels: le (méth)acrylate de sodium ou de potassium;
- (viii) les monomères amphotères suivants :
   la N,N-dimethyl-N-(2-methacryloyloxyethyl)-N-(3-sulfopropyl) ammonium bétaïne (notamment la SPE de la société Raschig); la N,N-dimethyl-N-(3-methacrylamidopropyl)-N-(3-sulfopropyl) ammonium bétaïne (SPP de Raschig), et la 1-(3-sulfopropyl)-2-vinylpyridinium bétaïne (SPV de Raschig), ainsi que la 2-méthacryloyloxyéthylphosphorylcholine.
- (ix) les monomères de formulé : 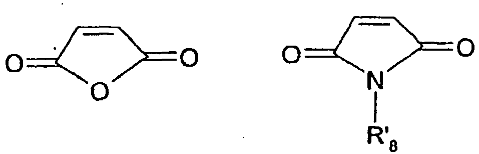 dans lesquelles R'8 est H ou un groupe alkyle linéaire ou ramifié, saturé ou insaturé, éventuellement aromatique, cyclique ou non, comprenant 1 à 25 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi -OH et les atomes d'halogène (Cl, Br, I est F); On peut notamment citer l'anhydride maléique et le N-méthyl maléimide.
- (x) les monomères de formule (I), (II) et (III) quaternisés, et les formes quaternisées des monomères additionnels ci-dessus.
   On peut notamment citer le chlorure de N,N'-diméthyldiallylammonium, le chlorure de triéthylammoniuméthyl methacrylate (MADQUAT), le chlorure de 4-methyl vinyl pyridinium, le chlorure de N-methyl N-vinyl imidazolinium, le chlorure de triméthylammonium propyl (méth)acrylamide.
- (xi) les composés multivalents comprenant au moins deux fonctions polymérisables de type vinylique, (méth)acrylique, allylique, (meth)acrylamide, et notamment les monomères difonctionnels tels que le 1,3 butanediol di(meth) acrylate ; le 1,6 hexanediol di(meth)acrylate, le chlorure de N,N'-diméthyldiallylammonium.

Parmi les monomères additionnels préférés, on peut citer ceux choisis parmi le vinyl néodécanoate, le terbutylbenzoate de vinyle; la vinylpyrrolidone ; la vinylcaprolactame ; le N-vinyl formamide ; le chlorure de N,N'-dimethyldiallylammonium; le chlorure de triethylammoniumethyl methacrylate (MADQUAT); les (méth)acrylates d'éthyle, de méthyle, de tertbutyle, ou d'isobornyle; l'acétate de vinyle, l'acide crotonique; l'acide (méth)acrylique, le methacryoylethylbétaine, l'octylacrylamide; le chlorure de N-methyl N-vinyl imidazolinium, le 1-eicosène, le terbutylacrylamide, l'acrylamide, l'hexadécène, et leurs mélanges.

Le polymère cationique selon l'invention comprend au moins trois unités répétitives; il peut se présenter sous la forme d'un homopolymère ou d'un copolymère qui peut être linéaire ou branché, réticulé ou non réticulé; il peut être statistique, alterné, à blocs ou à gradient, voire en étoiles. Il est de préférence linéaire, statistique ou à blocs.

Dans un mode de réalisation particulier de l'invention, le polymère cationique selon l'invention ne comprend pas de monomère de formule (I') : 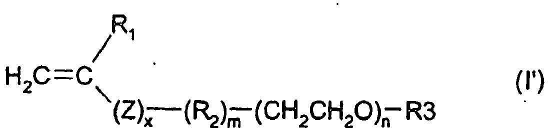 dans laquelle :
- R1 est un atome d'hydrogène ou un radical hydrocarboné, linéaire ou ramifié, de type CₚH₂ₚ₊₁, avec p étant un entier compris entre 1 et 12 inclus;
- Z est un groupement divalent choisi parmi -COO-, -CONH-, -CONCH₃-, -OCO- , - O-, -SO₂- -CO-O-CO- ou -CO-CH₂-CO-;
- x est 0 ou 1;
- R2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
- m est 0 ou 1
- n est un entier compris entre 3 et 300 inclus;
- R3 est un atome d'hydrogène ou un radical carboné, saturé ou insaturé, éventuellement aromatique, linéaire, ramifié ou cyclique, de 1 à 30 atomes de carbone, pouvant comprendre 1 à 20 hétéroatomes choisis parmi O N, S, F, Si et P; et leurs sels.

On peut notamment citer, parmi les polymères préférés selon l'invention,
- les copolymères poly(vinylpyrrolidone-co-dimethylaminoethyl méthacrylate), notamment ceux connus sous le nom commercial Copolymer 845, 937 et 958 four nis par ISP,
- les copolymères poly(vinylcaprolactam-co-vinylpyrrolidone-co-dimethylaminoethylmethacrylate), notamment ceux connus sous la dénomination commerciale : Gaffix VC 713 ou H20LD de ISP;
- les copolymères poly(vinylpyrrolidone-co-dimethylaminopropylmethacrylamide notamment celui connu sous le nom Styleze CC-10 de ISP;
- les copolymères poly(vinylpyrrolidone-co-polyvinylcaprolactam-co-dimethylaminopropylmethacrylamide), notamment l'Aquaflex SF-40 de ISP;
- la poly(vinylamine), la poly(allylamine), la poly(diallylamine);
- les copolymères poly(N-vinyi formamide-co-vinylamine).

Les polymères selon l'invention présentent une masse moléculaire moyenne en poids (Mw) qui est de préférence comprise entre 1000 et 3 000 000, notamment comprise entre 1500 et 1 000 000 et plus préférentiellement comprise entre 2 000 et 800 000, et encore mieux entre 2500 et 500 000. On détermine les masses molaires moyennes en poids (Mw) par chromatographie par perméation sur gel ou par diffusion de la lumière, selon l'accessibilité de la méthode (solubilité des polymères considérés).

Ces polymères peuvent éventuellement être fonctionnalisés de manière à leur conférer un caractère soluble ou dispersible notamment dans le solvant dans lequel ils sont destinés à être formulés, comme par exemple l'eau, les alcools et notamment l'éthanol, ou encore les huiles carbonées, esters, fluorées, silicones et/ou leurs mélanges.

Les polymères selon l'invention peuvent être préparés selon les méthodes usuelles de polymérisation radicalaire classique, bien connues de l'homme du métier, et telles que décrites par exemple dans l'ouvrage "Chimie et physicochimie des polymères" de Gnanou et al. (édition Dunod).
Notamment ces polymères peuvent être préparés par :
- polymérisation directe en solution dans l'eau avec pré-neutralisation ou non du motif cationique et/ou du motif anionique;
- polymérisation en émulsion dans l'eau avec pré-neutralisation ou non du motif cationique et/ou du motif anionique, avec utilisation d'un tensioactif;
- polymérisation dans un solvant organique, tel que l'éthanol ou la méthyléthylcétone, avec pré-neutralisation ou non du motif cationique et/ou du motif anionique, suivie d'une étape de mise en solution ou dispersion dans l'eau avec évaporation du solvant.
Ces polymérisations peuvent être effectuées en présence d'amorceur radicalaire notamment de type peroxyde (Trigonox 21 S : tert-butyl peroxy-2-ethylhexanoate) ou azoïque (AIBN ou V50: 2,2'-azo-bis(2-amidinopropane) dihydrochlorure) ou persulfate de potassium ou d'ammonium, qui peut être présent à raison de 0.1 à 5% en poids par rapport au poids total des monomères.

Selon un premier mode de réalisation, le polymère cationique peut être préparé par polymérisation via un mécanisme usuel, notamment radicalaire, anionique, de coordination (catalyse par métaux de transition), cationique, ou d'ouverture de cycle, d'au moins un monomère comprenant au moins une fonction amine protonable, et éventuellement d'un ou plusieurs comonomères additionnels. Dans ce cas, la fonction amine protonable découle de la copolymérisation d'un monomère la portant.

Selon un second mode de réalisation, le polymère cationique selon l'invention peut être préparé par polymérisation, via un mécanisme usuel, d'au moins un monomère portant une fonction amine protégée (précurseur de fonctions amines) et éventuellement d'un ou plusieurs comonomères additionnels. Dans ce cas, la fonction amine protonable résulte alors d'une étape de transformation postérieure à la polymérisation.

Ces monomères précurseurs peuvent comprendre :
- au moins un motif éthylénique du type (méth)acrylique, (méth)acrylamide, vinylique, allylique, éthylénique cyclique ou non comme les diènes, les norbornènes et dérivés, les diallylamines et dérivés ou leur mélanges capable de polymériser par polymérisation radicalaire, anionique ou cationique, par ouverture de cycle.
   Ce monomère précurseur peut comporter également un hétérocyclique du type oxazoline; et
- au moins un site capable d'engendrer, dans une étape postérieure à la polymérisation, une fonction amine primaire, secondaire ou tertiaire.

On peut ainsi citer comme monomère précurseur, la N-vinyl formamide qui conduit, après polymérisation, au poly(N-vinyl formamide), puis, par hydrolyse basique, à la poly(vinylamine) lorsque l'hydrolyse est totale, ou à un copolymère poly(vinylformamide-co-vinylamine) dans le cas d'une hydrolyse partielle.

Il peut également s'agir du BOC-3-exo-hydroxymethylbicyclo [2.2.1]Hept5-enyl-2-exoamine qui après déprotection de la fonction protectrice BOC conduit à l'obtention d'un polymère comportant une fonction amine protonable :

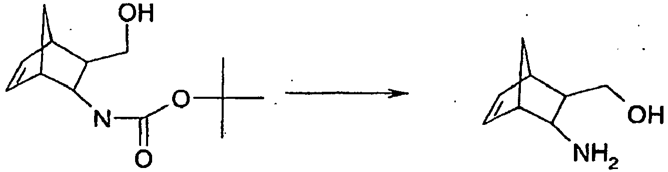

On peut également mentionner l'éthyloxazoline qui, après polymérisation et hydrolyse acide, peut conduire à un polyéthylène imine linéaire :

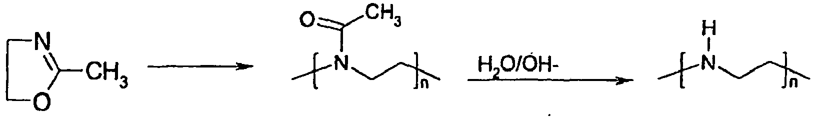

On peut encore mentionner l'ouverture de l'aziridine qui conduit à une polyéthylène imine branchée :

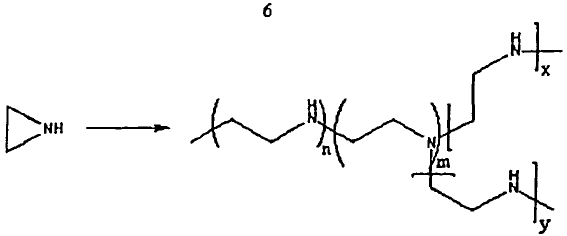

Il peut également s'agir de monomère tel que le 1,3 diacetyl-1,3-dihydro-2H-imidazol-2-one qui, par polymérisation puis hydrolyse basique, conduit à l'obtention de polyméthylène imine :

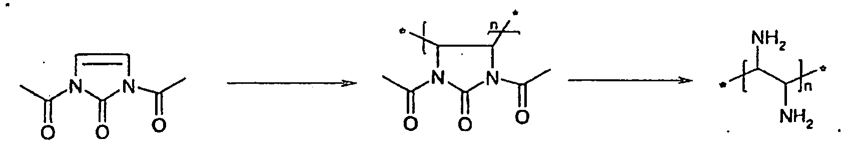

Ainsi le polymère cationique selon l'invention peut également comprendre des unités répétitives choisies, seules ou en mélanges, parmi :

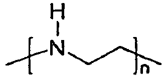

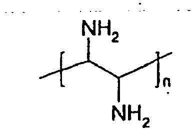

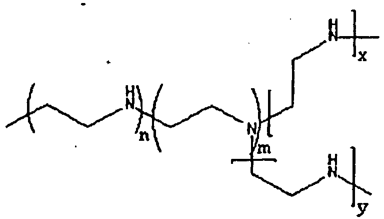

Le polymère selon l'invention est, lorsqu'il est prêt à être employé pour préparer des compositions selon l'invention par exemple, dans un état neutralisé (ses motifs amines sont neutralisés) par au moins un agent neutralisant particulier choisi parmi les acides organiques polymériques, comprenant au moins une fonction acide au sens de Bronsted.
On entend par là que les monomères, notamment ceux de formule (I) et/ou le polymère final, ont été neutralisés par au moins un tel agent neutralisant.

Il est ainsi possible de neutraliser les motifs amines primaires, secondaires et/ou tertiaires, des monomères de formule (I), (II) ou (III), entrant dans la constitution du polymère, avant leur polymérisation, puis de copolymériser l'ensemble des monomères afin d'obtenir le polymère selon l'invention; on parlera alors de pré-neutralisation.
On peut également tout d'abord polymériser l'ensemble des monomères, puis neutraliser le polymère après sa formation.
On parlera de neutralisation ou post-neutralisation.
De préférence, on neutralise le polymère après sa formation.

Par neutralisation, on entend selon l'invention, l'action d'un acide organique selon l'invention, comprenant au moins une fonction acide au sens de Bronsted, sur tous ou partie des monomères et/ou polymère ci-dessus mentionné, comprenant au moins une fonction basique (motifs amines) au sens de Bronsted.

L'agent neutralisant peut être ajouté en une quantité de 0,01 à 3 équivalent molaire, notamment 0,05 à 2,5, voire 0,1 à 2 équivalent molaire, par rapport aux fonctions amines totales du polymère ou des monomères.

Il est ainsi possible de neutraliser partiellement le polymère, c'est-à-dire que l'agent neutralisant peut être présent en une quantité nécessaire pour neutraliser 1. à 99%, notamment 5 à 90%, voire 10 à 80%, des fonctions amines totales du polymère ou des monomères; ce qui signifie qu'il est présent en une quantité de 0,01 à 0,99 équivalent molaire, notamment 0,05 à 0,9, voire 0,1 à 0,8 équivalent molaire.
Il est également possible de sur-neutraliser le polymère, c'est-à-dire que l'agent neutralisant peut être présent en excès, en une quantité nécessaire pour neutraliser 101 à 300%, notamment de 120 à 250%, voire de 150 à 200%, des fonctions amines totales du polymère ou des monomères; ceci peut être le cas lorsque l'on souhaite assurer au polymère une gamme de pH et/ou une force ionique adéquate vis-a-vis des formulations envisagées. Il peut donc être présent en une quantité de 1,01 à 3 équivalent molaire, notamment 1,2 à 2,5, voire 1,5 à 2 équivalent molaire, par rapport aux fonctions amines totales du polymère ou des monomères.
De préférence, l'agent neutralisant est présent en une quantité stoechiométrique par rapport aux fonctions amines totales du polymère ou des monomères; il est donc présent en une quantité nécessaire pour neutraliser 100% des motifs amines du polymère ou des monomères, soit 1 équivalent molaire.
Il est bien évidemment possible d'utiliser un mélange d'agents neutralisants selon l'invention.

Par ailleurs, lorsque le polymère est neutralisé partiellement par un agent neutralisant selon l'invention, il est possible d'ajouter en outre un second agent neutralisant, choisi parmi les acides minéraux, et notamment l'acide chlorhydrique, l'acide sulfurique, et/ou les acides organiques non polymériques, tels que l'acide tartrique, l'acide gluconique, l'acide lactique, l'acide benzoïque, l'acide acétique.

Préférentiellement, la nature et la quantité d'agent neutralisant peut être déterminée par l'homme du métier de manière à obtenir au final un polymère soluble ou dispersible dans l'eau.

L'agent neutralisant selon l'invention est polymérique; on entend par là qu'il est un polymère, homo- ou copolymère, comprenant au moins 3 unités répétitives. Dans un mode de réalisation particulier, l'agent neutralisant est un polymère qui comprend au moins trois unités répétitives de type alkylèneglycol, préférentiellement de type éthylène glycol : (-CH2-CH2-O-).
L'agent neutralisant polymérique peut être notamment de structure linéaire, branché, à greffons, à blocs (dibloc, tribloc, multiblocs), en étoiles, ou dendrimères. Il peut être statistique, alterné, à gradient.
On préfère les agents neutralisants polymériques linéaires, statistiques ou à blocs.

Il présente de préférence, une masse moléculaire en nombre (Mn) comprise entre 300 et 50000, notamment entre 350 et 20000 et, plus préféré entre 400 et 10000, voire 450 et 5000 (g/mol).

L'agent neutralisant comporte donc au moins une fonction acide au sens de Bronsted, et notamment 1 à 6 fonctions acides, voire 2 à 5 fonctions acides, choisies parmi les groupes acide carboxylique (-COOH), sulfonique (-SO₃H) et/ou phosphonique (-PO₃H₂); lesdits groupes étant capables de protoner, totalement ou partiellement, les fonctions amines primaire, secondaire et/ou tertiaire, du polymère cationique.

La/les fonctions acides peuvent être situées en bout de chaînes (aux extrémités) et/ou être réparties le long de la chaîne.
De préférence, au moins une fonction acide est en bout de chaîne; lorsqu'il y a deux fonctions acides, elles sont de préférence chacune à une extrémité de la chaîne.

Lorsque l'agent neutralisant est linéaire, il comprend de préférence 1 à 3 fonctions acides, dont de préférence l'une d'entre elle au moins à l'extrémité de la chaîne. Lorsque l'agent neutralisant est à greffons, branchés, en étoiles ou dendrimères, il comprend de préférence 1 à 6 fonctions acide, réparties de préférence de façon à avoir une fonction par branche.

De manière préférentielle, l'agent neutralisant est linéaire et comporte 2 fonctions acide, une à chaque extrémité de la chaîne.
Selon un autre mode de réalisation, l'agent neutralisant est linéaire et comporte une seule fonction acide, terminale à l'une des extrémités de la chaîne.
Selon encore un autre mode de réalisation, l'agent neutralisant est branché et les fonctions acides sont réparties aux extrémités des chaînes.
De préférence, l'agent neutralisant est soluble dans l'eau, à 25°C, à raison d'au moins 5% en poids.

De manière préférentielle, l'agent neutralisant comporte uniquement des séquences hydrophiles, qui sont terminées aux deux ou à une seule extrémité par une fonction acide.

L'agent neutralisant peut être choisi parmi :
- (i) les polymères comportant des unités répétitives de dextran comprenant au moins une fonction acide, notamment carboxylique, en bout de chaîne
- (ii) les poly(alkyloxazolines) terminées acides, de préférence la polyméthyloxazoline et la polyéthyloxazoline comprenant au moins une fonction acide.
- (iii) les poly(N-methyl) sarcosines fonctionnalisées acide, notamment carboxylique;
- (iv) les dendrimères ou molécules hyperbranchées fonctionnalisées en surface par des groupements acides, notamment carboxyliques; telles que par exemple les PAMAM starburst de Dow Chemical;
- (v) les polymères hyperbranchés de type polyesters obtenu par réaction entre l'acide adipique et le glycérol.
- (vi) les poly(glycérol) poly(hydroxyéthyl acrylate) et poly(vinylpyrrolidone) terminées acides, notamment carboxyliques;
   Toutefois, l'agent neutralisant selon l'invention est préférentiellement choisi parmi :
- (vii) les polymères comprenant au moins trois unités répétitives de type alkylènes glycols pour lesquels le groupe alkyle comprend 2 à 4 atomes de carbone, et leurs combinaisons; on peut citer les polyéthylène glycol, les polypropylène glycol, les polyéthylène-co-propylèneglycol (PEG/PPO), les polytétraméthylène oxyde-co-polyéthylène glycol (PTMO/PEG); étant entendu qu'ils comportent en outre au moins une fonction acide (notamment carboxylique, sulfonique, et/ou phosphonique);
   Il peut s'agir d'homopolymères ou de polymères statistiques, bloc, branché ou en étoiles; ils peuvent comprendre d'autres unités répétitives, par exemple du type siloxane, notamment PDMS..
   L'agent neutralisant est choisi parmi les homopolymères de polyalkylène glycols (alkyl en C2-C3), ou les copolymères, statistiques ou blocs, de polyalkylèneglycols (alkyle en C2-C3). On peut en particulier citer les polymères de structures suivantes :

   CH₃-O-(CH₂-CH₂-O-)n-co-(CH₂-CHR-O)m-CH₂-R₁₀

   CH₃-O-(CH₂-CH₂O-)n-b-(CH₂-CHR-O)m-CH₂-R₁₀

   CH₃-O-(CH₂-CH₂O-)n-co-(CH₂-CHR-O)m-CH₂-CH₂R₁₀

   CH₃-O-(CH₂-CH₂-O-)n-b-(CH₂-CHR-O)m-CH₂-CH₂R₁₀

   CH₃-O-(CH₂-CH₂-O-)n-CO-(CH₂-CHR-O)m-CH₂-CH₂CH₂R₁₀

   CH₃-O-(CH₂-CH₂-O-)n-b-(CH₂-CHR-O)m-CH₂-CH₂CH₂R₁₀

   CH₃-O-(CH₂-CH₂-O-)n-co-(CH₂-CHR-O)m-CH₂-CH₂OCO-CH₂-CH₂COOH

   CH₃-O-(CH₂-CH₂-O-)n-b-(CH₂-CHR-O)m-CH₂-CH₂OCO-CH₂-CH₂COOH

   R₁₀-(CH₂-CH₂-O-)n-co-(CH₂CHR-O)m-CH₂-CH₂R₁₀

   R₁₀-(CH₂-CH₂-O-)n-b-(CH₂-CHR-O)m-CH₂-CH₂R₁₀

   R₁₀-CH₂O-(CH₂-CH₂-O-)n-co-(CH₂-CHR-O)m-CH₂-R₁₀

   R₁₀-CH₂O-(CH₂-CH₂-O-)n-b-(CH₂-CHR-O)m-CH₂-R₁₀

   R₁₀-CH₂-CH₂-CH₂-O-(CH₂-CH₂-O-)n-co-(CH₂-CHR-O)m-CH₂-CH₂-CH₂-R₁₀

   R₁₀-CH₂-CH₂-CH₂-O-(CH₂-CH₂-O-)n-b-(CH₂-CHR-O)mCH₂-CH₂-CH₂-R₁₀

   R₁₀-CH₂-CH₂-COO-(CH₂-CH₂-O-)n-co-(CH₂-CHR-O)m-CH₂-CH₂-O-CO-CH₂-CH₂-R₁₀

   R₁₀-CH₂-CH₂-COO-(CH₂-CH₂-O-)n-b-(CH₂-CHR-O)M-CH₂-CH₂-O-CO-CH₂-CH₂-R₁₀

   R₁₀-CH₂-CH₂-CONH-(CH₂-CH₂-O-)n-co-(CH₂-CHR-O)m-CH₂-CH₂-NH-CO-CH₂-CH₂-R₁₀

   R₁₀-CH₂-CH₂-CONH-(CH₂-CH₂-O-)n-b-(CH₂-CHR-O)m-CH₂-CH₂-NH-CO-CH₂-CH₂-R₁₀

   dans lesquelles :
   ∘ le radical R, identique ou différent, représente -CH₃, -(CH₂)x-COOH;-(CH₂)x-SO₃H; (CH₂)x-PO₃H₂; avec x = 1 à 8;
   ∘ le radical R₁₀, identique ou différent, est -COOH, -SO₃H ou -PO₃H₂;
   ∘ n est un entier compris entre 3 et 1000;
   ∘ m est un entier compris entre 0 et 200.
   étant donné que le ratio n:m est supérieur à 5, quand m est non nul.

De préférence, R représente méthyle, ou encore les radicaux avec x = 2 à 3.
De préférence, n représente 4 à 30, notamment 5 à 15.

On peut bien évidemment utiliser un mélange de différents agents neutralisants.

Préférentiellement, l'agent neutralisant est choisi parmi les structures suivantes :

CH₃-O-(CH₂-CH₂-O-)n-co-(CH₂CHR-O)m-CH₂CH₂R₁₀

CH₃-O-(CH₂CH₂O-)n-co-(CH₂CHR-O)m-CH₂CH₂CH₂R₁₀

R₁₀-(CH₂-CH₂-O-)n-co-(CH₂-CHR-O)M-CH₂-CH₂R₁₀

R₁₀-CH₂O(CH₂-CH₂-O-)n-co-(CH₂-CHR-O)m-CH₂-R₁₀

dans lesquelles le radical R₁₀ est -COOH, -SO₃H ou -PO₃H₂; de préférence COOH; m est 0 à 200 et n est 3 à 1000, notamment 4 à 30, voire 5 à 15.

Encore plus préférentiellement, on choisit l'agent neutralisant parmi les structures suivantes :

CH₃-O-(CH₂-CH₂-O-)n-CH₂-CH₂R₁₀

CH₃-O-(CH₂-CH₂-O-)n-CH₂-CH₂CH₂R₁₀

R₁₀-(CH₂-CH₂-O-)n-CH₂-CH₂R₁₀

R₁₀-CH₂O-(CH₂-CH₂-O-)n-CH₂-R₁₀

dans lesquelles, le radical R₁₀ est COOH, SO₃H ou -PO₃H₂; de préférence COOH; est n est 3 à 1000, notamment 4 à 30, voire 5 à 15.

On peut en particulier citer:
- CH₃-O-(CH₂-CH₂-O-)n-CH₂-CH₂-SO₃H qui peut être dénommé O-méthyl, O'-(2-sulfoéthyl)polyéthylène glycol;
- CH₃-O-(CH₂-CH₂-O-)n-CH₂-CH₂-CH₂COOH qui peut être dénommé O-méthyl, O'-(2-carboxypropyl)polyéthylène glycol;
- HOCO-(CH₂CH₂-O-)n-CH₂CH₂COOH qui peut être dénommé O,O'-bis(2-carboxyéthyl)polyéthylèrie glycol;
- HOCO-CH₂O-(CH₂-CH₂-O-)n-CH₂-COOH qui peut être dénommé O,O'-bis(2-carboxyméthyl)polyéthylène glycol;
avec n = 3 à 1000, notamment 4 à 30, voire 5 à 15.

Les agents neutralisants de type (vii) peuvent notamment être préparés par réaction d'un polymère présentant des fonctions hydroxyles ou amines, en présence d'un anhydride tel que l'anhydride succinique ou glutarique. Ainsi, par exemple, la réaction d'un méthoxyPEG-OH (ou O-méthyl-PEG) en présence d'anhydride glutarique permet de préparer le PEG-glutarate correspondant.

Les polymères selon l'invention sont de préférence véhiculables en milieu aqueux, c'est-à-dire qu'ils sont de préférence hydrosolubles ou hydrodispersibles.
La mise en solution ou en dispersion dans l'eau peut être effectuée par solubilisation directe du polymère s'il est soluble, ou bien par neutralisation des motifs amines de façon à rendre le polymère soluble ou dispersible dans l'eau.
La mise en solution ou dispersion aqueuse peut également s'effectuer via une étape intermédiaire de solubilisation dans un solvant organique suivie de l'addition d'eau avant évaporation du solvant organique.
De façon préférentielle, la mise en solution ou en dispersion dans l'eau peut être effectuée par mélange du polymère sous forme pulvérulente, avec l'agent neutralisant, puis ajout d'eau.

Les polymères neutralisés selon l'invention trouvent une application toute particulière dans le domaine de la cosmétique. Ils peuvent être présents dans la composition sous forme solubilisée, par exemple dans l'eau ou un solvant organique notamment l'éthanol, l'acétate de butyle, le myristate d'isopropyle, ou encore dans un solvant siliconé tel que les huiles siliconées volatiles, notamment la D5.
Ils peuvent également être présents sous forme de dispersion aqueuse ou organique ou siliconée, notamment dans l'un de ces solvants.

Ils peuvent être utilisés dans les compositions cosmétiques ou pharmaceutiques à raison de 0,01 à 50% en poids de matière sèche, notamment de 0,1 à 30% en poids, voire de 0,3 à 10% en poids, encore mieux de 1 à 3% en poids, par rapport au poids total de la composition.

Les compositions cosmétiques ou pharmaceutiques selon l'invention comprennent, outre lesdits polymères, un milieu physiologiquement acceptable, notamment cosmétiquement ou pharmaceutiquement acceptable, c'est-à-dire un milieu compatible avec les matières kératiniques telles que la peau du visage ou du corps, les cheveux, les cils, les sourcils et les ongles.

La composition peut ainsi comprendre, un milieu hydrophile comprenant de l'eau ou un mélange d'eau et de solvant(s) organique(s) hydrophile(s) comme les alcools et notamment les monoalcools, linéaires ou ramifiés en C1-C6, comme l'éthanol, le tertiobutanol, le n-butanol, l'isopropanol ou le n-propanol, et les polyols comme la glycérine, la diglycérine, le propylène glycol, le sorbitol, le pentylène glycol, et les polyéthylène glycols, ou bien encore les éthers de glycols notamment en C₂ et des aldéhydes en C₂-C₄ hydrophiles.
L'eau ou le mélange d'eau et de solvants organiques hydrophiles peut être présent dans la composition selon l'invention en une teneur allant de 0,1% à 99% en poids, par rapport au poids total de la composition, et de préférence de 10% à 80% en poids.

La composition peut également comprendre une phase grasse, notamment constituée de corps gras liquides à température ambiante (25°C en général) et/ou de corps gras solides à température ambiante tels que les cires, les corps gras pâteux, les gommes et leurs mélanges. Ces corps gras peuvent être d'origine animale, végétale, minérale ou synthétique. Cette phase grasse peut, en outre, contenir des solvants organiques lipophiles.
Comme corps gras liquides à température ambiante, appelés souvent huiles, utilisables dans l'invention, on peut citer : les huiles hydrocarbonées d'origine animale telles que le perhydrosqualène; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol; des alcools gras ayant de 12 à 26 atomes de carbone comme l'octyldodécanol, le 2-butyloctanol, le 2-hexyldécanol, le 2-undécylpentadécanol, l'alcool oléique; les huiles fluorées partiellement hydrocarbonées et/ou siliconées; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes; leurs mélanges.
Ces huiles peuvent être présentes en une teneur allant de 0,01 à 90%, et mieux de 0,1 à 85% en poids, par rapport au poids total de la composition.

La composition selon l'invention peut également comprendre un ou plusieurs solvants organiques, physiologiquement acceptables.
Ces solvants peuvent être généralement présents en une teneur allant de 0,1 à 90%, de préférence de 0,5 à 85%, de préférence encore de 10 à 80% en poids, par rapport au poids total de la composition, et mieux de 30 à 50 %.
On peut notamment citer, outre les solvants organiques hydrophiles cités plus haut, les cétones liquides à température ambiante tels que méthyléthylcétone, méthylisobutylcétone, diisobutylcétone, l'isophorone, la cyclohexanone, l'acétone; les éthers de propylène glycol liquides à température ambiante tels que le monométhyléther de propylène glycol, l'acétate de monométhyléther de propylène glycol, le mono n-butyl éther de dipropylèhe glycol; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à 25°C tels que le diéthyléther, le diméthyléther ou le dichlorodiéthyléther; les alcanes liquides à 25°C tels que le décane, l'heptane, le dodécane, l'isododécane, le cyclohexane; les composés cycliques aromatiques liquides à 25°C tels que le toluène et le xylène ; les aldéhydes liquides à 25°C tels que le benzaldéhyde, l'acétaldéhyde et leurs mélanges.

Par cire au sens de la présente invention, on entend un composé lipophile, solide à température ambiante (25°C), à changement d'état solide/liquide réversible, ayant un point de fusion supérieur ou égal à 25°C pouvant aller jusqu'à 120°C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METTLER.
Les cires peuvent être hydrocarbonées, fluorées et/ou siliconées et être d'origine végétale, minérale, animale et/ou synthétique. En particulier, les cires présentent un point de fusion supérieur à 30°C et mieux supérieur à 45°C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméthicone ayant de 16 à 45 atomes de carbone. Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.
La nature et la quantité des corps solides sont fonction des propriétés mécaniques et des textures recherchées. A titre indicatif, la composition peut contenir de 0,1 à 50% en poids de cires, par rapport au poids total de la composition et mieux de 1 à 30% en poids.

La composition selon l'invention peut en outre comprendre, dans une phase particulaire, des pigments et/ou des nacres et/ou des charges habituellement utilisés dans les compositions cosmétiques.
La composition peut également comprendre d'autres matières colorantes choisies parmi les colorants hydrosolubles ou les colorants liposolubles bien connus de l'homme du métier.
Par pigments, il faut comprendre des particules de toute forme, blanches ou colorées, minérales ou organiques, insolubles dans le milieu physiologique, destinées à colorer la composition.
Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.
Par nacres, il faut comprendre des particules de toute forme irisées, notamment produites par certains mollusques dans leur coquille ou bien synthétisées.
Les pigments peuvent être présents dans la composition à raison de 0,01 à 25% en poids de la composition finale, et de préférence à raison de 3 à 10% en poids. Ils peuvent être blancs ou colorés, minéraux ou organiques. On peut citer les oxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium. On peut encore citer les pigments D&C et les laques couramment employées pour conférer aux lèvres et à la peau un effet de maquillage, qui sont des sels de calcium, de baryum, d'aluminium, de strontium ou de zirconium.
Les nacres peuvent être présentes dans la composition à raison de 0,01 à 20% en poids, de préférence à un taux de l'ordre de 3 à 10% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Parmi les colorants, liposolubles ou hydrosolubles, qui peuvent être présents dans la composition, seul ou en mélange, à raison de 0,001 à 15% en poids, de préférence 0,01 à 5% en poids et notamment de 0,1 à 2% en poids, par rapport au poids total de la composition, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène, le carmin de cochenille, les colorants halogéno-acides, azoïques, anthraquinoniques, le sulfate de cuivre ou de fer, le brun Soudan, le rouge Soudan et le rocou, ainsi que le jus de betterave et le carotène.
La composition selon l'invention peut comprendre en outre en outre une ou plusieurs charges, notamment en une teneur allant de 0,01% à 50% en poids, par rapport au poids total de la composition, de préférence allant de 0,02% à 30% en poids. Les charges peuvent être minérales ou organiques de toute forme, plaquettaires, sphériques ou oblongues. On peut citer le talc, le mica, la silice, le kaolin, les poudres de polyamide (Nylon®), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition peut également comprendre avantageusement au moins un agent tensioactif qui est généralement présent en une quantité comprise entre 0,01% et 50% en poids, de préférence entre 0,1% et 40% et encore plus préférentiellement entre 0,5% et 30%, par rapport au poids total de la composition.
Cet agent tensioactif peut être choisi parmi les agents tensioactifs anioniques, amphotères, non ioniques, cationiques ou leurs mélanges.
Les tensioactifs convenant à la mise en oeuvre de la présente invention sont notamment, seuls ou en mélange, :
- les tensioactifs anioniques parmi lesquels on peut citer, seuls ou mélanges, les sels (en particulier sels de métaux alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfinesulfonates, paraffine-sulfonates; les alkylsulfosuccinates, les alkyléthersulfosuccinates, les alkylamidesulfosuccinates; les alkylsulfosuccinamates; les alkylsulfoacétates; les alkyléther-phosphates; les acylsarcosinates; les acyliséthionates et les N-acyltaurates, le radical alkyle ou acyle de tous ces différents composés comportant de préférence de 8 à 24 atomes de carbone, et le radical aryle désignant de préférence un groupement phényle ou benzyle.
On peut également citer les sels d'acides gras tels que les sels des acides oléique, ricinoléique, palmitique, stéarique, les acides d'huile de coprah ou d'huile de coprah hydrogénée; les acyl-lactylates dont le radical acyle comporte 8 à 20 atomes de carbone; les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés, les acides alkyl (C₆-C₂₄) amido éther carboxyliques polyoxyalkylénés et leurs sels, en particulier ceux comportant de 2 à 50 groupements oxyde d'éthylène, et leurs mélanges. les tensioactifs non ioniques parmi lesquels on peut citer, seuls ou mélanges, les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C10-C14) amines ou les oxydes de N-acylaminopropylmorpholine.
- les tensioactifs amphotères parmi lesquels on peut citer, seuls ou mélanges, les dérivés d'amines secondaires ou tertiaires aliphatiques, dans lesquels le radical aliphatique est une chaîne linéaire ou ramifiée comportant 8 à 22 atomes de carbone et comprenant au moins un groupe anionique hydrosolubilisant (par exemple carboxylate, sulfonate, sulfate, phosphate ou phosphonate); on peut citer encore les alkyl (C₈-C₂₀) bétaïnes, les sulfobétaïnes, les alkyl (C₈-C₂₀) amidoalkyl (C₁-C₆) bétaïnes telles que la cocoamidopropylbétaïne ou les alkyl (C₈-C₂₀) amidoalkyl (C₁-C₆) sulfobétaïnes.
- les tensioactifs cationiques parmi lesquels on peut citer, seuls ou mélanges,
   A) les sels d'ammonium quaternaires de la formule générale (XVI) suivante : 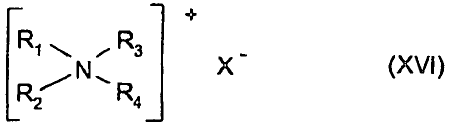 dans laquelle X est un anion choisi parmi les halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate, et
      a) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
         R4 désigne un radical alkyle, linéaire ou ramifié, comportant de 16 à 30 atomes de carbone.
         De préférence le tensioactif cationique est un sel (par exemple chlorure) de béhényl triméthyl ammonium.
      b) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
         R3 et R4, identiques ou différents, désignent un radical alkyle, linéaire ou ramifié, comportant de 12 à 30 atomes de carbone, ledit radical comprenant au moins une fonction ester ou amide.
         R3 et R4 sont notamment choisis parmi les radicaux alkyl(C₁₂₋C₂₂)amido alkyle(C₂-C₆), alkyl(C₁₂-C₂₂) acétate ;
         De préférence le tensioactif cationique est un sel (par exemple chlorure) de stéaramidopropyl diméthyl (myristylacétate) ammonium.
   B) - les sels d'ammonium quaternaire de l'imidazolinium, comme par exemple celui de formule (XVII) suivante : 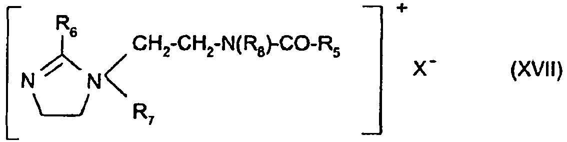 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
   C) - les sels de diammonium quaternaire de formule (XVIII) : 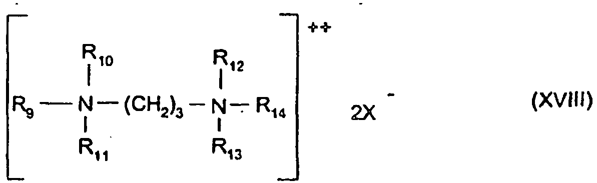 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
   D) - les sels d'ammonium quaternaire comprenant au moins une fonction ester de formule (XIX) suivante : 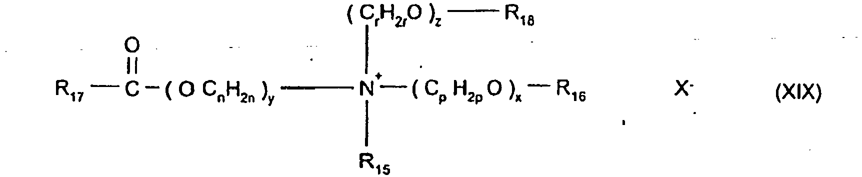 dans laquelle :
      - R15 est choisi parmi les radicaux alkyles en C₁-C₆ et les radicaux hydroxyalkyles ou dihydroxyalkyles en C1-C6 ;
      - R16 est choisi parmi :
         - le radical 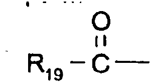
         - les radicaux R20 hydrocarbonés en C1-C22 linéaires ou ramifiés, saturés ou insaturés,
         - l'atome d'hydrogène,
         - R18 est choisi parmi :
         - le radical 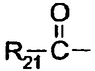
         - les radicaux R22 hydrocarbonés en C1-C6 linéaires ou ramifiés, saturés ou insaturés,
         - l'atome d'hydrogène,
      - R17, R19 et R21, identiques ou différents, sont choisis parmi les radicaux hydrocarbonés en C7-C22, linéaires ou ramifiés, saturés ou insaturés ;
      - n, p et r, identiques ou différents, sont des entiers valant de 2 à 6 ;
      - y est un entier valant de 1 à 10 ;
      - x et z, identiques ou différents, sont des entiers valant de 0 à 10 ;
      - X- est un anion simple ou complexe, organique ou inorganique ;
   sous réserve que la somme x + y + z vaut de 1 à 15, que lorsque x vaut 0 alors R16 désigne R20 et que lorsque z vaut 0 alors R18 désigne R22.

La composition selon l'invention peut également comprendre des ingrédients couramment utilisés en cosmétique, tels que les vitamines, les parfums, les agents nacrants, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, ou leurs mélanges. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter sous la forme d'une suspension, une dispersion notamment d'huile dans de l'eau grâce à des vésicules; une solution huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; Cette composition peut avoir l'aspect d'une lotion, d'une crème, d'une pommade, d'une pâte souple, d'un onguent, d'un solide coulé ou moulé et notamment en stick ou en coupelle, ou encore de solide compacté.

L'homme du métier pourra choisir la forme galénique appropriée, ainsi que sa-méthode de préparation, sur la base de ses connaissances générales, en tenant compte d'une part de la nature des constituants utilisés, notamment de leur solubilité dans le support, et d'autre part de l'application envisagée pour la composition.

La composition cosmétique selon l'invention peut se présenter sous la forme d'un produit de soin et/ou de maquillage de la peau du corps ou du visage, des lèvres et des cheveux, d'un produit solaire ou autobronzant, voire d'un produit capillaire.
Elle trouve notamment une application particulièrement intéressante dans le domaine capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse.

Dans un mode de réalisation préféré, les compositions conformes à l'invention peuvent être utilisées pour le lavage ou le traitement des matières kératiniques telles que les cheveux, la peau, les cils, les sourcils, les ongles, les lèvres, le cuir chevelu et plus particulièrement les cheveux.
Les compositions selon l'invention peuvent être des compositions détergentes telles que des shampooings, des gels-douche et des bains moussants. Dans ce mode de réalisation de l'invention, les compositions comprennent au moins une base lavante, généralement aqueuse, qui peut comprendre de 5 à 35% de tensioactif.
Elles peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Lorsque la composition se présente sous la forme d'un après-shampooing éventuellement à rincer, elle continent avantageusement au moins un tensioactif cationique, par exemple en une concentration généralement comprise entre 0,1 et 10% en poids et de préférence de 0,5 à 5% en poids par rapport au poids total de la composition.
Les compositions de l'invention peuvent encore se présenter sous la forme de compositions lavantes pour la peau, et en particulier sous la forme de solutions ou de gels pour le bain ou la douche ou de produits démaquillants.
Les compositions selon l'invention peuvent également se présenter sous forme de lotions aqueuses ou hydroalcooliques pour le soin de la peau et/ou des cheveux.

L'invention a encore pour objet un procédé de traitement cosmétique des matières kératiniques telles que la peau ou les cheveux, notamment un procédé de maintien de la coiffure, de traitement cosmétique, de soin, de lavage, de démaquillage et/ou de maquillage, des matières kératiniques, notamment de la peau du corps ou du visage, des ongles, des cheveux, des cils, des sourcils, caractérisé en ce qu'il consiste à appliquer sur lesdites matières kératiniques une composition cosmétique telle que définie précédemment, puis à effectuer éventuellement un rinçage à l'eau.

L'invention est illustrée plus en détail dans les exemples suivants.
Dans ces exemples, l'agent neutralisant est un poly(éthylène glycol) bis carboxy-méthyl éther de PM 600 g/mol, commercialisé par Aldrich : HOCO-CH₂O-(CH₂-CH₂-O-)n-CH₂-COOH.

### Exemple 1

On introduit 10 g de poly(4-vinylpyridine) de PM 160,000 g/mol (Aldrich) dans un bécher de 500 ml muni d'un barreau magnétique et on ajoute la quantité indiquée d'agent neutralisant. On chauffe ce mélange sous forme de pâte blanche à 60°C pendant 30 minutes, puis on ajoute 90 ml d'eau. On maintient à 60°C pendant 1 heure. On obtient une solution de polymère, partiellement ou totalement neutralisé.

De manière identique, on prépare différents homo- et copolymères que l'on neutralise avec le même agent neutralisant.
Les résultats sont rassemblés dans le tableau ci-dessous.

| Polymère | PM | Neutralisant* | Solution** |
|---|---|---|---|
| Poly(4-vinylpyridine) | 60 000 | 0,5 | pH = 3,72 22,9% MS |
| polyvinylpyridine | 60 000 | 0,25 | pH=5,11 18,5% MS |
| Poly(4-vinylpyridine) | 160 000 | 0,125 | pH = 4,69 15,01% MS |
| Poly(4-vinylpyridine) | 160 000 | 0,5 | pH = 3,53 23,4% MS |
| Poly(vinylpyridine) | 5 000 | 0,5 | pH = 3,42 8,42% MS |
| poly(styrene-co-vinyl pyridine) 30/70 | 130 000 | 0,5 | pH = 3,42 8,45% MS |
| poly(styrene-co-vinyl pyridine) 30/70 | 130 000 | 0,25 | pH = 3,80 9,02% MS |
| poly(styrene-co-vinyl pyridine) 30/70 | 220 000 | 0,25 | pH=3,08 9,09% MS |
| poly(styrene-co-vinyl pyridine) 30/70 | 220 000 | 0,5 | pH=3,42 8,45% MS |
| MS : matière sèche | | | |
| * neutralisant: quantité, en équivalent molaire, d'agent neutralisant ajouté | | | |
| ** pH et taux de matière sèche de la solution obtenue | | | |

Pour comparaison, on prépare :

| Polymère | PM | neutralisant* | Solution** |
|---|---|---|---|
| polyvinylpyridine | 160 000 | Neutralisé HCl 1eq. | pH = 1,1 10,9% MS |
| polyvinylpyridine | 60 000 | Neutralisé HCl 1eq. | pH=1 9,75% MS |
| Polyvinylpyridine | 5 000 | Neutralisé HCl 1eq. | pH = 1 8,68% MS |
| poly(styrene-co-vinyl pyridine) 30/70 | 220 000 | Neutralisé HCl 1eq. | pH = 1 7,5% MS |

### Exemple 2

De manière identique à l'exemple 1, on neutralise avec un neutralisant CO₂H-PEG-CO₂H:
- des copolymères à base de méthacrylamide de diméthylaminopropyle (DMAPMA) et de vinylpyrrolidone (VP) : produit commercial Styleze CC10 de ISP,
- un homopolymère de DMAPMA,
- des copolymères de poly(vinylamine-vinylformamide) VA/VF : produits commerciaux de BASF : Lupamin 1595 (taux hydrolyse VA ≥90%); Lupamin 9095 (taux hydrolyse VA ≥90% ), Lupamin 4595 (taux hydrolyse VA ≥90%); Lupamin 9030 (taux hydrolyse VA ≥30%);
- des homopolymères de poly(allylamine)

| Polymère | PM | Neutralisant* | Solution** |
|---|---|---|---|
| Poly(DMAPMA/VP) | | 0,5 | pH = 2,78 6,85% MS |
| Poly(DMAPMA/VP) | | 3 | pH = 11,38 24,9% MS |
| poly(DMAPMA) | | 0,5 | pH = 5,79 29,9% MS |

| Polymère | PM | Neutralisant* | Solution" |
|---|---|---|---|
| Poly(VA/VF), Lupamin 1595 | ≤10 000 | 0,25 | pH = 3,22 55,20% MS |
| Poly(VA/VF), Lupamin 9095 | 340 000 | 0,25 | pH = 3,20 25,97% MS |
| Poly(VA/VF), Lupamin 4595 | 45 000 | 0,5 | pH = 3,05 53,6% MS |
| Poly(VA/VF), Lupamin 3095 | 340 000 | 1 | pH = 2,85; 36,60% MS |
| Poly(VANF), Lupamin 3095 | 340 000 | 0,5 | pH = 2,99 30,2% MS |
| Poly(VA/VF), Lupamin 3095 | 340 000 | 0,125 | pH = 3,64 16,8% MS |
| Poly(VA/VF), Lupamin 3095 | 340 000 | 0,5 | pH =4,36 19,6% MS |
| Poly(allylamine) | 17 000 | 0,06 | pH = 10,06 20,5% MS |
| Poly(allylamine) | 17 000 | 0,2 | pH = 8,75 30,9% MS |
| Poly(allylamine) | 17 000 | 1 | pH = 3,16 34,1% MS |
| Poly(allylamine) | 65 000 | 0,06 | pH = 10,29 17,7% MS |
| Poly(allylamine) | 65 000 | 0,2 | pH = 8,70 18,9% MS |
| Poly(allylamine) | 65 000 | 1 | pH = 3,45 30,2% MS |
| Poly(allylamine) | 3000 | 0,2 | pH = 9,84 44,1% MS |
| Poly(allylamine) | 3000 | 1 | pH = 3,43 13,6% MS |

Pour comparaison, on prépare :

| Polymère | PM | Neutralisant* | Solution** |
|---|---|---|---|
| Polyallylamine | 17 000 | Neutralisé HCl 1eq. | 7,09% MS |
| polyallylamine | 65 000 | Neutralisé HCl 1eq. | 6,8% MS |
| polyallylamine | 3000 | Neutralisé HCl 1eq. | 40,0% MS |

### Exemple 3

De manière identique à l'exemple 1, on neutralise avec le même neutralisant, des homopolymères de poly(diallylamine).

La poly(diallylamine) est synthétisée comme suit : on dissout la diallylamine (5.0 g, 0.0515 mol) dans 6, 18 g (10% excès) d'acide chlorhydrique concentré (solution à 36%). La température est maintenue à 0°C, et la solution est ensuite purgée à l'argon.
On ajoute l'amorceur (V50 : 2,2'-azo-bis(2-amidinopropane) dihydrochlorure) (0.14g, 1 mol-%) et le mélange est à nouveau purgé à l'argon puis placé dans un bain d'huile à 60°C pendant 24 heures. La solution jaune visqueuse obtenue est diluée (1 volume) avec de l'eau de qualité HPLC. La solution est ensuite refroidie par un bain de glace et d'éthanol. On ajoute 2 éq. de soude très lentement, puis le polymère précipité est filtré, lavé avec de l'eau qualité HPLC et lyophilisé. Ce polymère peut alors être neutralisé.
A titre de comparaison, on effectue une neutralisation avec HCl 1 N (solution aqueuse à 10%), en une quantité stoechiométrique.

| | neutralisant | forme |
|---|---|---|
| Exemple A | 1 eq. neutralisant selon l'invention | Solution aqueuse 26% MS |
| Exemple B | 0,75 eq. neutralisant selon l'invention | Solution aqueuse 19% MS |
| Exemple C | 0,5 eq. neutralisant selon l'invention | Solution aqueuse 19% MS |
| Exemple D | 0,25 eq. neutralisant selon l'invention | Solution aqueuse 14% MS |
| Contre-exemple E | 1 eq HCl | |
| *MS : matière sèche | | |

On introduit 1% de matière active (MA) des polymères neutralisés A et E ci-dessus dans une base tensioactive comprenant 12,5% MA de lauryléthersulfate et 2,5% MA de cocoylbétaïne, dans l'eau.

Avec la composition selon l'invention, on observe un bon démêlage en milieu humide et une bonne cosmétique en milieu sec (toucher, douceur). On observe un démêlage et un lissage très supérieur avec la composition selon l'invention, par rapport à la composition comprenant le contre-exemple E.

### Exemple 4 : lotion de coiffage

On introduit dans un flacon-pompe :
- 6% MA de solution aqueuse de polymère A de l'exemple 3,
- qs conservateur
- qsp 100% eau
La solution de polymère est sprayée sur une mèche de cheveux de 1,5 g pendant 5 secondes. La mèche est enroulée autour d'un bigoudi de 1 cm de diamètre puis séchée à 70°C pendant 30 minutes. On déroule ensuite la mèche.

On effectue la même chose en remplaçant le polymère A par le polymère E (contre-exemple).

On note une meilleure tenue sur mèches de la coiffure et des boucles lorsqu'elles sont présentes, un toucher amélioré, notamment en terme de douceur, ainsi qu'un meilleur démêlage, pour la coiffure traitée avec la composition selon l'invention, en comparaison avec la coiffure traitée avec le contre-exemple.

## Revendications

1. Composition cosmétique ou pharmaceutique comprenant, dans un milieu physiologiquement acceptable, notamment cosmétiquement ou pharmaceutiquement acceptable, un polymère éthylénique cationique comprenant au moins une fonction amine primaire, secondaire ou tertiaire, protonable, ladite fonction étant au moins partiellement neutralisée par un agent neutralisant, **caractérisée en ce que** ledit agent neutralisant est un acide organique polymérique comprenant au moins une fonction acide carboxylique, sulfonique et/ou phosphonique; et est choisi parmi:
- les polymères comportant des unités répétitives de dextran comprenant au moins une fonction acide, notamment carboxylique, en bout de chaîne;
- les dendrimères ou molécules hyperbranchées fonctionnalisés en surface par des groupements acides, notamment carboxylique;
- les polymères comprenant au moins trois unités répétitives de type alkylènes glycols pour lesquels le group alkyle comprend 2 à 4 atomes de carbone, et leurs combinaisons; étant entendu qu'ils comportent en outre au moins une fonction acide carboxylique, sulfonique et/ou phosphonique choisi parmi les polymères de structures suivantes :
CH₃-O-(CH₂-CH₂-O-)n-co-(CH₂-CHR-O)m-CH₂-R₁₀
CH₃-O-(CH₂-CH₂-O-)n-b-(CH₂-CHR-O)m-CH₂-R₁₀
CH₃-O-(CH₂-CH₂-O-)n-co-(CH₂-CHR-O)m-CH₂-CH₂R₁₀
CH₃-O-(CH₂-CH₂-O-)n-b-(CH₂-CHR-O)m-CH₂-CH₂R₁₀
CH₃-O-(CH₂-CH₂-O-)n-co-(CH₂-CHR-O)m-CH₂-CH₂CH₂R₁₀
CH₃-O-(CH₂-CH₂-O-)n-b-(CH₂-CHR-O)m-CH₂-CH₂CH₂R₁₀
CH₃-O-(CH₂-CH₂-O-)n-co-(CH₂-CHR-O)m-CH₂-CH₂OCO-CH₂-CH₂COOH CH₃-O-(CH₂-CH₂-O)n-b-(CH₂-CHR-O)m-CH₂-CH₂OCO-CH₂-CH₂COOH
R₁₀-(CH₂-CH₂-O-)n-co-(CH₂-CHR-O)M-CH₂-CH₂R₁₀
R₁₀-(CH₂-CH₂-O-)n-b-(CH₂-CHR-O)m-CH₂-CH₂R₁₀
R₁₀-CH₂-O-(CH₂-CH₂-O-)n-co-(CH₂-CHR-O)m-CH₂-R₁₀
R₁₀-CH₂O-(CH₂-CH₂-O-)n-b-(CH₂-CHR-O)m-CH₂-R₁₀
R₁₀-CH₂-CH₂-CH₂-O-(CH₂-CH₂-O-)n-co-(CH₂-CHR-O)m-CH₂-CH₂-CH₂-R₁₀
R₁₀-CH₂-CH₂-CH₂-O-(CH₂-CH₂-O-)n-b-(CH₂-CHR-O)m-CH₂-CH₂-CH₂-R₁₀
R₁₀-CH₂-CH₂-COO-(CH₂-CH₂-O-)n-CO-(CH₂-CHR-O)m-CH₂-CH₂-O-CO-CH₂-CH₂-R₁₀
R₁₀-CH₂-CH₂-COO-(CH₂-CH₂-O-)n-b-(CH₂-CHR-O)m-CH₂-CH₂-O-CO-CH₂-CH₂-R₁₀
R₁₀-CH₂-CH₂-CONH-(CH₂CH₂-O-)n-co-(CH₂-CHR-O)m-CH₂-CH₂-NH-CO-CH₂-CH₂-R₁₀
R₁₀-CH₂-CH₂-CONH-(CH₂-CH₂-O)n-b-(CH₂-CHR-O)m-CH₂-CH₂-NH-CO-CH₂-CH₂-R₁₀
dans lesquelles:
- le radical R, identique ou différent, représente -CH₃, -(CH₂)x-COOH, -(CH₂)x-SO3H; (CH₂)x-PO₃H₂H₂; avec x = 1 à 8;
- le radical R₁₀, identique ou différent, est -COOH, -SO₃H ou -PO₃H₂;
- n est un entier compris entre 3 et 1000;
- m est un entier compris entre 0 et 200.
étant donné que le ratio n:m est supérieur à 5, quand m est non nul

2. Composition selon la revendication 1, dans laquelle le polymère comprend au moins un monomère de formule (I), seul ou en mélange 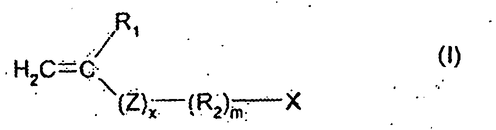 dans laquelle:
- R1 est un atome d'hydrogène ou un radical hydrocarboné, linéaire ou ramifié, de type CₚH₂ₚ₊₁, avec p étant un entier compris entre 1 et 12 inclus;
- Z est un groupememt divalent choisi parmi -COO-, -CONH-, -CONCH₃- -OCO- ou -O-, -SO₂- -CO-O-CO- ou
- CO-CH₂-CO-; de préférence, COO et CONH.
- x est 0 ou 1, de préférence 1.
- R₂ est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Cl, Br, Si et P;
- m est 0 ou 1;
- X est
(a) un groupe guanidino, amidino, ou bien
(b) un groupe de formule -N(R₆)(R₇) avec R6 et R7 représentant, indépendamment l'un de l'autre, (i) un atome d'hydrogène, (ii) un groupement alkyle linéaire, ramifié ou cyclique, saturé ou insaturé, éventuellement aromatique, comprenant de 1 à 18 atomes de carbone, pouvant comprendre 1 à 10 hétéroatomes choisis parmi O, N, S, F, Si et P; ou (iii) R6 et R7 forment avec l'atome d'azote un cycle de formule : 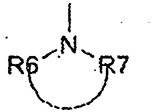 saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit premier cycle pouvant être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 4 hétéroatomes choisi parmi O; S et N.
(c) un cycle: 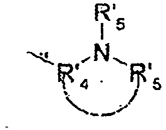
dans lequel R'4 et R'5 forment avec l'atome d'azote un cycle, saturé ou insaturé, éventuellement aromatique comprenant au total 5, 6, 7 ou 8 atomes, et notamment 4, 5 ou 6 atomes de carbone et/ou 2 à 4 hétéroatomes, choisi parmi O, S et N; ledit cycle pouvant être fusionné avec un ou plusieurs autres cycles, saturés ou insaturés, éventuellement aromatiques, comprenant chacun 5, 6 ou 7 atomes, et notamment 4, 5, 6, 7 ou 8 atomes de carbone et/ou 2 à 4 hétéroatomes choisi parmi O, S et N;
et R'6 est choisi parmi H, -CH₃ et -C₂H₅;
étant entendu que ledit monomère de formule (I) comprend au moins une fonction amine, primaire, secondaire ou tertiaire, protonable à un pH choisi entre pH 1 et pH 12; cette fonction pouvant être, ou être portée par, les radicaux R2 et/ou X.

3. Composition selon la revendication 2, dans laquelle, dans le monomère de formule (I), R2 est ou comprend :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 25 hétéroatomes choisis parmi N, O, S, F, Si et/ou P; ou bien un radical benzylène -C₆H₄-CH₂- éventuellement substitué, par un radical alkyle en C1-C12 comprenant éventuellement 1 à 25 hétéroatomes choisis parmi O, N, S, F, Si et P;
- un radical de formule -CO-, -O-CO-O-, -CO-O-, -O-, -O-CO-NH-, -CO-NH-, -NHCO-, -N(R')CO-, -NH-CO-NH-, -NR'-, epoxy, -N-CO- avec R' représentant un alkyle linéaire ou ramifié en C1-C22 comprenant éventuellement 1 à 12 hétéroatomes choisis parmi O, N, S, F, Si et P;
- ou un mélange de ces radicaux.

4. Composition selon l'une des revendications 2 à 3, dans laquelle dans le monomère de formule (I), X est un hétérocycle, aromatique ou non, contenant un azote secondaire ou tertiaire, notamment un radical de type indolyle, isoindolinyle, imidazolyle, imidazolinyle, piperidinyl, pyrazolynyl, pyrazolyl, quinoline, pyridinyl, piperazinyl, pyrrolidinyl, quinidinyl, thiazolinyl, morpholine, guanidino, amidino, et leurs mélanges.

5. Composition selon l'une des revendications 2 à 4, dans laquelle le monomère de formule (I) est choisi parmi, seul ou en mélange, le (méth)acrylamide de diméthylaminopropyle, le (méth)acrylamide de diméthylaminoéthyle, le (méth)acrylate de diéthylaminoéthyle, le (méth)acrylate de diméthylaminoéthyle, le (méth)acrylate de tert-butylaminoéthyle, le (méth)acrylate de morpholinoéthyle, la vinylimidazole,
la vinylpyridine, la vinylamine, l'allylamine, et les monomères ci-après dans lesquels R = H ou méthyl :

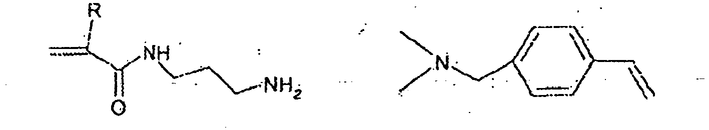

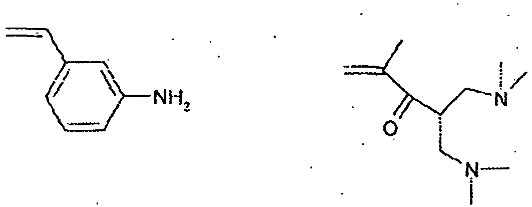

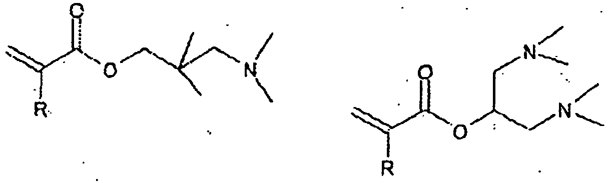

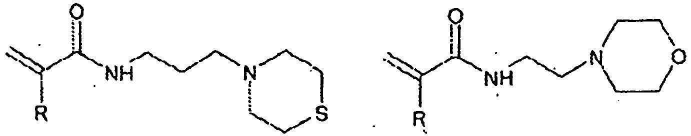

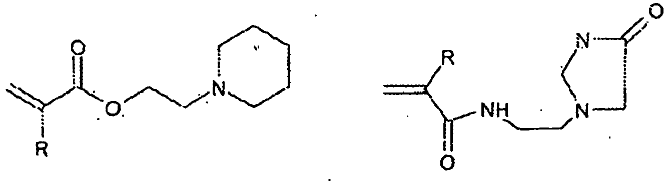

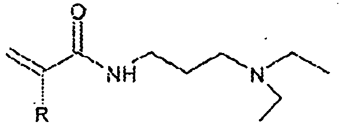

6. Composition selon la revendication 1, dans laquelle le polymère comprend au moins un monomère de formule (II), seul ou en mélange: 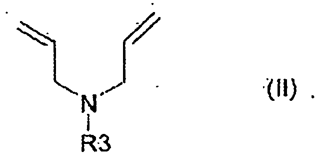
dans laquelle R3 est l'hydrogéne ou un radical carboné, notamment hydrocarboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Cl, Br, Si et P.
étant entendu que le monomère de formule (II) comprend au moins une fonction amine, primaire, secondaire ou tertiaire, protonable à un pH choisi entre pH 1 et pH 12.

7. Composition selon la revendication 6, dans laquelle R3 est choisi parmi:
- un radical alkyle tel que méthyle, éthyle, propyle, n-butyle, isobutyle, tertiobutyle, n-hexyle, n-octyle, n-dodécyle, n-octadécyle, n-tétradécyle, n-docosanyle; comprenant éventuellement 1 à 25 hétéroatomes choisis parmi O, N, S, F, Si et P et/ou éventuellement substitué par au moins un des groupements cités ci-dessus;
- un radical phényle -C₆H₅ éventuellement substitué par un radical alkyle en C1-C12 comprenant éventuellement 1 à 25 hétéroatomes choisis parmi N, O, S, F, Cl, Br, Si et/ou P; et/ou éventuellement substitué par au moins un des groupements cités ci-dessus
- un radical benzyl -C₆H₄-CH₃ éventuellement substitué par un radical alkyle en C1-C12 comprenant éventuellement 1 à 25 hétéroatomés choisis parmi O, N, S, F, Cl, Br, Si et P; et/ou éventuellement substitué par au moins un des groupements cités ci-dessus
- ou un mélange de ces radicaux.

8. Composition selon l'une des revendications 6 à 7. dans laquelle le monomère de formule (II) est choisi parmi, seul ou en mélange la N-méthyldiallylamine et la diallylamine.

9. Composition selon la revendication 1, dans laquelle le polymère comprend au moins un monomère de formule (III), seul ou en mélange : 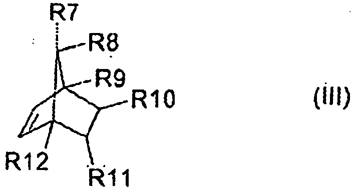 dans laquelle R7 à R12, indépendamment les uns des autres, représentent :
- un atome d'hydrogène,
- un groupement -NR13R'13 avec R13 et R'13, indépendamment l'un de l'autre, représentant l'hydrogène ou un alkyle linéaire, ramifié ou cyclique, saturé ou insaturé, éventuellement aromatique, comprenant de 1 à 18 atomes de carbone, pouvant comprendre 1 à 10 hétéroatomes choisis parmi O, N, S, F, Si et P et notamment R13 et R'13 peuvent être choisis parmi l'hydrogène, un groupement méthyle, éthyle, propyle, isopropyle, n-butyle, t-butyle, isobutyle, octyle, lauryle, stéaryle.
- un radical carboné, notamment hydrocarboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
étant entendu que le monomère de formule (III) comprend au moins une fonction amine, primaire, secondaire ou tertiaire, protonable à un pH choisi entre pH 1 et pH 12.

10. Composition selon la revendication 9, dans laquelle R7 à R12 peuvent être:
- H
- NH2
- un radical alkyle tel que méthyle, éthyle, propyle, n-butyfe, isobutyle, tertiobutyle, n-hexyle, n-octyle, n-dodécyle, n-octadécyle, n-tétradécyle, n-docosanyle; comprenant éventuellement 1 à 25 hétéroatomes choisis parmi O, N, S, F, Si et P, et/ou éventuellement substitué par au moins un des groupements cités ci-dessus
- un radical phényle -C₆H₅ éventuellement substitué parun radical alkyle en C1-C12 comprenant éventuellement 1 à 25 hétéroatomes, choisis parmi N, O, S, F, Si et/ou P; et/ou éventuellement substitué par au moins un des groupements cités ci-dessus
- un radical benzyle -C₆H₄-CH₃ éventuellement substitué par un radical alkyle en C1-C12 comprenant éventuellement 1 à 25 hétéroatomes choisis parmi O, N, S, F, Si et P; et/ou éventuellement substitué par au moins un des groupements cités ci-dessus
- ou un mélange de ces radicaux.

11. Composition selon l'une des revendications 9 à 10, dans laquelle le monomère de formule (III) est choisi parmi, seul ou en mélange

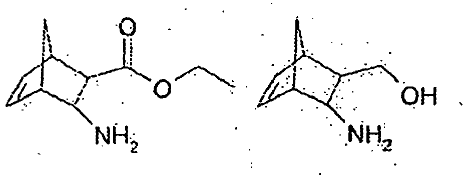

12. Composition selon l'une des revendications précédentes, dans laquelle les monomères de formule (I), (II) et/ou (III), représentent 1 à 100% en poids du poids monomères de formule (I), (II) et/ou (III), représentent 1 à 100% en poids du poids du polymère final, notamment 1 à 99,9% en poids, en particulier 30 à 95% en poids, de préférence de 35 à 90% en poids, voire 40 à 85% en poids.

13. Composition selon l'une des revendications précédentes, dans laquelle le polymère comprend en outre 0,01 à 99% en poids par rapport au poids du polymère final, notamment de 5 à 70% en poids, de préférence de 100 à 65% en poids, voire de 15 à 60% en poids, de monomères additionnels.

14. Composition selon la révendication 13, dans laquelle le monomère additionnel est choisi parmi les monomères suivants, seuls ou en mélange, ainsi que leurs sels :
- (i) les esters de l'acide (méth)acrylique de formule CH₂=CHCOOR'1 ou CH₂=C(CH3)COOR'1 avec R'1 représentant une chaîne carbonée, notamment hydrocarbonée (alkyle), ayant 1 à, 30 atomes de carbone, linéaire, ramifiée ou cyclique, saturée ou insaturée, éventuellement aromatique (aryle, aralkyle ou alkylaryle), comprenant éventuellement 0 à 2 fonctions éthers (-O-) et éventuellement 0 à 12, notamment 1 à 8, fonctions choisies parmi -OH (hydroxy), -OR' avec R' alkyle en C1-C6 (alcoxy), -CN, -X (halogène, notamment Cl, F, Br ou I), -CO-, -SO3H, -COOH-OCOO-, -COO-, -OCONR-, -OCONH-, -NH-CONH-, -NR-CONR-, -CF3, -CN, epoxy, -NHCO-, -N(R)CO- avec R=alkyle linéaire ou ramifié en C1-C22 comprenant éventuellement 1-12 hétéroatomes;
- (ii) les amides de l'acide (méth)acrylique de formule CH₂=CHCONR'2R"2 ou CH₂=C(CH₃)CONR'2R"2 avec R'2, R"2, identiques ou différents, représentant l'hydrogène ou une chaîne carbonée, notamment hydrocarbonée (alkyle), ayant 1 à 30 atomes de carbone, linéaire, ramifiée ou cyclique, saturée ou insaturée, éventuellement aromatique, comprenant éventuellement 0 à 2 fonctions éthers (-O-) et éventuellement 0 à 12, notamment 1 à 8, fonctions choisies parmi -OH (hydroxy), -OR' avec R' alkyle en C1-C6 (alcoxy), -CN, -X (halogène, notamment Cl, F, Br ou 1), -CO-, -SO3H, -COOH, -OCOO-, -COO-, -OCONR-, -OCONH-, -NH-CONH-, -NR-CONR-, -CF3, -CN, epoxy, -NHCO-, -N(R)CO- avec R=alkyle linéaire ou ramifié en C1-C22 comprenant éventuellement 1-12 hétéroatomes;
- (iii) les esters de vinyle de formule CH₂=CH-OCO-R'3 ou CH₂=C(CH₃)-OCO-R'3 avec R'3 représentant une chaîne carbonée, notamment hydrocarbonée, ayant 1 à 30 atomes de carbone, linéaire, ramifiée ou cyclique, saturée ou insaturée, éventuellement aromatique, comprenant éventuellement 0 à 2 fonctions éthers (-O-) et éventuellement 0 à 12, notamment 1 à 8, fonctions choisies parmi -OH (hydroxy), -OR' avec R' alkyle en C1-C6 (alcoxy), -CN, -X (halogène, notamment Cl, F, Br ou I), -CO-, -SO3H, -COOH, -OCOO-, -COO-, -OCONR-, -OCONH-, -NH-CONH-, -NR-CONR-, -CF3, -CN, epoxy, -NHCO-, -N(R)CO- avec R=alkyle linéaire ou ramifié en C1-C22 comprenant éventuellement 1-12 hétéroatomes;
- (iv) les éthers de vinyle de formule CH₂=CHR'4 ou CH₂=C(CH₃)OR'4 avec R'4 représentant une chaîne carbonée, notamment hydrocarbonée, ayant 1 à 30 atomes de carbone, linéaire, ramifiée ou cyclique, saturée ou insaturée, éventuellement aromatique, comprenant éventuellement 0 à 2 fonctions éthers (-O-) et éventuellement 0 à 12, notamment 1 à 8, fonctions choisies parmi -OH (hydroxy), -OR' avec R' alkyle en C1-C6 (alcoxy), -CN, -X (halogène, notamment Cl, F, Br ou I), -CO-, -SO3H, -COOH, -COO-, -COO-, -OCONR-, -OCONH-, -NH-CONH-, -NR-CONR-, -CF3, -CN, epoxy, -NHCO-, -N(R)CO- avec R=alkyle linéaire ou ramifié en C1-C22 comprenant éventuellement 1-12 hétéroatomes;
- (v) les composés vinyliques de formule CHR"5=CR5R'5 dans laquelle :
R"5 est H ou COOH, et
R5 est H, CN ou COOH, et
R'5 est choisi parmi :
- un atome d'hydrogène, ou un groupe choisi parmi -OH, -CH=O, halogène (Cl, Br, I Notamment),
- COOH, -CH2COOH, -NHC(O)H, -N(CH3)-C(O)H, -NHC(O)CH₃,
- N(CH3)-C(O)CH₃;
- un cycle : 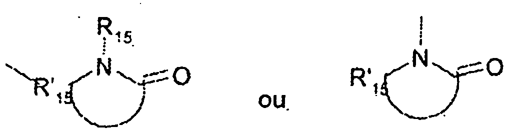 où R'15 et R15 représentent, indépendamment l'un de l'autre, H, un groupe alkyle linéaire ou ramifié, saturé ou insaturé, éventuellement aromatique, cyclique ou non, comprenant à 25 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituant choisis parmi -OH et les atomes d'halogène (Cl, Br, 1 et F);
- un groupe alkyle linéaire ou ramifié, comprenant 1 à atomes de carbone,
- un groupe alkyle linéaire ou ramifié, comprenant, 1 a 25 atomes de carbone.
- un groupe cycloalkyle en C₃ à C₈ tel que cyclohexane.
- un groupe aryle en Ce à C₂₀ tel que phényle,
- un groupe aralkyle en C₇ à C₃₀ (groupe alkyle en C₁ à C₄) tel que 2-phényléthyle ou benzyle,
- un groupe héterocyclique de 4 à 12 chaînons contenant un ou plusieurs hétéroatomes choisis parmi O, N, et S,
- un groupe hétérocycloalkyle (alkyle de 1 à 4 carbones), tel que furfuryle, furfurylméthyle ou tétrahydrofurfurylméthylke,
lesdits groupes alkyles, cycloalkyle, aryle, aralkyle; hétérocyclique ou hétérocycloalkyle pouvant comprenant éventuellement 0 à 2fonctions éthers (-O-) et éventuellement 0 à 12, notamment 1 à 8, fonctions choisies parmi -OH (hydroxy), -OR' avec R' alkyle en C1-C6 (alcoxy), -CN, -X (halogène, notamment Cl, F; Brou I), -CO-, -SO3H, -COOH, -OCOO-, -COO-, -OCONR-, -OCONH-, -NH-CONH-, -NR-CONR-, -CF3, -CN, epoxy, -NHCO-, -N(R)CO- avec R=alkyle linéaire ou ramifié en-C1-C22 comprenant éventuellement 1-12 hétéroatomes;
et/ou pouvant être éventuellement substitué par un ou plusieurs groupes alkyles en C1-C4, linéaires ou ramifiés, eux-mêmes comprenant éventuellement. 0 à 2 fonctions éthers (-O-) et éventuellement 0 à 12, notamment 1 à 8, foncions choisies parmi -OH (hydroxy), -OR' avec R' alkyle en C1-C6 (alcoxy), -CN, -X (halogène, notamment Cl; F, Br ou I), -CO-, -SO3H, -COOH. -OCOO-, -COO-, -OCONH-, -NH-CONH-, -CF3, CN, epoxy, -NHCO-, -N(R)CO- avec R=alkyle linéaire ou ramifié en C1-C22comprenant éventuellement 1-12 hétéroatomes,
- (vii) les monoméres anioniques suivants, et leurs sels :
l'anhydride maléique, l'acid acrylique, l'acide méthacrylique, l'acide crotonique, l'acide itaconique, l'acide fumarique, l'acide maléique, l'acrylate de 2-carboxyéthyle (CH2=CH-C(O)-O-(CH₂)₂-COOH); l'acide styrènesulfonique, l'acide 2-acrylamido-2-methylpropanesulfonique, l'acide vinylbenzoïque, l'acide vinylphosphonique, le (méth)acrylate de sulfopropyle, et parmi les sels: le (méth)acrylate de sodium ou de potassium;
- (viii) les monomères amphotères suivants:
la N,N-dimethyl-N-(2-methacryloyloxyethyl)-N-(3-sulfopropyl) ammonium bétaïne (notamment la SPE de la société Raschig); la N,N-dimethyl-N-(3-methacrylamidopropyl)-N-(3-sulfopropyl) ammonium bétaïne (SPP de Raschig), et la 1-(3-sulfopropyl)-2-vinylpyridinium bétaïne (SPV de Raschig), ainsi que la 2-méthacryloyloxyéthylphosphorylcholine.
- (ix) les monomères de formule : 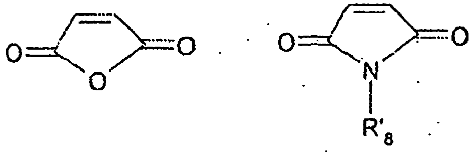 dans lesquelles R'8 est H ou un groupe alkyle linéaire ou ramifié, saturé ou insaturé, éventuellement aromatique, cyclique ou non, comprenant 1 à 25 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi -OH et les atomes d'halogène (Cl, Br, I et F);
- (x) les monomères de formule- (I), (II) et (III) quaternisés, et les formes quaternisées des monomères additionnel ci-dessus.
- (xi) les composés multivalents comprenant au moins deux fonctions polymérisables de type vinylique, (méth) acrylique, allytique, (meth)acrylamide.

15. Composition selon la revendication 14, dans laquelle, le monomère additionnel est choisi parmi, seul ou en mélange ; les (méth)acrylates de méthyle, d'éthyle, de propyle, d'isopropyle, de n-butyle, d'isobutyle, de tertiobutyle, d'hexyle, de cyclohexyle de 2-éthylhexyle, d'octyle, d'isooctyle, d'isodécyle, de décyle, de dodécyle; de myristyle, de cétyle, de palmityle, de stéaryle, de béhényle, d'oléyle, de tridécyle, d'hexadécyle, d'isobornyle; d'hydroxyéthyle, d'hydroxyprppyle; de phényle, de benzoyle, de furfuryl; de tétrahydrofurfuryle, d'éthoxyéthyle, de méthoxyéthyle; de glycérol, de 2,2,2-trifluoroéthyle; de poly(éthylène-isobutylène); les (méth)acrylonitriles; la (méth)acrylamide, la N-méthyl (méth)acrylamide, la N-isopropyle (méth)acrylamide, la N-tertbutyl (méth)acrylamide, la N-octyl, (méth) acrylamide, la N-undécyl(méth)acrylamide, la N,N-diméthyl (méth)acrylamide, la N,N-dibutyl(méth)acryrlamide; l'acétate de vinyle, le propionate de vinyle, le butyrate (ou butanoate) de vinyle, l'éthylhexanoate de vinyle, le néononanoate de vinyle et le néododécanoate de vinyle, le néodécanoate de vinyle, le pivalate de vinyle, le cyclohexanoate de vinyle, le benzoate de vinyle, le 4-tert-butylbenzoate de vinyle, le trifluoroacétate de vinyle; le méthylvinyléther, l'éthylvinyléther, l'éthylhexylvinyléther et le butylvinyléther, le cyclohexylvinyléther, l'isobutylvinyléther; l'alcool vinylique, le vinylcyclohexane; la vinylpyrrolidone, la vinylcaprolactame, la N-vinyl acétamide, la N-vinylformamide, le N-méthyl-N-vinylformamide, la N-vinylacétamide ; la N-méthyl-N-vinylacétamide; le styrène, le méthylstyrène, le 4-tert-butylstyrene, le 4-acétoxystyrène, le 4-methoxystyrene, le 3-méthylstyrene; le 4-méthylstyrene, le 2-chlorostyrene, le 3-chlorostyrene, le 4-chlorostyrene, le diméthylstyrène, le 2,6-dichlorostyrène, le 2,4-diméthylstyrène; le
2,5-diméthylstyrène, le 3,5-éthoxystyrène, le 2,4-éthoxystyrène, le 4-fluorostyrène; le vinyl butyral; le vinyl carbazole: le vinyl chlorure; le vinyl formal; le chlorure de vinylidène, le fluorure de vinylidène, le 2-vinyl naphtalène; le N-méthyl maléimide; le 1-octene, le 1-butene, le cis-chlorobutadiene, le trans-chlorobutadiene, le chlorotrifluoroethylene; le cis-isoprene, le trans-isoprene, le -1-octadecene, le butadiène, l'hexadécène et l'eicosène; l'anhydride maléique et le N-méthyl maléimide; le 1,3-butanediol di(meth)acrylate; le 1,6-hexanediol di(meth)acrylate, le chlorure de N,N'-diméthyldiallylammonium, le chlorure de triéthylammoniuméthyl methacrylate (MADQUAT), le chlorure de 4-methyl, vinyl pyridinium, le chlorure de N-methyl N-vinyl imidazolinium, le chlorure de triméthylammonium propyl (méth) acrylamide.

16. Composition selon l'une des revendications 14 à 15, dans laquelle le monomère additionnel est choisi parmi le vinyl néodécanoate, le terbutylbenzoate de vinyle; la vinylpyrrolidone; la vinylcaprolactame; le N-vinyl formamide: le chlorure de N,N'-dimethyldialtylammonium; le chlorure de triethylammoniumethyl methacrylate: les (méth)acrylates d'éthyle, de méthyle, de tertbutyle, ou d'isobornyle; l'acétate de vinyle, l'acide crotonique; l'acide (méth)acrylique, le methacryoylethylbétaine, l'octylacrylamide; le chlorure de N-methyl N-vinyl imidazolinium, le 1-eicosène, le terbutylacrylamide, l'acrylamide, l'hexadécène, et leurs mélanges.

17. Composition selon l'une des revendications précédentes, dans laquelle le polymère se présente sous la forme d'un homopolymère ou d'un copolymère qui peut être linéaire ou branché, réticulé ou non réticulé; statistique, alterné, à blocs ou à gradient, voire en étoiles; de préférence linéaire, statistique ou à blocs.

18. Composition selon l'une des revendications précédentes, dans laquelle le polymère est choisi parmi :
- les copolymères poly(vinylpyrrolidone-co-dimethylaminoethyl méthacrylate),
- les copolymères poly(vinylcaprolactam-co-vinylpyrrolidone-co-dimethylaminoethylmethacrylate),
- les copolymères poly(vinylpyrrolidone-co-dimethylaminopropylmethacrylamide)
- les copolymères poly(vinylpyrrolidone-co-polyvinylcaprolactam-co-dimethylaminopropylmethacrylamide),
- la poly(vinylamine), la poly(allylamine), la poly(diallylamine);
- les copolymères poly(N-vinyl formamide-co-vinylamine).

19. Composition selon l'une des revendications précédentes, dans laquelle le polymère présente une masse moléculaire moyenne en poids (Mw) comprise entre 1000 et 3 000 000, notamment comprise entre 1500 et 1 000 000 et plus préférentiellement comprise entre 2 000 et 800 000, et encore mieux entre 2500 et 500 000.

20. Composition selon la revendication 1, dans laquelle le polymère comprend au moins 3 unités répétitives choisies, seules ou en mélanges, parmi :

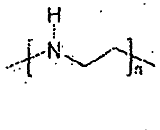

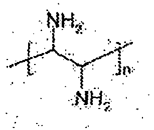

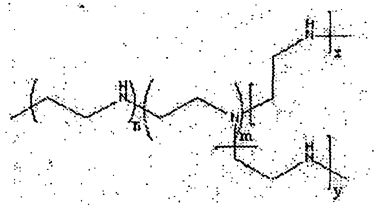

21. Composition selon l'une des revendications précédentes, dans laquelle l'agent neutralisant est ajouté en une quantité de 0,01 à 3 équivalent molaire, notamment 0,05 à 2,5, voire 0,1 à 2 équivalent molaire, par rapport aux fonctions amines totales du polymère ou des monomères.

22. Composition selon l'une des revendications précédentes, dans laquelle l'agent neutralisant est ajouté en une quantité stoechiométrique par rapport aux fonctions amines totales du polymère ou des monomères.

23. Compostion selon l'une des revendications précédentes, dans laquelle l'agent neutralisant présente une masse moléculaire en nombre (Mn) comprise entre 300 et 50000, notamment entre 350 et 20000 et, plus préféré entre 400 et 10000, voire 450 et 5000.

24. Composition selon l'une des revendications précédentes, dans laquelle l'agent neutralisant est linéaire et comporte 2 fonctions acide, une à chaque extrémité de la chaîne.

25. Composition selon l'une des revendications précédentes, dans laquelle l'agent neutralisant est choisi parmi :
- les polymères comprenant au moins trois unités répétitives de type alkylènes glycols pour lesquels le groupe alkyle comprend 2 à 4 atomes de carbone, et leurs combinaisons.

26. Composition selon la revendication 25, dans laquelle l'agent neutralisant est choisi parmi les polymères pour lesquels:
- R représente méthyle, ou -(CH₂)x-COOH; -(CH₂)x-SO₃H; (CH₂)x-PO₃H₂ avec x = 2 à 3; et/ou
- n représente 4 à 30, notamment 5 à 15.

27. Composition selon l'une des revendications précédentes, dans laquelle l'agent neutralisant est choisi parmi :
CH₃-O-(CH₂-CH₂-O-)n-co-(CH₂-CHR-O)m-CH₂-CH₂R₁₀
CH₃-O-(CH₃-CH₂-O-)n-co-(CH₂-CHR-O)m-CH₂-CH₃CH₂R₁₀
R₁₀-(CH₂-CH₂-O-)n-co-(CH₂-CHR-O)m-CH₂-CH₂R₁₀
R₁₀-CH₂O-(CH₂-CH₂-O-)n-co-(CH₂-CHR-O)m-GH2-R₁₀
dans lesquelles le radical R₁₀ est -COOH, -SO₃H ou -PO₃H₂; de préférence COOH; m est 0 à 200 et n est 3 à 1000, notamment 4 à 30, voire 5 à 15.

28. Composition selon l'une des revendications précédentes, dans laquelle l'argent neutralisant est choisi parmi :
CH₃-O-(CH₂-CH₂-R₁₀
CH₃-O-(CH₂-CH₂-O-)nCH₂-CH₂-CH₂R₁₀
R₁₀-(CH₂-CH₂-O-)n-CH₂-CH₂R₁₀
R₁₀-CH₂O-(CH₂-CH₂-O-)n-CH₂-R₁₀
dans lesquelles, le radical R₁₀ est COOH, SO₃H ou -PO₃H₂; de préférence COOH; et n est 3 in 1000, notamment 4 à 30, voire 5 à 15.

29. composition selon l'une des revendications précédentes, dans laquelle l'agent neutralisant est choisi parmi :
- CH₃-O-(CH₂-CH₂-O-)n-CH₂-CH₂-SO₂H;
- CH₃-O-(CH₂-CH₂-O-)n-CH₂-CH₂-CH₂COOH;
- HOCO-(CH₂-CH₂-O-)n-CH₂-CH₂COOH; et
- HOCO-CH₂O-(CH₂-CH₂-O-)n-CH₂-COOH;
avec n = 3 à 1000, notamment 4 à 30, voire 5 à 15.

30. Composition selon l'une des revendications 1 à 29, dans laquelle le polymère est présent à raison de 0,01 à 50% en poids de matière sèche, notamment de 0,1 à 30% en poids, voire de 0,3 à 10% en poids, encore mieux de 1 à 3% en poids, par rapport au poids total de la composition cosmétique ou pharmaceutique.

31. Composition selon la revendication 30, comprenant en outre au moins un constituant choisi parmi l'eau, les solvants organiques hydrophiles comme les alcools et notamment les monoalcools, linéaires ou ramifiés en C1-C6, comme l'éthanol, le tertiobutanol, le n-butanol, l'isopropanol ou le n-propanol, et les polyols comme la glycérine, la diglycérine, le propylène glycols, le sorbitol, le pentylène glycol, et les polyéthylène glycols; les éthers de glycols notamment en C₂ et des aldéhydes en C₂-C₄ hydrophiles; les cires, les corps gras pâteux, les gommes; les solvants organiques lipophiles; les huiles et notamment les huiles hydrocarbonée d'origine animale telles que le perhydrosqualène; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam, les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucaté d'octyl-2-dodécyle, l'isostéarate d'isostéaryle; les esters hydroxyles comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol; et les esters du pentaérythritol; des alcools gras ayant de 12 à 26 atomes de carbone comme l'octyldodécanol, le 2-butyloctanol, le 2-hexyldécanol, le 2-undécylpentadécanol, l'alcool oléique; les huiles fluorées partiellement hydrocarbonées et/ou siliconées; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes; les cétones liquides à température ambiante; les éthers de propylène glycol liquides à température ambiante; les esters à chaîne courte (ayant de 3 à 8 atomes de carbone au total); les éthers liquides à 25°C; les alcanes liquides à 25°C; les composés cycliques aromatiques liquides à 25°C; les aldéhydes liquides à 25°C; les pigments, les nacrés, les charges, les colorants hydrosolubles; les colorants liposolubles les polymères notamment filmogènes; les tensioactifs anioniques, amphotères, non ioniques cationiques ou leurs mélanges; les vitamines, les parfums, les agents nacrants, les épaississants, les gélifiants, les oligo-éléments, les adoucissants, les séquestrants, les parfums, les agents alcalinisants ou acidifiants, les conservateur, les filtres solaires, les tensioactifs, les anti-oxydants, les agents anti-chutes des cheveux les agents anti-pelliculaires, les agents propulseurs, les céramides; les mélanges de ces constituants.

32. Composition selon l'une des revendication 1 à 31, se présenter sous la forme d'un produit de soin et/ou de maquillage de la peau du corps ou du visage, des lèvres et des cheveux, d'un produit solaire ou autobronzant, d'un produit capillaire, notamment pour le maintien de la coiffure ou la mise en forme des cheveux.

33. Compostion selon l'unes des revendication 1 à 32, se présentant sous la forme de shampooing, de gels, lotions de mise en plis, de lotions pour le brushing, de compositions de fixation et de coiffage telles que les laques ou spray, de gels-douche, de bains moussants; d'après-shampooing à rincer ou non, de compositions pour permanente, défrisage, coloration ou décoloration, ou encore sous forme de compositions à rincer, à appliquer avant ou après une coloration, une décoloration une perr-naneri te ou un défrisage ou encore entre les deux étapes d'une permanente ou d'un défrisage; de compositions lavantes pour la peau, et en particulier sous la forme de solutions ou de gels pour le bain ou la douche ou de produits démaquillants; de lotions aqueuses ou hydroalcooliques pour le soin de la peau et/ou des cheveux.

34. Procédé de traitement cosmétique des matières kératiniques telles que la peau du corps ou du visage, des ongles, des cheveux, des cils et/ou des sourcils, **caractérisé en ce qu'**il consiste à appliquer sur lesdites matières kératiniques une composition cosmétique telle que definie à l'une des revendication 1 à 33.

35. Procédé de maintien de la coiffure, de traitement cosmétique, de soin, de lavage, de démaquillage et/ou de maquillage, des matières kératiniques, notamment de la peau du corps ou du visage, des ongles, des cheveux, des cils, des sourcils, **caractérisé en ce qu'**il consiste à appliquer sur lesdites matières kératiniques une composition cosmétique telle que définie à l'une des revendications 1 à 37 ou 41 à 44, puis à effectuer éventuellement un rinçage à l'eau.

36. Polymère éthylénique cationique comprenant au moins une fonction amine primaire, secondaire ou tertiaire, protonable, ladite fonction étant au moins partiellement neutralisée par un agent neutralisant choisi parmi les acides organiques polymériques comprenant au moins une fonction acide carboacylique, sulfonique et/ou phosphonique, ledit polymère au moins partiellement neutralisé étant véhiculable en milieu aqueux, c'est-à-dire hydrosoluble ou hydrodispersible.

37. polymère selon la revendication 36, comprenant au moins un monomère de formule (I), seul ou en mélange : 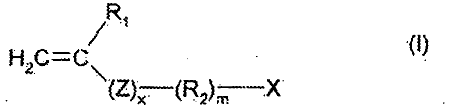 dans laquelle :
- R1 est un atome d'hydrogène ou un radical hydrocarboné, linéaires ou ramifié, de type CₚH₂ₚ₊₁, avec p étant un entier compris entre 1 et 12, inclus;
- Z est und groupement divalent choisi parmi -COO-, -CONH-, -CONCH₃-, -OCO- ou -O-, -SO₂- -CO-O-CO- ou -CO-CH₂-CO-; de préférence, COO et CONH.
- x est 0 ou 1, de préférence 1.
- R2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Cl, Br, Si et P;
- m est 0 ou 1;
- X est
(a) un groupe guanidino, amidino, ou bien
(b) un groupe de formule -N(R₆)(R₇) avec R6 et R7 représentant, indépendamment l'un de l'autre, (i) un atome d'hydrogène, (ii) un groupement alkyle linéaire, ramifié ou cyclique, saturé ou insaturé, éventuellement aromatique, comprenant de 1 à 18 atomes de carbone, pouvant comprendre 1 à 10 hétéroatomes choisis parmi O, N, S, F, Si et P; ou (iii) R6 et R7 forment avec l'atome d'azote un cycle de formule : 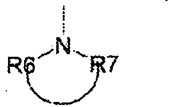 saturé ou insaturé, éventuellement aromatique, comprenant au total 5, 6, 7 ou 8 atomes, et notamment 4, 5 ou 6 atomes de carbone et/ou 2 à 4 hétéroatomes, choisi parmi O, S et N; ledit premier cycle pouvant être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 4 héteroatomes choisi parmi O, S et N.
(c) un cycle : 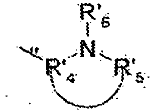
dans lequel R'4 et R'5 forment avec l'atome d'azote un cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant être fusionné avec un ou plusieurs autres cycles, saturés ou insaturés, éventuellement aromatiques, comprenant chacun 5, 6 ou 7 atomes, et notamment 4, 5, 6, 7 ou 8 atomes de carbone et/ou 2 à 4 hétéroatomes choisi parmi O, S et N;
et R'6 est choisi parmi H, -CH₃ et -C₂H₅;
étant entendu que ledit monomère de formule (I) comprend au moins une fonction amine, primaire, secondaire ou tertiaire, protonable à un pH choisi entre pH 1 et pH 12; cette fonction pouvant être, ou être portée par, les radicaux R2 et/ou X.

38. Polymère selon l'une des revendications 36 à 37, dans lequel le monomère de formule (I) est choisi parmi, seul ou en mélange, le (méth)acrylamide de diméthylaminopropyle, le (méth)acrylamide de diméthylaminoéthyle, le (méth)acrylate de diéthylaminoéthyle, le (méth)acrylate de diméthylaminoéthyle, le (méth)acrylate de tert-butylaminoéthyle, le (méth)acrylate de morpholinoéthyle, la vinylimidazole, la vinylpyridine, la vinylamine, l'allylamine, et les monomères ci-après dans lesquels R = H ou méthyl :

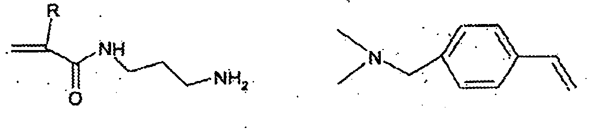

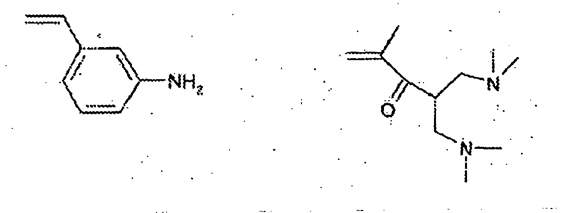

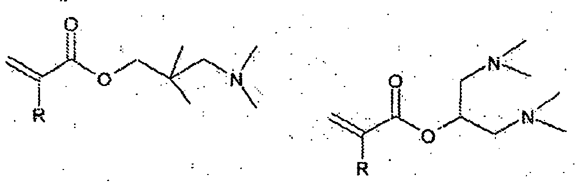

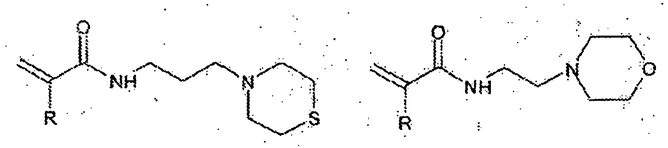

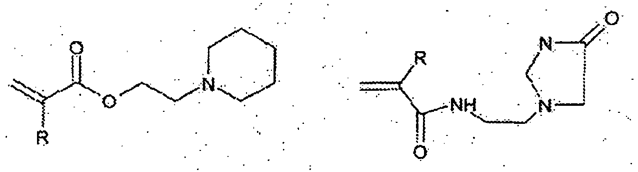

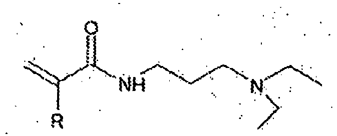

39. Polymère selon la revendication 36, comprenant au moins un monomère de formule (II), seul ou en mélange : 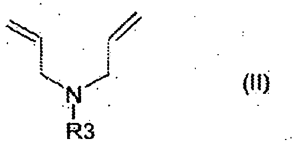 dans laquelle R3 est l'hydrogène ou un radical carboné, notamment hydrocarboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Cl, Br, Si et P.
étant entendu que le monomère de formule (II) comprend au moins une fonction amine, primaire, secondaire ou tertiaire, protonable à un pH choisi entre pH 1 et pH 12.

40. Polymère selon la revendication 39, dans lequel le monomère de formule (II) est choisi parmi, seul ou en mélange la N-méthyldiallylamine et la diallylamine.

41. Polymère selon la revendication 36, comprenant au moins un monomère de formule (III), seul ou en mélange : 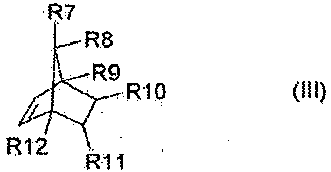 dans laquelle R7 à R12, indépendamment les uns des autres, représentent :
- un atome d'hydrogène,
- un groupement -NR13R'13 avec R13 et R'13, indépendamment l'un de l'autre, représentant l'hydrogène ou un alkyle linéaire, ramifié ou cyclique, saturé ou insaturé, éventuellement aromatique, comprenant de 1 à 18 atomes de carbone, pouvant comprendre 1 à 10 hétéroatomes choisis parmi O, N, S, F, Si et P; et notamment R13 et R'13 peuvent être choisis parmi l'hydrogène, un groupement méthyle, éthyle, propyle, isopropyle, n-butyle, t-butyle, isobutyle, octyle, lauryle, stéaryle.
- un radical carboné, notamment hydrocarboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
étant entendu que le monomère de formule (III) comprend au moins une fonction amine, primaire, secondaire ou tertiaire, protonable à un pH choisi entre pH 1 et pH 12.

42. Polymère selon la revendication 41, dans lequel le monomère de formule (III) est choisi parmi, seul ou en mélange :

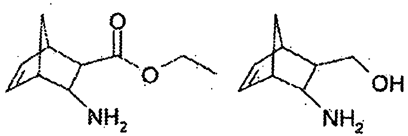

43. Polymère selon l'une des revendications 36 à 42, comprenant en outre 0,01 à 99% en poids par rapport au poids du polymère final, notamment de 5 à 70% en poids, de préférence de 10 à 65% en poids, voire à raison de 15 à 60% en poids, de monomères additionnels.

44. Polymère selon la revendication 43, dans lequel le monomère additionnel est choisi parmi les monomères suivants, seuls ou en mélange, ainsi que leurs sels :
- (i) les esters de l'acide (méth)acrylique de formule CH₂=CHCOOR'1 ou CH₂=C(CH₃)COOR'1 avec R'1 représentant une chaîne carbonée, notamment hydrocarbonée (alkyle), ayant 1 à 30 atomes de carbone, linéaire, ramifiée ou cyclique, saturée ou insaturée, eventuellement aromatique (aryle, aralkyle ou alkylaryle), comprenant éventuellement 0 à 2 fonctions éthers (-O-) et eventuellement 0 à 12, notamment 1 à 8, fonctions choisies parmi -OH (hydroxy), -OR' avec R' alkyle en C1-C6 (alcoxy), -CN, -X (halogène, notamment Cl, F, Br ou I), -CO-,-SO3H, - COOH, -OCOO-, -COO-, -OCONR-, -OCONH-, -NH-CONH-, -NR-CONR-, -CF3, - CN, epoxy, -NHCO-, -N(R)CO- avec R=alkyle linéaire ou ramifié en C1-C22 comprenant éventuellement 1-12 hétéroatomes;
- (ii) les amides de l'acide (méth)acrylique de formule CH₂=CHCONR'2R"2 ou CH₂=C(CH₃)CONR'2R"2 avec R'2, R"2, identiques ou différents, représentant l'hydrogéne ou une chaîne carbonée, notamment hydrocarbonée (alkyle), ayant 1 à 30 atomes de carbone, linéaire ramifiée ou cyclique, saturée ou insaturée, éventuellement aromatique, comprenant éventuellement 0 à 2 fonctions éthers (-O-) et éventuellement 0 à 12, notamment 1 à 8, fonctions choisies parmi -OH (hydroxy) - OR' avec R' alkyle en C1-C6 (alcoxy), -CN, -X (halogène, notamment Cl, F, Br ou I), - CO-, -COOH, -OCOO-, -COO-, -OCONR-, -OCONH-, NR-CONR-, -CF3, -CN, epoxy, -NHCO-, linéaire ou ramifié en C1-C22 comprenant éventuellement 1-12 hétéroatomes;
- (iii) les esters de vinyle de formule CH₂=CH-OCO-R'3 ou CH₂=C(CH₃)-OCO-R'3 avec R'3 représentant une chaîne carboné, notamment hydrocarbonée, ayant 1 à 30 atomes de carbone, linéaire, ramifiée ou cyclique, saturée ou insaturée, éventuellement aromatique, comprenant éventuellement 0 à 2 fonctions éthers (-O-) et éventuellement 0 à 12, notamment 1 à 8, fonctions choisies parmi -OH (hydroxy), - OR' avec R' alkyle en C1-C6 (alkoxy), -CN, -X (halogène, notamment Cl, F, Br ou I), - CO-, -SO3H, -COOH, -OCOO-, -COO-, OCONR-, OCONH, -NH-CONH-,-NR-CONR-, -CF3, -CN, epoxy, -NHCO-, -N(R)CO- avec R=alkyle linéaire ou ramifié en C1-C22 comprenant éventuellement 1-12 hétéroatomes;
- (iv) les éthers de vinyle de formule CH₂=CHOR'4 ou CH₂=C(CH₃)OR'4 avec R'4 représentant une chaîne carbonée, notamment hydrocarbonée, ayant 1 à 30 atomes de carbone, linéaire, ramifieé ou cyclique, saturée ou insaturée, éventuellement aromatique, comprenant éventuellement 0 à 2 fonctions éthers (-O-) et éventuellement 0 à 12, notamment 1 à 8, fonctions choisies parmi -OH (hydroxy), - OR' avec R' alkyl en C1-C6 (alcoxy), -CN, -X (halogène, notamment Cl, F, Br ou I),-CO-, -SO3H, -COOH, -OCOO-, -COO-, -OCONR-, -OCONH-, -NH-CONH-, -NR-CON-, -CF3, -CN, epoxy, -NHCO-, -N(R)CO- avec R=alkyle linéaire ou ramifié en C1-C22 comprenant éventuellement 1-12 hétéroatomes;
- (v) les composés vinyliques de formule CHR"5=CR5R'5 dans laquelle :
R"5 est H ou COOH, et
R5 est H, CN ou COOH, et
R'5 est choisi parmi:
- un atome d'hydrogène, ou un groupe choisi parmi -OH, -CH=O, halogène (Cl, Br, I notamment), -COOH, -CH2COOH, -NHC(O)H, -N(CH3)-C(O)H, -NHC(O)CH₃,
- N(CH3)-C(O)CH₃;
- un cycle : 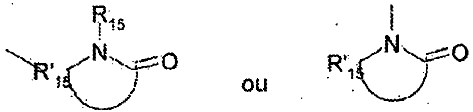 où R'15 et R15 représentent, indépendamment l'un de l'autre, H, un groupe alkyle linéaire ou ramifié, saturé ou insaturé, éventuellement aromatique, cyclique ou non, comprenant 1 à 25 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi -OH et les atomes d'halogène (Cl, Br, I et F);
- un groupe alkyle linéaire ou ramifié, comprenant 1 à 25 atomes de carbone,
- un groupe alkyle linéaire ou ramifié, comprenant 1 à 25 atomes de carbone,
- un groupe cycloalkyle en C₃ à C₈ tel que cyclohexane,
- un groupe aryle en C₆ à C₂₀ tel que phényle,
- un groupe aralkyle en C₇ à C₃₀ (groupe alkyle en C₁ à C₄) tel que 2-phényléthyle ou benzyle,
- un groupe hétérocyclique de 4 à 12 chaînons contenant un ou plusieurs hétéroatomes choisis parmi O, N, et S,
- un groupe hétérocycloalkyle (alkyle de 1 à 4 carbones), tel que furfuryle, furfurylméthyle ou tétrahydrofurfurylméthyle,
lesdits groupes alkyles, cycloalkyle, aryle, aralkyle, hétérocyclique ou hétérocycloalkyle pouvant comprenant éventuellement 0 à 2 fonctions éthers (-O-) et éventuellement 0 à 12, notamment 1 à 8, fonctions choisies parmi -OH (hydroxy), - OR' avec R' alkyle en C1-C6 (alcoxy), -CN, -X (halogéne, notamment Ci, F, Br ou I),-CO-, -SO3H, -COOH, -COO-, -COO-, -OCONR-, -OCONH-, -NH-CONH-, -NR-CONR-, -CF3, -CN, epoxy, -NHCO-, -N(R)CO- avec R=alkyle linéaire ou ramifié en C1-C22 comprenant éventuellement 1-12 hétéroatomes;
et/ou pouvant être éventuellement substitué par un ou plusieurs groupes alkyles en C1-C4, linéaires ou ramifiés, eux-mêmes comprenant éventuellement 0 à 2 fonctions éthers (-O-) et éventuellement 0 à 12, notamment 1 à 8, fonctions choisies parmi -OH (hydroxy), -OR' avec R' alkyle en C1-C6 (alcoxy), -CN, -X (halogène, notamment Cl, F, Br ou I), -CO-, -S03H, -COOH, -OCOO-, -COO-,-OCONH-, -NH-CONH-, -CF3, - CN, epoxy, -NHCO-, -N(R)CO- avec R=alkyle linéaire ou ramifié en C1-C22 comprenant éventuellement 1-12 hétéroatomes.
- (vii) les monomères anioniques suivants, et leurs sels :
l'anhydride maléique, l'acide acrylique, l'acide méthacrylique, l'acide crotonique, l'acide itaconique, l'acide fumarique, l'acide maléique, l'acrylate de 2-carboxyéthyle (CH2=CH-C(O)-O-(CH₂)₂-COOH); l'acide styrènesulfonique, l'acide 2-acrylamido-2-methylpropanesulfonique, l'acide vinylbenzoïque, l'acide vinylphosphonique, le (méth)acrylate de sulfopropyle, et parmi les sels: le (méth)acrylate de sodium ou de potassium;
- (viii) les monomères amphotères suivants :
la N,N-dimethyl-N-(2-methacryloyloxyethyl)-N-(3-sulfopropyl) ammonium bétaïne (notamment la SPE de la société Raschig); la N,N-dimethyl-N-(3-methacrylamidopropyl)-N-(3-sulfopropyl) ammonium bétaïne (SPP de Raschig), et la 1-(3-sulfopropyl)-2-vinylpyridinium bétaïne (SPV de Raschig), ainsi que la 2-méthacryloyloxyéthyl phosphorylcholine
- (ix) les monomères de formule : 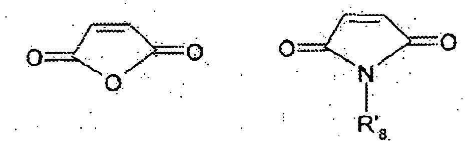 dans lesquelles R'8 est H ou un groupe alkyle linéaire ou ramifié, saturé ou insaturé, éventuellement aromatique, cyclique ou non, comprenant 1 à 25 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi -OH et les atomes d'halogène (Cl, Br, I et F);
- (x) les monomères de formule (I), (II) et (III) quaternisés, et les formes quaternisées des monomères additionnels ci-dessus.
- (xi) les composés multivalents comprenant au moins deux fonctions polymérisables de type vinylique, (méth)acrylique, allylique, (meth)acrylamide.

45. Polymère selon la revendication ,44, dans lequel le monomère additionnel est choisi parmi le vinyl néodécanoate" le terbutylbenzoate de vinyle; la vinylpyrrolidone:
la vinylcaprolactame; le N-vinylformamide; le chlorure de N,N'-dimethyldiallylammonium; le chlorure de triethylammoniumethyl methacrylate (MADQUAT); les (méth)acrylates d'éthyle, de méthyle, de tertbutyle, ou d'isobornyle; l'acétate de vinyle, l'acide crotonique; l'acide (méth)acrylique, le methacryoylethylbétaine, l'octylacrylamide; le chlorure de N-methyl N-vinyl imidazolinium, le 1-eioosène, le terbutylacrylamide, l'acrylamide, l'hexadécène, et leurs mélanges.

46. Polymère selon l'une des revendications 36 à 45, choisi parmi :
- les copolyméres poly(vinylpyrrolidone-co-dimethylamioethyl méthacrylate),
- les copolymères poly(vinylcaprolactam-co-vinylpyrrolidone-co-dimethylaminoethylmethacrylate),
- les copolymères poly(vinylpyrrolidone-co-dimethylaminopropylmethacrylamide)
- les copolymères poly(vinylpyrrolidone-co-polyvinylcaprolactam-co-diméthylaminopropylmethacrylamide)
- la poly(vinylamine), la poly(allylamine), la poly(diallylamine);
- les copolymères poly(N-vinyl formamide-co-vinylamine)

47. Polymère selon la révendication 36, comprenant au moins 3 unités répétitives choisies, seules ou en mélanges parmi :

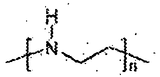

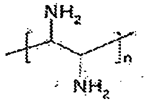

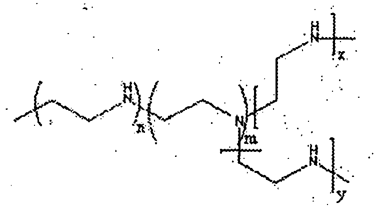

48. Polymère selon l'une des revendications 36 à 47, dans lequel l'agent neutralisant est ajouté en une quantité de 0,01 à 3 équivalent molaire, notamment 0,05 à 2,5, voire. 0,1 à 2 équivalent molaire, par rapports aux fonctions amines totales du polymère ou des monomères.

49. Polymère selon l'une des revendications 36 à 48, dans lequel l'agent neutralisant est choisi parmi :
- (i) les polymères comportant des unités répétitives de dextran comprenant au moins une fonction acide, notamment carboxylique, en bout de chaîne
- (ii) les poly(alkyloxazolines) terminées acides, de préférence la polyméthyloxazoline et la polyéthyloxazoline comprenant au moins une fonction acide.
- (iii) les poly(N-methyl) sarcosines fonctionnalisées acide, notamment carboxylique;
- (iv) les dendrimères ou molécules hyperbranchées fonctionnalisées en surface par des groupements acides, notamment carboxyliques; telles que par exemple les PAMAM starburst de Dow Chemical;
- (v) les polymères hyperbranchés de type polyesters obtenu par réaction entre l'acide adipique et le glycérol,
- (vi) les poly(glycérol) poly(hydroxyéthyl acrylate) et poly(vinylpyrrolidone) terminées acides, notamment carboxyliques,
- (vii) les polymères comprenant au moins trois unités répétitives de type alkylènes glycols pour lesquels le groupe alkyle comprend 2 à 4 atomes de carbone, et leurs combinaisons; on peut citer les polyéthylène glycol, les polypropylène glycol, les polyéhylène-co-propylèneglycol (PEG/PPO), les potytétraméthylène oxyde-co-polyéthylène glycol (PTMO/PEG); étant entendu qu'ils comportent en outre au moins une fonction acide (notamment carboxylique, sulfonique, et/ou phosphonique);

50. Polymère selon l'une des revendications 36 à 49, dans lequel l'agent neutralisant est un polymère qui comprend au moins trois unités répétitives de type alkylèneglycol, préférentiellement de type éthylène glycol: (-CH₂-CH₂-O-).

51. Polymère selon l'une des revendications 36 à 50, dans lequel l'agent neutralisant est choisi parmi les polymères de structures suivantes:
CH₃-O-(CH₂-CH₂-O-)n-co-(CH₂-CHR-O)m-CH₂-R₁₀
CH₃-O-(CH₃-CH₂-O-)n-b-(CH₂-CHR-O)m-CH₂-R₁₀
CH₃-O-(CH₂-CH₂-O-)n-co-(CH₂-CHR-O)m-CH₂-CH₂R₁₀
CH₃-O-(CH₂-CH₂-O-)n-b-(CH₂-CHR-O)m-CH₂-CH₂R₁₀
CH₃-O-(CH₂-CH₂-O-)n-co-(CH₂-CHR-O)m-CH₂-CH₂CH₂R₁₀
CH₃-O-(CH₂-CH₂₋O-)n-b-(CH₂-CHR-O)m-CH₂-CH₂CH₂R₁₀
CH₃-O-(CH₂-CH₂-O-)n-co-(CH₂-CHR-O)m-CH₂-CH₂OCO-CH₂-CH₂COOH
CH₃-O-(CH2-CH₂-O-)n-b-(CH₂-CHR-O)m-CH₂-CH₂OCO-CH₂-CH₂COOH
R₁₀-(CH₂-CH₂-O-)n-co-(CH₂-CHR-O)m-CH₂-CH₂R₁₀
R₁₀-(CH₂-CH₂-O-)n-b-(CH₂-CHR-O)m-CH₂-CH₂R₁₀
R₁₀-CH₂O-(CH₂-CH₂-O)n-CO-(CH₂-CHR-O)M-CH₂R₁₀
R₁₀-CH₂O-(CH₂-CH₂-O-)n-b-(CH₂-CHR-O)m-CH₂-R₁₀
R₁₀-CH₂-CH₂CH₂-O-(CH₂-CH₂-O-)n-co-(CH₂-CHR-O)m-CH₂-CH₂-CH₂-R₁₀R₁₀-CH₂-CH₂-CH₂-O-(CH₂-CH₂-O-)n-co-(CH₂CHR-O)m-CH₂-CH₂
R₁₀-CH₂-CH₂-CH₂-O-(CH₂-CH₂-O-)n-b(CH₂-CHR-O)m-CH₂-CH₂-CH₂-R₁₀
R₁₀-CH₂-CH₂-COO-(CH₂-CH₂-O-)n-co-(CH₂-CHR-O)m-CH₂-CH₂-O-CO-CH₂-CH₂-R₁₀
R₁₀-CH₂-CH₂-COO-(CH₂-CH₂-O-)n-b-(CH₂-CHR-O)m-CH₂-CH₂-O-CO-CH₂-CH₂-R₁₀
R₁₀-CH₂-CH₂-CONH-(CH₂-CH₂-O-)n-co-(CH₂-CHR-O)m-CH₂-CH₂-NH-CO-CH₂-
R₁₀-CH₂-CH₂-CONH-(CH₂-CH₂-O-)n-b(CH₂-CHR-O-)m-CH₂-CH₂-NH-CO-CH₂-CH₂-R₁₀
dans lesquelles:
- le radical R, identique ou différent, repréente -CH₃, -(CH₂)x-COOH-(CH₂)x-le radical R, identique ou different, représente -CH₃, -CH₂)xCOOH-(CH₂)x-S03H, (CH₂)x-PO₃H₂; avec x =1 à 8;
- le radical R₁₀, identique ou différent, est -COOH, -SO₃H ou -PO₃H₂;
- n est un entier compris entre 3 et 1000;
- m est un entier compris entre 0 et 200.
étant donné que le ratio n:m est supérieur à 5 quand m est nul.

52. Polymère selon l'une des revendications 36 à 51, dans lequel l'agent neutralisant est choisi parmi:
CH₃-O-(CH₂-CH₂-O-)n-CH₂-CH₂-R₁₀
CH₃-O-(CH₂-CH₂-O-)n-CH₂-CH₂-CH₂R₁₀
R₁₀-(CH₂-CH₂-O-)n-CH₂-CH₂R₁₀
R₁₀-CH₂O-(CH₂-(CH₂-CH₂-CH₂-O-)n-CH₂-R₁₀
dans lesquelles le radical R₁₀ est COOH; SO₃H ou -PO₃H₂ de préférence COOH; et n'est 3 à 1000, notamment 4 à 30 voire à 15.

## Patentansprüche

1. Kosmetische oder pharmazeutische Zusammensetzung, umfassend in einem physiologisch unbedenklichen, insbesondere kosmetisch oder pharmazeutisch unbedenklichen, Medium ein kationisches ethylenisches Polymer mit mindestens einer protonierbaren primären, sekundären oder tertiären Aminfunktion, die zumindest teilweise durch ein Neutralisationsmittel neutralisiert ist, **dadurch gekennzeichnet, daß** es sich bei dem Neutralisationsmittel um eine polymere organische Säure mit mindestens einer Carbonsäure-, Sulfonsäure- oder Phosphonsäurefunktion handelt, die unter
- Polymeren mit Dextran-Wiederholungseinheiten mit mindestens einer Säurefunktion, insbesondere Carbonsäurefunktion, am Kettenende;
- Dendrimeren oder hyperverzweigten Molekülen, die durch Säuregruppen, insbesondere Carbonsäuregruppen, oberflächenfunktionalisiert sind;
- Polymeren mit mindestens drei Wiederholungseinheiten vom Alkylenglykol-Typ mit 2 bis 4 Kohlenstoffatomen in der Alkylgruppe und Kombinationen davon; mit der Maßgabe, daß sie außerdem mindestens eine Carbonsäure-, Sulfonsäure- oder Phosphonsäurefunktion umfassen; ausgewählt unter Polymeren mit den folgenden Strukturen:
CH₃-O-(CH₂-CH₂-O-)ₙ-CO-(CH₂-CHR-O)ₘ-CH₂-R₁₀
CH₃-O- (CH₂-CH₂-O-)ₙ-b-(CH₂-CHR-O)ₘ-CH₂-R₁₀
CH₃-O-(CH₂-CH₂-O-)ₙ-CO-(CH₂-CHR-O)ₘ-CH₂-CH₂R₁₀
CH₃-O-(CH₂-CH₂-O-)ₙ-b-(CH₂-CHR-O)ₘ-CH₂-CH₂R₁₀
CH₃-O-(CH₂-CH₂-O-)ₙ-CO-(CH₂-CHR-O)ₘ-CH₂-CH₂CH₂R₁₀
CH₃-O-(CH₂-CH₂-O-)ₙ-b-(CH₂-CHR-O)ₘ-CH₂-CH₂CH₂R₁₀
CH₃-O-(CH₂-CH₂-O-)ₙ-CO-(CH₂-CHR-O)ₘ-CH₂-CH₂OCO-CH₂-CH₂COOH
CH₃-O-(CH₂-CH₂-O-)ₙ-b-(CH₂-CHR-O)ₘ-CH₂-CH₂OCO-CH₂-CH₂COOH
R₁₀-(CH₂-CH₂-O-)ₙ-CO-(CH₂-CHR-O)ₘ-CH₂-CH₂ R₁₀
R₁₀-(CH₂-CH₂-O-)ₙ-b-(CH₂-CHR-O)ₘ-CH₂-CH₂R₁₀
R₁₀-CH₂O-(CH₂-CH₂-O-)ₙ-CO-(CH₂-CHR-O)ₘ-CH₂-R₁₀
R₁₀-CH₂O-(CH₂-CH₂-O-)ₙ-b-(CH₂-CHR-O)ₘ-CH₂-R₁₀
R₁₀-CH₂-CH₂-CH₂-O-(CH₂-CH₂-O-)ₙ-CO-(CH₂-CHR-O)ₘ-CH₂-CH₂-CH₂-R₁₀
R₁₀-CH₂-CH₂-CH₂-O-(CH₂-CH₂-O-)ₙ-b-(CH₂-CHR-O)ₘ-CH₂-CH₂-CH₂-R₁₀
R₁₀-CH₂-CH₂-COO-(CH₂-CH₂-O-)ₙ-CO-(CH₂-CHR-O)ₘ-CH₂-CH₂-O-CO-CH₂-CH₂-R₁₀
R₁₀-CH₂-CH₂-COO-(CH₂-CH₂-O-)ₙ-b-(CH₂-CHR-O)ₘ-CH₂-CH₂-O-CO-CH₂-CH₂-R₁₀
R₁₀-CH₂-CH₂-CONH-(CH₂-CH₂-O-)ₙ-CO-(CH₂-CHR-O)ₘ-CH₂-CH₂-NH-CO-CH₂-CH₂-R₁₀
R₁₀-CH₂-CH₂-CONH-(CH₂-CH₂-O-)ₙ-b-(CH₂-CHR-O)ₘ-CH₂-CH₂-NH-CO-CH₂-CH₂-R₁₀
worin:
- der Rest R gleich oder verschieden ist und für
- CH₃, -(CH₂)ₓ-COOH; -(CH₂)ₓ-SO₃H oder (CH₂)ₓ-PO₃H₂ mit x = 1 bis 8 steht;
- der Rest R₁₀ gleich oder verschieden ist und für
- COOH, -SO₃H oder -PO₃H₂ steht;
- n für eine ganze Zahl zwischen 3 und 1000 steht und
- m für eine ganze Zahl zwischen 0 und 200 steht; mit der Maßgabe, daß das Verhältnis n:m größer 5 ist, wenn m nicht gleich null ist,
ausgewählt ist.

2. Zusammensetzung nach Anspruch 1, in der das Polymer mindestens ein Monomer der Formel (I) für sich alleine oder im Gemisch umfaßt: 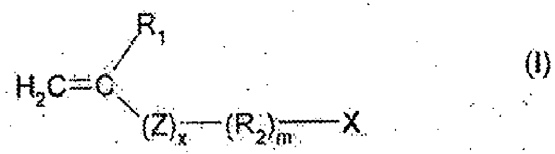 worin:
- R1 für ein Wasserstoffatom oder einen linearen oder verzweigten Kohlenwasserstoffrest vom Typ CₚH₂ₚ₊₁, wobei p für eine ganze Zahl zwischen 1 und 12 inklusive steht, steht;
- Z für eine unter -COO-, -CONH-, -CONCH₃-, -OCO- oder -O-, -SO₂-, -CO-O-CO- oder -CO-CH₂-CO-, vorzugsweise COO und CONH, ausgewählte zweiwertige Gruppe steht;
- x für 0 oder 1, vorzugsweise 1, steht;
- R2 für einen gesättigten oder ungesättigten, gegebenenfalls aromatischen, linearen, verzweigten oder cyclischen zweiwertigen Kohlenstoffrest mit 1 bis 30 Kohlenstoffatomen, der 1 bis 18 unter O, N, S, F, Cl, Br, Si und P ausgewählte Heteroatome umfassen kann, steht;
- m für 0 oder 1 steht;
- X für:
(a) eine Guanidino- oder Amidinogruppe oder auch
(b) eine Gruppe der Formel -N(R₆) (R₇) steht, wobei R6 und R7 unabhängig voneinander für (i) ein Wasserstoffatom oder (ii) eine lineare, verzweigte oder cyclische, gesättigte oder ungesättigte, gegebenenfalls aromatische Alkylgruppe mit 1 bis 18 Kohlenstoffatomen, die 1 bis 10 unter O, N, S, F, Si und P ausgewählte Heteroatome umfassen kann, stehen; oder (iii) R6 und R7 mit dem Stickstoffatom einen Ring der Formel: 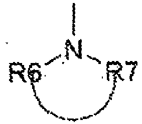 bilden, der gesättigt oder ungesättigt, gegebenenfalls aromatisch, ist und insgesamt 5, 6, 7 oder 8 Atome und insbesondere 4, 5 oder 6 Kohlenstoffatome und/oder 2 bis 4 unter O, S und N ausgewählte Heteroatome umfaßt; wobei der erste Ring mit einem oder mehreren weiteren gesättigten oder ungesättigten, gegebenenfalls aromatischen Ringen mit jeweils 5, 6 oder 7 Atomen und insbesondere 4, 5, 6 oder 7 Kohlenstoffatomen und/oder 2 bis 4 unter O, S und N ausgewählten Heteroatomen anelliert sein kann;
(c) einen Ring: 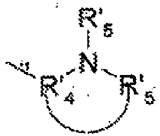
worin R'4 und R'5 mit dem Stickstoffatom einen gesättigten oder ungesättigten, gegebenenfalls aromatischen Ring mit insgesamt 5, 6, 7 oder 8 Atomen und insbesondere 4, 5 oder 6 Kohlenstoffatomen und/oder 2 bis 4 unter O, S und N ausgewählten Heteroatomen bilden; wobei der Ring mit einem oder mehreren weiteren gesättigten oder ungesättigten, gegebenenfalls aromatischen Ringen mit jeweils 5, 6 oder 7 Atomen und insbesondere 4, 5, 6, 7 oder 8 Kohlenstoffatomen und/oder 2 bis 4 unter O, S und N ausgewählten Heteroatomen anelliert sein kann; und
R'6 unter H, -CH₃ und -C₂H₅ ausgewählt ist;
steht;
mit der Maßgabe, daß das Monomer der Formel (I) mindestens eine bei einem pH-Wert zwischen pH 1 und pH 12 protonierbare primäre, sekundäre oder tertiäre Aminfunktion umfaßt; wobei es sich bei dieser Funktion um die Reste R2 und/oder X handeln kann oder diese Funktion von den Resten R2 und/oder X getragen werden kann.

3. Zusammensetzung nach Anspruch 2, in der in dem Monomer der Formel (I) R2:
- einen Alkylenrest wie Methylen, Ethylen, Propylen, n-Butylen, Isobutylen, tert.-Butylen, n-Hexylen, n-Octylen, n-Dodecylen, n-Octadecylen, n-Tetradecylen, n-Docosanylen;
- einen (ortho-, meta- oder para-)-C₆H₄-Phenylenrest, der gegebenenfalls durch einen C₁-C₁₂-Alkylrest substituiert ist, der gegebenenfalls 1 bis 25 unter N, O, S, F, Si und/oder P ausgewählte Heteroatome umfaßt; oder auch einen -C₆H₄-CH₂-Benzylenrest, der gegebenenfalls durch einen C₁-C₁₂-Alkylrest substituiert ist, der gegebenenfalls 1 bis 25 unter O, N, S, F, Si und P ausgewählte Heteroatome umfaßt;
- einen Rest der Formel -CO-, -O-CO-O-, -CO-O-, -O-, -O-CO-NH-, -CO-NH-, -NHCO-, -N(R')CO-, -NH-CO-NH-, -NR'-, Epoxid oder -N-CO- steht, wobei R' für eine lineares oder verzweigtes C₁-C₂₂-Alkyl, das gegebenenfalls 1 bis 12 unter O, N, S, F, Si und P ausgewählte Heteroatome umfaßt, steht;
- oder ein Gemisch dieser Reste
bedeutet oder umfaßt.

4. Zusammensetzung nach einem der Ansprüche 2 bis 3, in der es sich bei dem Monomer der Formel (I) bei X um einen aromatischen oder nichtaromatischen Heterocyclus mit einem sekundären oder tertiären Stickstoff, insbesondere einen Rest vom Indolyl-, Isoindolinyl-, Imidazolyl-, Imidazolinyl-, Piperidinyl-, Pyrazolinyl-, Pyrazolyl-, Chinolin-, Pyridinyl-, Piperazinyl-, Pyrrolidinyl-, Chinidinyl-, Thiazolinyl-, Morpholin-, Guanidino- oder Amidino-Typ, und Gemische davon handelt.

5. Zusammensetzung nach einem der Ansprüche 2 bis 4, in der das Monomer der Formel (I) unter Dimethylaminopropyl(meth)acrylamid, Dimethylaminoethyl(meth)acrylamid, Diethylaminoethyl(meth)-acrylat, Dimethylaminoethyl(meth)acrylat, *tert.-*Butylaminoethyl(meth)acrylat, Morpholinoethyl-(meth)acrylat, Vinylimidazol, Vinylpyridin, Vinylamin, Allylamin und den nachstehenden Monomeren, in denen R = H oder Methyl, für sich alleine oder im Gemisch ausgewählt ist:

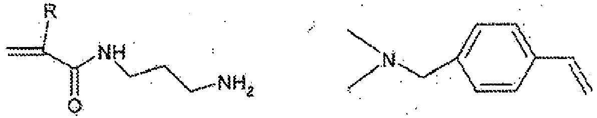

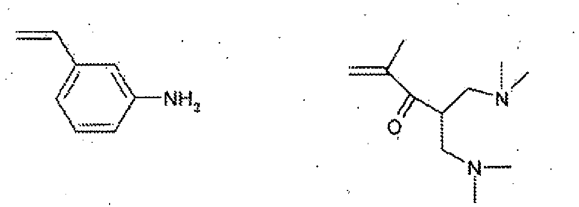

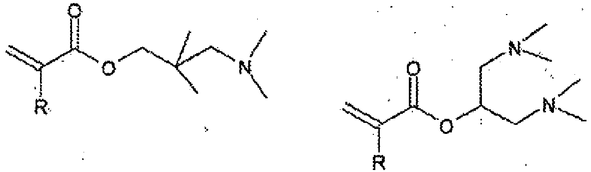

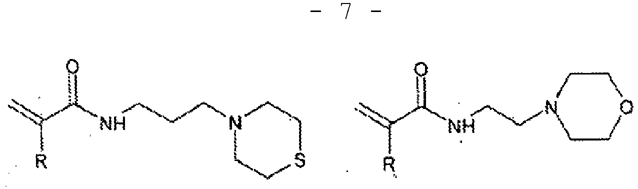

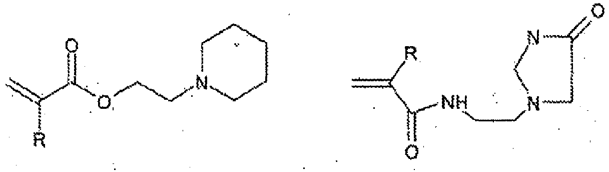

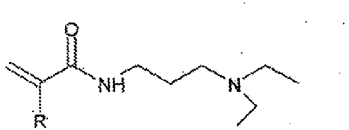

6. Zusammensetzung nach Anspruch 1, in der das Polymer mindestens ein Monomer der Formel (II) für sich alleine oder im Gemisch umfaßt: 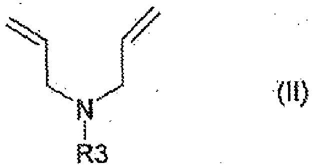
worin R3 für Wasserstoff oder einen linearen, verzweigten oder cyclischen, gesättigten oder ungesättigten, gegebenenfalls aromatischen Kohlenstoffrest, insbesondere Kohlenwasserstoffrest, mit 1 bis 30 Kohlenstoffatomen, der 1 bis 18 unter O, N, S, F, Cl, Br, Si und P ausgewählte Heteroatome umfassen kann, steht;
mit der Maßgabe, daß das Monomer der Formel (II) mindestens eine bei einem pH-Wert zwischen pH 1 und pH 12 protonierbare primäre, sekundäre oder tertiäre Aminfunktion umfaßt.

7. Zusammensetzung nach Anspruch 6, in der R3 unter:
- einem Alkylrest wie Methyl, Ethyl, Propyl, n-Butyl, Isobutyl, *tert*.-Butyl, n-Hexyl, n-Octyl, n-Dodecyl, n-Octadecyl, n-Tetradecyl, n-Docosanyl, der gegebenenfalls 1 bis 25 unter O, N, S, F, Si und P ausgewählte Heteroatome umfaßt und/oder gegebenenfalls durch mindestens eine der oben aufgeführten Gruppen substituiert ist;
- einem -C₆H₅-Phenylrest, der gegebenenfalls durch einen C₁-C₁₂-Alkylrest substituiert ist, der gegebenenfalls 1 bis 25 unter N, O, S, F, Cl, Br, Si und/oder P ausgewählte Heteroatome umfaßt und/oder gegebenenfalls durch mindestens eine der oben aufgeführten Gruppen substituiert ist;
- einem -C₆H₄-CH₃-Benzylrest, der gegebenenfalls durch einen C₁-C₁₂-Alkylrest substituiert ist, der gegebenenfalls 1 bis 25 unter O, N, S, F, Cl, Br, Si und P ausgewählte Heteroatome umfaßt und/oder gegebenenfalls durch mindestens eine der oben aufgeführten Gruppen substituiert ist;
- oder einem Gemisch dieser Reste
ausgewählt ist.

8. Zusammensetzung nach Anspruch 1, in der das Monomer der Formel (II) unter N-Methyldiallylamin und Diallylamin für sich alleine oder im Gemisch ausgewählt ist.

9. Zusammensetzung nach Anspruch 1, in der das Polymer mindestens ein Monomer der Formel (III) für sich alleine oder im Gemisch umfaßt: 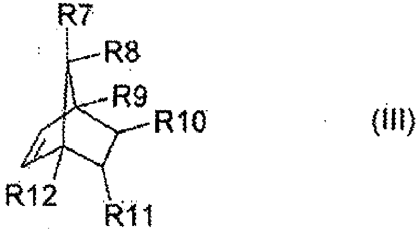 worin R7 bis R12 unabhängig voneinander für
- ein Wasserstoffatom,
- eine -NR13R'13-Gruppe, wobei R13 und R'13 unabhängig voneinander für Wasserstoff oder eine lineare, verzweigte oder cyclische, gesättigte oder ungesättigte, gegebenenfalls aromatische Alkylgruppe mit 1 bis 18 Kohlenstoffatomen, die 1 bis 10 unter O, N, S, F, Si und P ausgewählte Heteroatome umfassen kann, stehen und R13 und R'13 insbesondere unter Wasserstoff und einer Methyl-, Ethyl-, Propyl-, Isopropyl-, n-Butyl-, t-Butyl-, Isobutyl-, Octyl-, Lauryl- oder Stearylgruppe ausgewählt sein können;
- einen linearen, verzweigten oder cyclischen, gesättigten oder ungesättigten, gegebenenfalls aromatischen Kohlenstoffrest, insbesondere Kohlenwasserstoffrest, mit 1 bis 30 Kohlenstoffatomen, der 1 bis 18 unter O, N, S, F, Si und P ausgewählte Heteroatome umfassen kann,
stehen;
mit der Maßgabe, daß das Monomer der Formel (III) mindestens eine bei einem pH-Wert zwischen pH 1 und pH 12 protonierbare primäre, sekundäre oder tertiäre Aminfunktion umfaßt.

10. Zusammensetzung nach Anspruch 9, worin R7 bis R12 für
- H;
- NH₂;
- einen Alkylrest wie Methyl, Ethyl, Propyl, n-Butyl, Isobutyl, *tert*.-Butyl, n-Hexyl, n-Octyl, n-Dodecyl, n-Octadecyl, n-Tetradecyl, n-Docosanyl, der gegebenenfalls 1 bis 25 unter O, N, S, F, Si und P ausgewählte Heteroatome umfaßt und/oder gegebenenfalls durch mindestens eine der oben aufgeführten Gruppen substituiert ist;
- einen -C₆H₅-Phenylrest, der gegebenenfalls durch einen C₁-C₁₂-Alkylrest substituiert ist, der gegebenenfalls 1 bis 25 unter N, O, S, F, Si und/oder P ausgewählte Heteroatome umfaßt und/oder gegebenenfalls durch mindestens eine der oben aufgeführten Gruppen substituiert ist;
- einen -C₆H₄-CH₃-Benzylrest, der gegebenenfalls durch einen C₁-C₁₂-Alkylrest substituiert ist, der gegebenenfalls 1 bis 25 unter O, N, S, F, Si und P ausgewählte Heteroatome umfaßt und/oder gegebenenfalls durch mindestens eine der oben aufgeführten Gruppen substituiert ist;
- oder ein Gemisch dieser Reste
stehen können.

11. Zusammensetzung nach einem der Ansprüche 9 bis 10, in der das Monomer der Formel (III) unter 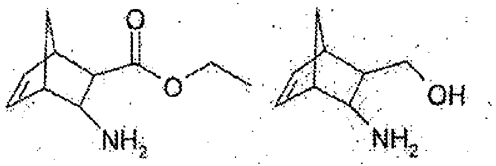 für sich alleine oder im Gemisch ausgewählt ist.

12. Zusammensetzung nach einem der vorhergehenden Ansprüche, in der die Monomere der Formel (I), (II) und/oder (III) 1 bis 100 Gew.-% des fertigen Polymers, insbesondere 1 bis 99,9 Gew.-%, speziell 30 bis 95 Gew.-%, vorzugsweise 35 bis 90 Gew.-% oder sogar 40 bis 85 Gew.-% ausmachen.

13. Zusammensetzung nach einem der vorhergehenden Ansprüche, in der das Polymer außerdem 0,01 bis 99 Gew.-%, bezogen auf das Gewicht des fertigen Polymers, speziell 5 bis 70 Gew.-%, vorzugsweise 10 bis 65 Gew.-% oder sogar 15 bis 60 Gew.-% zusätzliche Monomere umfaßt.

14. Zusammensetzung nach Anspruch 13, in der das zusätzliche Monomer unter den folgenden Monomeren für sich alleine oder im Gemisch sowie Salzen davon ausgewählt ist:
- (i) (Meth)acrylsäureestern der Formel CH₂=CHCOOR'1 oder CH₂=C(CH₃)COOR'1, wobei R'1 für eine lineare, verzweigte oder cyclische, gesättigte oder ungesättigte, gegebenenfalls aromatische Kohlenstoffkette (Aryl, Aralkyl oder Alkylaryl), insbesondere Kohlenwasserstoffkette (Alkyl), mit 1 bis 30 Kohlenstoffatomen, die gegebenenfalls 0 bis 2 Etherfunktionen (-O-) und gegebenenfalls 0 bis 12, insbesondere 1 bis 8, unter -OH (Hydroxyl), -OR' mit R' gleich C₁-C₆-Alkyl (Alkoxy), -CN, -X (Halogen, insbesondere Cl, F, Br oder I), -CO-, -SO3H, -COOH, -OCOO-, -COO-, -OCONR-, -OCONH-, -NH-CONH-, -NR-CONR-, -CF₃, -CN, Epoxid, -NHCO-, -N(R)CO- mit R = lineares oder verzweigtes C₁-C₂₂-Alkyl, das gegebenenfalls 1-12 Heteroatome umfaßt, ausgewählte Funktionen umfaßt, steht;
- (ii) (Meth)acrylsäureamiden der Formel CH₂=CHCONR'2R"2 oder CH₂=C(CH₃)CONR'2R"2, wobei R'2 und R"2 gleich oder verschieden sind und für Wasserstoff oder eine lineare, verzweigte oder cyclische, gesättigte oder ungesättigte, gegebenenfalls aromatische Kohlenstoffkette, insbesondere Kohlenwasserstoffkette (Alkyl), mit 1 bis 30 Kohlenstoffatomen, die gegebenenfalls 0 bis 2 Etherfunktionen (-O-) und gegebenenfalls 0 bis 12, insbesondere 1 bis 8, unter -OH (Hydroxyl), -OR' mit R' gleich C₁-C₆-Alkyl (Alkoxy), -CN, -X (Halogen, insbesondere Cl, F, Br oder I), -CO-, -SO3H, -COOH, -OCOO-, -COO-, -OCONR-, -OCONH-, -NH-CONH-, -NR-CONR-, -CF₃, -CN, Epoxid, -NHCO-, -N(R)CO- mit R = lineares oder verzweigtes C₁-C₂₂-Alkyl, das gegebenenfalls 1-12 Heteroatome umfaßt, ausgewählte Funktionen umfaßt, stehen;
- (iii) Vinylestern der Formel CH₂=CH-OCO-R'3 oder CH₂=C(CH₃)-OCO-R'3, wobei R'3 für eine lineare, verzweigte oder cyclische, gesättigte oder ungesättigte, gegebenenfalls aromatische Kohlenstoffkette, insbesondere Kohlenwasserstoffkette, mit 1 bis 30 Kohlenstoffatomen, die gegebenenfalls 0 bis 2 Etherfunktionen (-O-) und gegebenenfalls 0 bis 12, insbesondere 1 bis 8, unter -OH (Hydroxyl), -OR' mit R' gleich C₁-C₆-Alkyl (Alkoxy), -CN, -X (Halogen, insbesondere Cl, F, Br oder I), -CO-, -SO3H, -COOH, -OCOO-, -COO-, -OCONR-, -OCONH-, -NH-CONH-, -NR-CONR-, -CF₃, -CN, Epoxid, -NHCO-, -N(R)CO- mit R = lineares oder verzweigtes C₁-C₂₂-Alkyl, das gegebenenfalls 1-12 Heteroatome umfaßt, ausgewählte Funktionen umfaßt, steht;
- (iv) Vinylethern der Formel CH₂=CHOR'4 oder CH₂=C(CH₃)OR'4, wobei R'4 für eine lineare, verzweigte oder cyclische, gesättigte oder ungesättigte, gegebenenfalls aromatische Kohlenstoffkette, insbesondere Kohlenwasserstoffkette, mit 1 bis 30 Kohlenstoffatomen, die gegebenenfalls 0 bis 2 Etherfunktionen (-O-) und gegebenenfalls 0 bis 12, insbesondere 1 bis 8, unter -OH (Hydroxyl), -OR' mit R' gleich C₁-C₆-Alkyl (Alkoxy), -CN, -X (Halogen, insbesondere Cl, F, Br oder I), -CO-, -SO3H, -COOH, -OCOO-, -COO-, -OCONR-, -OCONH-, -NH-CONH-, -NR-CONR-, -CF₃, -CN, Epoxid, -NHCO-, -N(R)CO- mit R = lineares oder verzweigtes C₁-C₂₂-Alkyl, das gegebenenfalls 1-12 Heteroatome umfaßt, ausgewählte Funktionen umfaßt, steht;
- (v) Vinylverbindungen der Formel CHR"5=CR5R'5, worin:
R"5 für H oder COOH steht und
R5 für H, CN oder COOH steht und
R'5 unter:
- einem Wasserstoffatom oder einer unter -OH, -CH=O, Halogen (insbesondere Cl, Br, I), -COOH, -CH₂COOH, -NHC(O)H, -N(CH₃)-C(O)H, -NHC(O)CH₃, -N(CH₃)-C(O)CH₃ ausgewählten Gruppe:
- einem Ring: 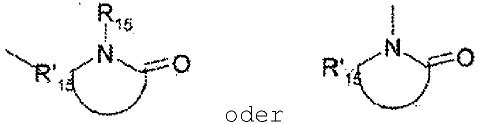 wobei R'15 und R15 unabhängig voneinander für H, eine lineare oder verzweigte, gesättigte oder ungesättigte, gegebenenfalls aromatische, cyclische oder nichtcyclische Alkylgruppe mit 1 bis 25 Kohlenstoffatomen, in die gegebenenfalls ein oder mehrere unter O, N, S und P ausgewählte Heteroatome eingeschoben sind, stehen; wobei die Alkylgruppe außerdem gegebenenfalls durch einen oder mehrere unter -OH und Halogenatomen (Cl, Br, I und F) ausgewählte Substituenten substituiert sein kann;
- einer linearen oder verzweigten Alkylgruppe mit 1 bis 25 Kohlenstoffatomen,
- einer linearen oder verzweigten Alkylgruppe mit 1 bis 25 Kohlenstoffatomen,
- einer C₃- bis C₈-Cycloalkylgruppe wie Cyclohexan,
- einer C₆- bis C₂₀-Arylgruppe wie Phenyl,
- einer C₇- bis C₃₀-Aralkylgruppe (C₁- bis C₄-Alkylgruppe) wie 2-Phenylethyl oder Benzyl,
- einer 4- bis 12-gliedrigen heterocyclischen Gruppe mit einem oder mehreren unter O, N und S ausgewählten Heteroatomen,
- einer Heterocycloalkylgruppe (Alkyl mit 1 bis 4 Kohlenstoffatomen) wie Furfuryl, Furfurylmethyl oder Tetrahydrofurfurylmethyl
ausgewählt ist, wobei die Alkylgruppen, Cycloalkylgruppen, Arylgruppen, Aralkylgruppen, heterocyclischen Gruppen oder Heterocycloalkylgruppen gegebenenfalls 0 bis 2 Etherfunktionen (-O-) und gegebenenfalls 0 bis 12, insbesondere 1 bis 8, unter -OH (Hydroxyl), -OR' mit R' gleich C₁-C₆-Alkyl (Alkoxy), -CN, -X (Halogen, insbesondere Cl, F, Br oder I), -CO-, -SO3H, -COOH, -OCOO-, -COO-, -OCONR-, -OCONH-, -NH-CONH-, -NR-CONR-, -CF₃, -CN, Epoxid, -NHCO-, -N(R)CO- mit R = lineares oder verzweigtes C₁-C₂₂-Alkyl, das gegebenenfalls 1-12 Heteroatome umfaßt, ausgewählte Funktionen umfassen können
und/oder gegebenenfalls durch eine oder mehrere lineare oder verzweigte C₁-C₄-Alkylgruppen substituiert sein können, die gegebenenfalls selbst 0 bis 2 Etherfunktionen (-O-) und gegebenenfalls 0 bis 12, insbesondere 1 bis 8, unter -OH (Hydroxyl), -OR' mit R' gleich C₁-C₆-Alkyl (Alkoxy), -CN, -X (Halogen, insbesondere Cl, F, Br oder I), -CO-, -SO3H, -COOH, -OCOO-, -COO-, -OCONH-, -NH-CONH-, -CF₃, -CN, Epoxid, -NHCO-, -N(R)CO- mit R = lineares oder verzweigtes C₁-C₂₂-Alkyl, das gegebenenfalls 1-12 Heteroatome umfaßt, ausgewählte Funktionen umfassen;
- (vii) den folgenden anionischen Monomeren und ihren Salzen:
Maleinsäureanhydrid, Acrylsäure, Methacrylsäure, Crotonsäure, Itaconsäure, Fumarsäure, Maleinsäure, 2-Carboxyethylacrylat (CH₂=CH-C(O)-O-(CH₂)₂-COOH), Styrolsulfonsäure, 2-Acrylamido-2-methylpropan-sulfonsäure, Vinylbenzoesäure, Vinylphosphonsäure, Sulfopropyl(meth)acrylat und unter den Salzen:
Natrium- oder Kalium(meth)acrylat;
- (viii) den folgenden amphoteren Monomeren:
N,N-Dimethyl-N-(2-methacryloyloxyethyl)-N-(3-sulfopropyl)ammoniumbetain (insbesondere SPE von der Firma Raschig); N,N-Dimethyl-N-(3-methacryl-amidopropyl)-N-(3-sulfopropyl)ammoniumbetain (SPP von Raschig) und 1-(3-Sulfopropyl)-2-vinyl-pyridiniumbetain (SPV von Raschig) sowie 2-(Meth-acryloyloxy)ethylphosphorylcholin;
- (ix) Monomeren der Formel: 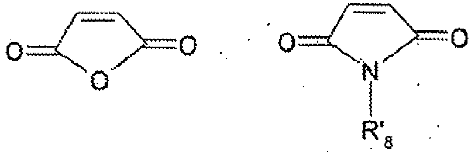 worin R'8 für H oder eine lineare oder verzweigte, gesättigte oder ungesättigte, gegebenenfalls aromatische, cyclische oder nichtcyclische Alkylgruppe mit 1 bis 25 Kohlenstoffatomen, in die gegebenenfalls ein oder mehrere unter O, N, S und P ausgewählte Heteroatome eingeschoben sind, steht; wobei die Alkylgruppe außerdem gegebenenfalls durch einen oder mehrere unter -OH und Halogenatomen (Cl, Br, I und F) ausgewählte Substituenten substituiert sein kann;
- (x) quaternisierten Monomeren der Formel (I), (II) und (III) und den quaternisierten Formen der obigen zusätzlichen Monomere;
- (xi) multivalenten Verbindungen mit mindestens zwei polymerisierbaren Funktionen vom Vinyl-, (Meth)acryl-, Allyl- oder (Meth)acrylamid-Typ.

15. Zusammensetzung nach Anspruch 14, in der das zusätzliche Monomer unter Methyl(meth)acrylat, Ethyl(meth)acrylat, Propyl(meth)acrylat, Isopropyl(meth)acrylat, n-Butyl(meth)acrylat, Isobutyl(meth)acrylat, tert.-Butyl(meth)acrylat, Hexyl(meth)acrylat, Cyclohexyl(meth)acrylat, 2-Ethylhexyl(meth)acrylat, Octyl(meth)acrylat, Isooctyl(meth)acrylat, Isodecyl(meth)acrylat, Decyl-(meth)acrylat, Dodecyl(meth)acrylat, Myristyl-(meth)acrylat, Cetyl(meth)acrylat, Palmityl(meth)-acrylat, Stearyl(meth)acrylat, Behenyl(meth)-acrylat, Oleyl(meth)acrylat, Tridecyl(meth)-acrylat, Hexadecyl(meth)acrylat, Isobornyl(meth)-acrylat, Hydroxyethyl(meth)acrylat, Hydroxypropyl-(meth)acrylat, Phenyl(meth)acrylat, Benzyl(meth)-acrylat, Furfuryl(meth)acrylat; Tetrahydrofurfuryl(meth)acrylat, Ethoxyethyl(meth)acrylat, Methoxyethyl(meth)acrylat; Glycerin(meth)acrylat, 2,2,2,-Trifluoroethyl(meth)acrylat und Poly-(ethylen-isobutylen)(meth)acrylat; (Meth)acryl-nitrilen; (Meth)acrylamid, N-Methyl(meth)acrylamid, N-Isopropyl(meth)acrylamid, N-tert.-Butyl(meth)acrylamid, N-Octyl(meth)acrylamid, N-Undecyl(meth)acrylamid, N,N-Dimethyl(meth)acrylamid, N,N-Dibutyl(meth)acrylamid; Vinylacetat, Vinylpropionat, Vinylbutyrat (oder Vinylbutanoat), Vinylethylhexanoat, Vinylneononanoat und Vinylneododecanoat, Vinylneodecanoat, Vinylpivalat, Vinylcyclohexanoat, Vinylbenzoat, Vinyl-4-tert.-butylbenzoat, Vinyltrifluoracetat; Methylvinylether, Ethylvinylether, Ethylhexylvinylether und Butylvinylether, Cyclohexylvinylether, Isobutylvinylether; Vinylalkohol, Vinylcyclohexan; Vinylpyrrolidon, Vinylcaprolactam, N-Vinylacetamid, N-Vinylformamid, N-Methyl-N-vinylformamid, N-Vinylacetamid; N-Methyl-N-vinylacetamid; Styrol, Methylstyrol, 4-tert.-Butylstyrol, 4-Acetoxystyrol, 4-Methoxystyrol, 3-Methylstyrol, 4-Methylstyrol, 2-Chlorstyrol, 3-Chlorstyrol, 4-Chlorstyrol, Dimethylstyrol, 2,6-Dichlorstyrol, 2,4-Dimethylstyrol; 2,5-Dimethylstyrol, 3,5-Ethoxystyrol, 2,4-Ethoxystyrol, 4-Fluorstyrol; Vinylbutyral; Vinylcarbazol; Vinylchlorid; Vinylformal; Vinylidenchlorid, Vinylidenfluorid, 2-Vinylnaphthalin; N-Methylmaleinimid; 1-Octen, 1-Buten, cis-Chlorbutadien, trans-Chlorbutadien, Chlortrifluorethylen; cis-Isopren, trans-Isopren, 1-Octadecen, Butadien, Hexadecen und Eicosen; Maleinsäureanhydrid und N-Methylmaleinimid; 1,3-Butandioldi(meth)acrylat; 1,6-Hexandioldi(meth)-acrylat, N,N'-Dimethyldiallylammoniumchlorid; Triethylammoniumethylmethacrylatchlorid (MADQUAT), 4-Methylvinylpyridiniumchlorid, N-Methyl-N-vinylimidazoliniumchlorid und Trimethylammoniumpropyl-(meth)acrylamidchlorid für sich alleine oder im Gemisch davon ausgewählt ist.

16. Zusammensetzung nach Anspruch 14 bis 15, in der das zusätzliche Monomer unter Vinylneodecanoat, Vinyl-tert.-butylbenzoat; Vinylpyrrolidon; Vinylcaprolactam; N-Vinylformamid; N,N'-Dimethyldiallylammoniumchlorid; Triethylammoniumethylmethacrylatchlorid; Ethyl(meth)acrylat, Methyl(meth)-acrylat, tert.-Butyl(meth)acrylat oder Isobornyl-(meth)acrylat; Vinylacetat, Crotonsäure; (Meth)-acrylsäure, Methacryloylethylbetain, Octylacrylamid; N-Methyl-N-vinylimidazoliniumchlorid, 1-Eicosen, tert.-Butylacrylamid, Acrylamid, Hexadecen und Gemischen davon ausgewählt ist.

17. Zusammensetzung nach einem der vorhergehenden Ansprüche, in der das Polymer in Form eines Homopolymers oder eines Copolymers, das linear oder verzweigt, vernetzt oder nicht vernetzt und statistisch, alternierend, blockartig oder gradientenartig oder sogar sternförmig, vorzugsweise linear, statistisch oder blockartig, aufgebaut sein kann, vorliegt.

18. Zusammensetzung nach einem der vorhergehenden Ansprüche, in der das Polymer unter:
- Poly(vinylpyrrolidon-co-dimethylaminoethylmethacrylat)-Copolymeren;
- Poly(vinylcaprolactam-co-vinylpyrrolidon-co-dimethylaminoethylmethacrylat)-Copolymeren;
- Poly(vinylpyrrolidon-co-dimethylaminopropylmethacrylamid)-Copolymeren;
- Poly(vinylpyrrolidon-co-polyvinylcaprolactam-co-dimethylaminopropylmethacrylamid)-Copolymeren;
- Poly(vinylamin), Poly(allylamin), Poly(diallylamin) und
- Poly(N-vinylformamid-co-vinylamin)-Copolymeren ausgewählt ist.

19. Zusammensetzung nach einem der vorhergehenden Ansprüche, in der das Polymer eine gewichtsmittlere Molmasse (Mw) zwischen 1000 und 3.000.000, insbesondere zwischen 1500 und 1.000.000 und weiter bevorzugt zwischen 2000 und 800.000 und noch weiter bevorzugt zwischen 2500 und 500.000 aufweist.

20. Zusammensetzung nach Anspruch 1, in der das Polymer mindestens 3 Wiederholungseinheiten umfaßt, die für sich alleine oder im Gemisch unter: 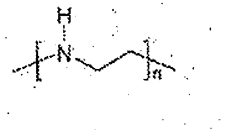 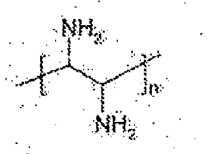 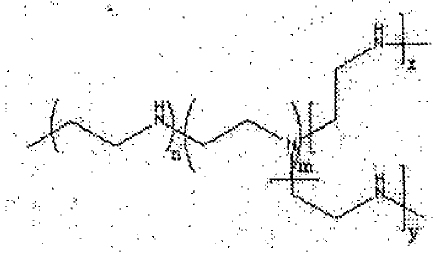 ausgewählt sind.

21. Zusammensetzung nach einem der vorhergehenden Ansprüche, in der das Neutralisationsmittel in einer Menge von 0,01 bis 3 Moläquivalenten, insbesondere 0,05 bis 2,5 Moläquivalenten oder sogar 0,1 bis 2 Moläquivalenten, bezogen auf die gesamten Aminfunktionen des Polymers bzw. der Monomere, zugesetzt ist.

22. Zusammensetzung nach einem der vorhergehenden Ansprüche, in der das Neutralisationsmittel in einer stöchiometrischen Menge, bezogen auf die gesamten Aminfunktionen des Polymers bzw. der Monomere, zugesetzt ist.

23. Zusammensetzung nach einem der vorhergehenden Ansprüche, in der das Neutralisationsmittel eine zahlenmittlere Molmasse (Mn) zwischen 300 und 50.000, insbesondere zwischen 350 und 20.000 und weiter bevorzugt zwischen 400 und 10.000 oder sogar 450 und 5.000 aufweist.

24. Zusammensetzung nach einem der vorhergehenden Ansprüche, in der das Neutralisationsmittel linear ist und 2 Säurefunktionen, eine an jedem Kettenende, umfaßt.

25. Zusammensetzung nach einem der vorhergehenden Ansprüche, in der das Neutralisationsmittel unter:
- Polymeren mit mindestens drei Wiederholungseinheiten vom Alkylenglykol-Typ mit 2 bis 4 Kohlenstoffatomen in der Alkylgruppe und Kombinationen davon ausgewählt ist;
ausgewählt unter Polymeren mit den folgenden Strukturen:
CH₃-O-(CH₂-CH₂-O-)ₙ-CO-(CH₂-CHR-O)ₘ-CH₂-R₁₀
CH₃-O-(CH₂-CH₂-O-)ₙ-b-(CH₂-CHR-O)ₘ-CH₂-R₁₀
CH₃-O-(CH₂-CH₂-O-)ₙ-CO-(CH₂-CHR-O)ₘ-CH₂-CH₂ R₁₀
CH₃-O-(CH₂-CH₂-O-)ₙ-b-(CH₂-CHR-O)ₘ-CH₂-CH₂R₁₀
CH₃-O-(CH₂-CH₂-O-)ₙ-CO-(CH₂-CHR-O)ₘ-CH₂-CH₂CH₂R₁₀
CH₃-O-(CH₂-CH₂-O-)ₙ-b-(CH₂-CHR-O)ₘ-CH₂-CH₂CH₂R₁₀
CH₃-O-(CH₂-CH₂-O-)ₙ-CO-(CH₂-CHR-O)-CH₂-CH₂OCO-CH₂-CH₂COOH
CH₃-O-(CH₂-CH₂-O-)ₙ-b-(CH₂-CHR-O)-CH₂-CH₂OCO-CH₂-CH₂COOH
R₁₀-(CH₂-CH₂-O-)ₙ-CO-(CH₂-CHR-O)ₘ-CH₂-CH₂ R₁₀
R₁₀-(CH₂-CH₂-O-)ₙ-b-(CH₂-CHR-O)ₘ-CH₂-CH₂R₁₀
R₁₀-CH₂O-(CH₂-CH₂-O-)ₙ-CO-(CH₂-CHR-O)ₘ-CH₂-R₁₀
R₁₀-CH₂O-(CH₂-CH₂-O-)ₙ-b-(CH₂-CHR-O)ₘ-CH₂-R₁₀
R₁₀-CH₂-CH₂-CH₂-O-(CH₂-CH₂-O-)ₙ-CO-(CH₂-CHR-O)ₘ-CH₂-CH₂-CH₂-R₁₀
R₁₀-CH₂-CH₂-CH₂-O-(CH₂-CH₂-O-)ₙ-b-(CH₂-CHR-O)ₘ-CH₂-CH₂-CH₂-R₁₀
R₁₀-CH₂-CH₂-COO-(CH₂-CH₂-O-)ₙ-CO-(CH₂-CHR-O)ₘ-CH₂-CH₂-O-CO-CH₂-CH₂-R₁₀
R₁₀-CH₂-CH₂-COO-(CH₂-CH₂-O-)ₙ-b-(CH₂-CHR-O)ₘ-CH₂-CH₂-O-CO-CH₂-CH₂-R₁₀
R₁₀-CH₂-CH₂-CONH-(CH₂-CH₂-O-)ₙ-CO-(CH₂-CHR-O)ₘ-CH₂-CH₂-NH-CO-CH₂-CH₂-R₁₀
R₁₀-CH₂-CH₂-CONH-(CH₂-CH₂-O-)ₙ-b-(CH₂-CHR-O)ₘ-CH₂-CH₂-NH-CO-CH₂-CH₂-R₁₀
worin:
- der Rest R gleich oder verschieden ist und für -CH₃, -(CH₂)ₓ-COOH; -(CH₂)ₓ-SO₃H oder (CH₂)ₓ-PO₃H₂ mit x = 1 bis 8 steht;
- der Rest R₁₀ gleich oder verschieden ist und für -COOH, -SO₃H oder -PO₃H₂ steht;
- n für eine ganze Zahl zwischen 3 und 1000 steht und
- m für eine ganze Zahl zwischen 0 und 200 steht; mit der Maßgabe, daß das Verhältnis n:m größer 5 ist, wenn m nicht gleich null ist.

26. Zusammensetzung nach Anspruch 25, in der das Neutralisationsmittel unter Polymeren ausgewählt ist, für die:
- R für Methyl oder -(CH₂)ₓ-COOH; -(CH₂)ₓ-SO₃H oder (CH₂)ₓ-PO₃H₂ mit x = 2 bis 3 steht und/oder
- n für 4 bis 30, insbesondere 5 bis 15, steht.

27. Zusammensetzung nach einem der vorhergehenden Ansprüche, in der das Neutralisationsmittel unter:
CH₃-O-(CH₂-CH₂-O-)ₙ-CO-(CH₂-CHR-O)ₘ-CH₂-CH₂ R₁₀
CH₃-O-(CH₂-CH₂-O-)ₙ-CO-(CH₂-CHR-O)ₘ-CH₂-CH₂CH₂R₁₀
R₁₀-(CH₂-CH₂-O-)ₙ-CO-(CH₂-CHR-O)ₘ-CH₂-CH₂ R₁₀
R₁₀-CH₂O-(CH₂-CH₂-O-)ₙ-CO-(CH₂-CHR-O)ₘ-CH₂-R₁₀
worin der Rest R₁₀ für -COOH, -SO₃H oder -PO₃H₂, vorzugsweise COOH, steht; n für 0 bis 200 steht und n für 3 bis 1000, insbesondere 4 bis 30 oder sogar 5 bis 15 steht;
ausgewählt ist.

28. Zusammensetzung nach einem der vorhergehenden Ansprüche, in der das Neutralisationsmittel unter:
CH₃-O-(CH₂-CH₂-O-)ₙ-CH₂-CH₂ R₁₀
CH₃-O-(CH₂-CH₂-O-)ₙ-CH₂-CH₂CH₂R₁₀
R₁₀-(CH₂-CH₂-O-)ₙ-CH₂-CH₂R₁₀
R₁₀-CH₂O-(CH₂-CH₂-O-)ₙ-CH₂-R₁₀
worin der Rest R₁₀ für COOH, SO₃H oder -PO₃H₂, vorzugsweise COOH, steht; und n für 3 bis 1000, insbesondere 4 bis 30 oder sogar 5 bis 15 steht; ausgewählt ist.

29. Zusammensetzung nach einem der vorhergehenden Ansprüche, in der das Neutralisationsmittel unter:
- CH₃-O-(CH₂-CH₂-O-)ₙ-CH₂-CH₂-SO₃H;
- CH₃-O-(CH₂-CH₂-O-)ₙ-CH₂-CH₂-CH₂COOH;
- HOCO-(CH₂-CH₂-O-)ₙ-CH₂-CH₂COOH und
- HOCO-CH₂O-(CH₂-CH₂-O-)ₙ-CH₂-COOH;
mit n = 3 bis 1000, insbesondere 4 bis 30 oder sogar 5 bis 15,
ausgewählt ist.

30. Zusammensetzung nach einem der Ansprüche 1 bis 29, in der das Polymer in einer Menge von 0,01 bis 50 Gew.-% Trockensubstanz, insbesondre 0,1 bis 30 Gew.-% oder sogar 0,3 bis 10 Gew.-%, noch besser 1 bis 3 Gew.-%, bezogen auf das Gesamtgewicht der kosmetischen oder pharmazeutischen Zusammensetzung, vorliegt.

31. Zusammensetzung nach Anspruch 30, die außerdem mindestens einen Bestandteil, der unter: Wasser, hydrophilen organischen Lösungsmitteln wie Alkoholen und insbesondere linearen oder verzweigten C₁-C₆-Monoalkoholen, wie Ethanol, *tert.-*Butanol, n-Butanol, Isopropanol oder n-Propanol, und Polyolen wie Glycerin, Diglycerin, Propylenglykol, Sorbitol, Pentylenglykol und Polyethylenglykolen; Glykolethern, insbesondere C₂-Glykolethern und hydrophilen C₂-C₄-Aldehyden; Wachsen, pastösen Fettkörpern, Gummen; lipophilen organischen Lösungsmitteln; Ölen und insbesondere Kohlenwasserstoffölen tierischen Ursprungs wie Perhydrosqualen; pflanzlichen Kohlenwasserstoffölen wie flüssigen Triglyceriden von Fettsäuren mit 4 bis 10 Kohlenstoffatomen wie Heptansäure- oder Octansäuretriglyceriden oder auch Sonnenblumenöl, Maisöl, Sojabohnenöl, Traubenkernöl, Sesamöl, Aprikosenöl, Macadamiaöl, Rizinusöl oder Avocadoöl, Capryl/Caprinsäuretriglyceriden, Jojobaöl oder Sheabutteröl; linearen oder verzweigten Kohlenwasserstoffen mineralischen oder synthetischen Ursprungs wie Paraffinölen und Derivaten davon, Vaseline, Polydecenen, hydriertem Polyisobuten wie Parleam; synthetischen Estern und Ethern, insbesondere von Fettsäuren wie beispielsweise Purcellinöl, Isopropylmyristat, 2-Ethylhexylpalmitat, 2-Octyldodecylstearat, 2-Octyldodecylerucat, Isostearylisostearat; hydroxylgruppenhaltigen Estern wie Isostearyllactat, Octylhydroxystearat, Octyldodecylhydroxystearat, Diisostearylmalat, Triisocetylcitrat, Heptanoaten, Octanoaten und Decanoaten von Fettalkoholen; Polyolestern wie Propylenglykoldioctanoat, Neopentylglykoldiheptanoat, Diethylenglykoldiisononanoat und Pentaerythritestern; Fettalkoholen mit 12 bis 26 Kohlenstoffatomen wie Octyldodecanol, 2-Butyloctanol, 2-Hexyldecanol, 2-Undecylpentadecanol, Oleylalkohol; teilfluorierten Kohlenwasserstoff- und/oder Silikonölen; Silikonölen wie flüchtigen oder nichtflüchtigen, linearen oder cyclischen, bei Raumtemperatur flüssigen oder pastösen Polymethylsiloxanen (PDMS) wie Cyclomethiconen oder Dimethiconen, die gegebenenfalls eine Phenylgruppe umfassen, wie Phenyltrimethiconen, Phenyltrimethylsiloxydiphenylsiloxanen, Diphenylmethyldimethyltrisiloxanen, Diphenyldimethiconen, Phenyldimethiconen, Polymethylphenylsiloxanen; Ketonen, die bei Umgebungstemperatur flüssig sind; Polypropylenglykolethern, die bei Umgebungstemperatur flüssig sind; kurzkettigen Estern (mit insgesamt 3 bis 8 Kohlenstoffatomen); Ethern, die bei 25°C flüssig sind; Alkanen, die bei 25°C flüssig sind; cyclischen aromatischen Verbindungen, die bei 25°C flüssig sind; Aldehyden, die bei 25°C flüssig sind; Pigmenten, Perlmutt, Füllstoffen; wasserlöslichen Farbmitteln; fettlöslichen Farbmitteln; Polymeren, insbesondere filmbildenden Polymeren; anionischen, amphoteren, nichtionischen oder kationischen Tensiden oder Gemischen davon; Vitaminen, Duftstoffen, Perlglanzmitteln, Verdickungsmitteln, Gelierungsmitteln, Spurenelementen, Glättungsmitteln, Sequestriermitteln, Parfümen, alkalinisierenden oder ansäuernden Mitteln, Konservierungsmitteln, Sonnenschutzmitteln, Tensiden, Antioxidantien, Mitteln gegen Haarausfall, Antischuppenmitteln, Treibmitteln oder Ceramiden ausgewählt ist, oder Gemische dieser Bestandteile umfaßt.

32. Zusammensetzung nach einem der Ansprüche 1 bis 31, die in Form eines Produkts zur Pflege und/oder zum Make-up der Körper- oder Gesichtshaut, der Lippen und der Haare, eines Sonnenschutz- oder Selbstbräunungsprodukts, eines Haarprodukts, insbesondere zur Festigung der Frisur oder zur Haargestaltung, vorliegt.

33. Zusammensetzung nach einem der Ansprüche 1 bis 32, die in Form von Shampoos, Gelen, Lotionen für die Wasserwelle, Lotionen für die Fönwelle, Zusammensetzungen zur Fixierung der Frisur wie Lacken oder Sprays, Duschgelen, Schaumbädern; Wash-out- oder Leave-in-Haarpflegespülungen, Dauerwellen-, Entkrausungs-, Färbe- oder Bleichzusammensetzungen oder auch in Form von Wash-out-Zusammensetzungen zur Anwendung vor oder nach einer Färbung, Bleichung, Dauerwelle oder Entkrausung oder auch zwischen zwei Schritten einer Dauerwelle oder Entkrausung; Waschzusammensetzungen für die Haut und insbesonder in Form von Lösungen oder Gelen für Bad oder Dusche oder Abschminkprodukten; wäßrigen oder wäßrig-alkoholischen Lösungen für die Pflege der Haut und/oder der Haare vorliegt.

34. Verfahren zur kosmetischen Behandlung von Keratinmaterialien wie der Körper- oder Gesichtshaut, der Nägel, der Haare, der Wimpern und/oder der Augenbrauen, **dadurch gekennzeichnet, daß** es daraus besteht, daß man auf die Keratinmaterialien eine kosmetische Zusammensetzung gemäß einem der Ansprüche 1 bis 33 aufbringt.

35. Verfahren zur Festigung der Frisur, kosmetischen Behandlung, Pflege, Wäsche, Abschminkung und/oder Schminkung von Keratinmaterialien, insbesondere der Körper- oder Gesichtshaut, der Nägel, der Haare, der Wimpern und/oder der Augenbrauen, **dadurch gekennzeichnet, daß** es daraus besteht, daß man auf die Keratinmaterialien eine kosmetische Zusammensetzung gemäß einem der Ansprüche 1 bis 37 oder 41 bis 44 aufbringt und dann gegebenenfalls mit Wasser auswäscht.

36. Kationisches ethylenisches Polymer mit mindestens einer protonierbaren primären, sekundären oder tertiären Aminfunktion, die zumindest teilweise durch ein unter polymeren organischen Säuren mit mindestens einer Carbonsäure-, Sulfonsäure- und/oder Phosphonsäure ausgewähltes Neutralisationsmittel neutralisiert ist, wobei das zumindest teilweise neutralisierte Polymer in ein wäßriges Medium befördert werden kann, d.h. wasserlöslich oder wasserdispergierbar ist.

37. Polymer nach Anspruch 36, das mindestens ein Monomer der Formel (I) für sich alleine oder im Gemisch umfaßt: 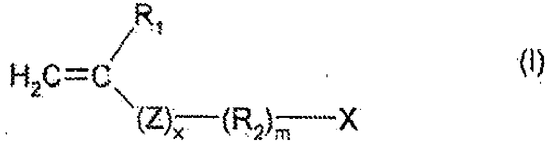 worin:
- R1 für ein Wasserstoffatom oder einen linearen oder verzweigten Kohlenwasserstoffrest vom Typ CₚH₂ₚ₊₁, wobei p für eine ganze Zahl zwischen 1 und 12 inklusive steht, steht;
- Z für eine unter -COO-, -CONH-, -CONCH₃-, -OCO- oder -O-, -SO₂-, -CO-O-CO- oder -CO-CH₂-CO-, vorzugsweise COO und CONH, ausgewählte zweiwertige Gruppe steht;
- x für 0 oder 1, vorzugsweise 1, steht;
- R2 für einen gesättigten oder ungesättigten, gegebenenfalls aromatischen, linearen, verzweigten oder cyclischen zweiwertigen Kohlenstoffrest mit 1 bis 30 Kohlenstoffatomen, der 1 bis 18 unter O, N, S, F, Cl, Br, Si und P ausgewählte Heteroatome umfassen kann, steht;
- m für 0 oder 1 steht;
- X für:
(a) eine Guanidino- oder Amidinogruppe oder auch
(b) eine Gruppe der Formel -N(R₆)(R₇) steht, wobei R6 und R7 unabhängig voneinander für (i) ein Wasserstoffatom oder (ii) eine lineare, verzweigte oder cyclische, gesättigte oder ungesättigte, gegebenenfalls aromatische Alkylgruppe mit 1 bis 18 Kohlenstoffatomen, die 1 bis 10 unter O, N, S, F, Si und P ausgewählte Heteroatome umfassen kann, stehen; oder (iii) R6 und R7 mit dem Stickstoffatom einen Ring der Formel: 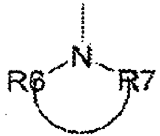 bilden, der gesättigt oder ungesättigt, gegebenenfalls aromatisch, ist und insgesamt 5, 6, 7 oder 8 Atome und insbesondere 4, 5 oder 6 Kohlenstoffatome und/oder 2 bis 4 unter O, S und N ausgewählte Heteroatome umfaßt; wobei der erste Ring mit einem oder mehreren weiteren gesättigten oder ungesättigten, gegebenenfalls aromatischen Ringen mit jeweils 5, 6 oder 7 Atomen und insbesondere 4, 5, 6 oder 7 Kohlenstoffatomen und/oder 2 bis 4 unter O, S und N ausgewählten Heteroatomen anelliert sein kann;
(c) einen Ring: 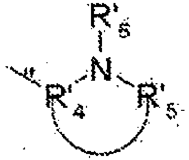
worin R'4 und R'5 mit dem Stickstoffatom einen gesättigten oder ungesättigten, gegebenenfalls aromatischen Ring mit insgesamt 5, 6, 7 oder 8 Atomen und insbesondere 4, 5 oder 6 Kohlenstoffatomen und/oder 2 bis 4 unter O, S und N ausgewählten Heteroatomen bilden; wobei der Ring mit einem oder mehreren weiteren gesättigten oder ungesättigten, gegebenenfalls aromatischen Ringen mit jeweils 5, 6 oder 7 Atomen und insbesondere 4, 5, 6, 7 oder 8 Kohlenstoffatomen und/oder 2 bis 4 unter O, S und N ausgewählten Heteroatomen anelliert sein kann; und
R'6 unter H, -CH₃ und -C₂H₅ ausgewählt ist;
steht;
mit der Maßgabe, daß das Monomer der Formel (I) mindestens eine bei einem pH-Wert zwischen pH 1 und pH 12 protonierbare primäre, sekundäre oder tertiäre Aminfunktion umfaßt; wobei es sich bei dieser Funktion um die Reste R2 und/oder X handeln kann oder diese Funktion von den Resten R2 und/oder X getragen werden kann.

38. Polymer nach einem der Ansprüche 36 bis 37, wobei das Monomer der Formel (I) unter Dimethylaminopropyl(meth)acrylamid, Dimethylaminoethyl(meth)-acrylamid, Diethylaminoethyl(meth)acrylat, Dimethylaminoethyl(meth)acrylat, tert.-Butylaminoethyl(meth)acrylat, Morpholinoethyl(meth)acrylat, Vinylimidazol, Vinylpyridin, Vinylamin, Allylamin und den nachstehenden Monomeren, in denen R = H oder Methyl, für sich alleine oder im Gemisch ausgewählt ist:

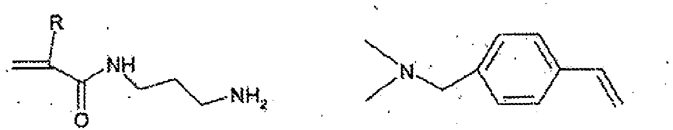

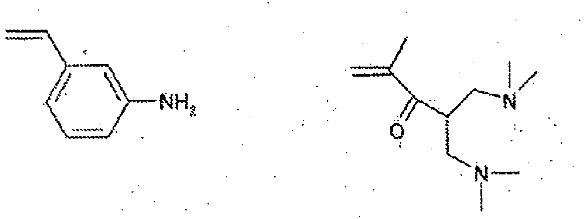

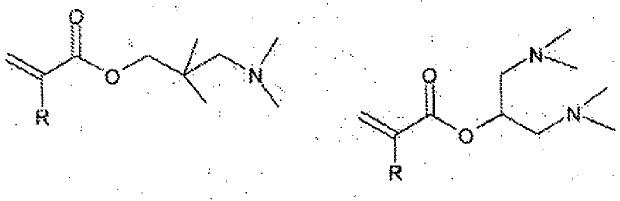

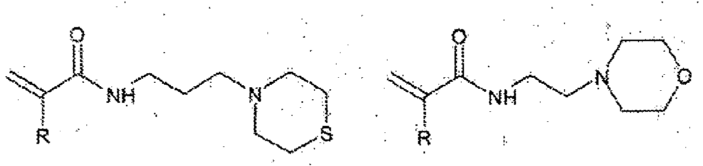

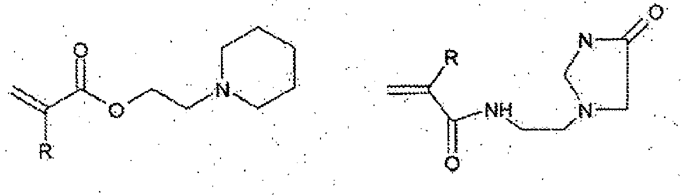

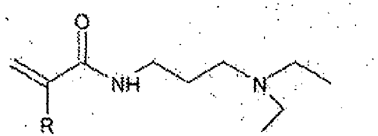

39. Polymer nach Anspruch 36, das mindestens ein Monomer der Formel (II) für sich alleine oder im Gemisch umfaßt: 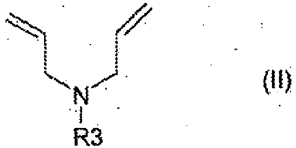
worin R3 für Wasserstoff oder einen linearen, verzweigten oder cyclischen, gesättigten oder ungesättigten, gegebenenfalls aromatischen Kohlenstoffrest, insbesondere Kohlenwasserstoffrest, mit 1 bis 30 Kohlenstoffatomen, der 1 bis 18 unter O, N, S, F, Cl, Br, Si und P ausgewählte Heteroatome umfassen kann, steht;
mit der Maßgabe, daß das Monomer der Formel (II) mindestens eine bei einem pH-Wert zwischen pH 1 und pH 12 protonierbare primäre, sekundäre oder tertiäre Aminfunktion umfaßt.

40. Polymer nach Anspruch 39, wobei das Monomer der Formel (II) unter N-Methyldiallylamin und Diallylamin für sich alleine oder im Gemisch ausgewählt ist.

41. Polymer nach Anspruch 36, das mindestens ein Monomer der Formel (III) für sich alleine oder im Gemisch umfaßt: 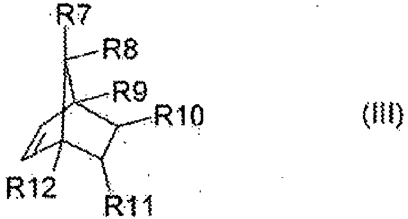 worin R7 bis R12 unabhängig voneinander für
- ein Wasserstoffatom,
- eine -NR13R'13-Gruppe, wobei R13 und R'13 unabhängig voneinander für Wasserstoff oder eine lineare, verzweigte oder cyclische, gesättigte oder ungesättigte, gegebenenfalls aromatische Alkylgruppe mit 1 bis 18 Kohlenstoffatomen, die 1 bis 10 unter O, N, S, F, Si und P ausgewählte Heteroatome umfassen kann, stehen und R13 und R'13 insbesondere unter Wasserstoff und einer Methyl-, Ethyl-, Propyl-, Isopropyl-, n-Butyl-, t-Butyl-, Isobutyl-, Octyl-, Lauryl- oder Stearylgruppe ausgewählt sein können;
- einen linearen, verzweigten oder cyclischen, gesättigten oder ungesättigten, gegebenenfalls aromatischen Kohlenstoffrest, insbesondere Kohlenwasserstoffrest, mit 1 bis 30 Kohlenstoffatomen, der 1 bis 18 unter O, N, S, F, Si und P ausgewählte Heteroatome umfassen kann,
stehen;
mit der Maßgabe, daß das Monomer der Formel (III) mindestens eine bei einem pH-Wert zwischen pH 1 und pH 12 protonierbare primäre, sekundäre oder tertiäre Aminfunktion umfaßt.

42. Polymer nach Anspruch 41, wobei das Monomer der Formel (III) unter 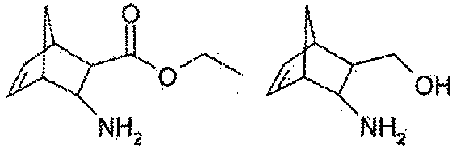 für sich alleine oder im Gemisch ausgewählt ist.

43. Polymer nach einem der Ansprüche 36 bis 42 53, das außerdem 0,01 bis 99 Gew.-%, bezogen auf das Gewicht des fertigen Polymers, speziell 5 bis 70 Gew.-%, vorzugsweise 10 bis 65 Gew.-% oder sogar 15 bis 60 Gew.-% zusätzliche Monomere umfaßt.

44. Polymer nach Anspruch 43, wobei das zusätzliche Monomer unter den folgenden Monomeren für sich alleine oder im Gemisch sowie Salzen davon ausgewählt ist:
- (i) (Meth)acrylsäureestern der Formel CH₂=CHCOOR'1 oder CH₂=C(CH₃)COOR'1, wobei R'1 für eine lineare, verzweigte oder cyclische, gesättigte oder ungesättigte, gegebenenfalls aromatische Kohlenstoffkette (Aryl, Aralkyl oder Alkylaryl), insbesondere Kohlenwasserstoffkette (Alkyl), mit 1 bis 30 Kohlenstoffatomen, die gegebenenfalls 0 bis 2 Etherfunktionen (-O-) und gegebenenfalls 0 bis 12, insbesondere 1 bis 8, unter -OH (Hydroxyl), -OR' mit R' gleich C₁-C₆-Alkyl (Alkoxy), -CN, -X (Halogen, insbesondere Cl, F, Br oder I), -CO-, -SO3H, -COOH, -OCOO-, -COO-, -OCONR-, -OCONH-, -NH-CONH-, -NR-CONR-, -CF₃, -CN, Epoxid, -NHCO-, -N(R)CO- mit R = lineares oder verzweigtes C₁-C₂₂-Alkyl, das gegebenenfalls 1-12 Heteroatome umfaßt, ausgewählte Funktionen umfaßt, steht;
- (ii) (Meth)acrylsäureamiden der Formel CH₂=CHCONR'2R"2 oder CH₂=C(CH₃)CONR'2R"2, wobei R'2 und R"2 gleich oder verschieden sind und für Wasserstoff oder eine lineare, verzweigte oder cyclische, gesättigte oder ungesättigte, gegebenenfalls aromatische Kohlenstoffkette, insbesondere Kohlenwasserstoffkette (Alkyl), mit 1 bis 30 Kohlenstoffatomen, die gegebenenfalls 0 bis 2 Etherfunktionen (-O-) und gegebenenfalls 0 bis 12, insbesondere 1 bis 8, unter -OH (Hydroxyl), -OR' mit R' gleich C₁-C₆-Alkyl (Alkoxy), -CN, -X (Halogen, insbesondere Cl, F, Br oder I), -CO-, -SO3H, -COOH, -OCOO-, -COO-, -OCONR-, -OCONH-, -NH-CONH-, -NR-CONR-, -CF₃, -CN, Epoxid, -NHCO-, -N(R)CO- mit R = lineares oder verzweigtes C₁-C₂₂-Alkyl, das gegebenenfalls 1-12 Heteroatome umfaßt, ausgewählte Funktionen umfaßt, stehen;
- (iii) Vinylestern der Formel CH₂=CH-OCO-R'3 oder CH₂=C(CH₃)-OCO-R'3, wobei R'3 für eine lineare, verzweigte oder cyclische, gesättigte oder ungesättigte, gegebenenfalls aromatische Kohlenstoffkette, insbesondere Kohlenwasserstoffkette, mit 1 bis 30 Kohlenstoffatomen, die gegebenenfalls 0 bis 2 Etherfunktionen (-O-) und gegebenenfalls 0 bis 12, insbesondere 1 bis 8, unter -OH (Hydroxyl), -OR' mit R' gleich C₁-C₆-Alkyl (Alkoxy), -CN, -X (Halogen, insbesondere Cl, F, Br oder I), -CO-, -SO3H, -COOH, -OCOO-, -COO-, -OCONR-, -OCONH-, -NH-CONH-, -NR-CONR-, -CF₃, -CN, Epoxid, -NHCO-, -N(R)CO- mit R = lineares oder verzweigtes C₁-C₂₂-Alkyl, das gegebenenfalls 1-12 Heteroatome umfaßt, ausgewählte Funktionen umfaßt, steht;
- (iv) Vinylethern der Formel CH₂=CHOR'4 oder CH₂=C(CH₃)OR'4, wobei R'4 für eine lineare, verzweigte oder cyclische, gesättigte oder ungesättigte, gegebenenfalls aromatische Kohlenstoffkette, insbesondere Kohlenwasserstoffkette, mit 1 bis 30 Kohlenstoffatomen, die gegebenenfalls 0 bis 2 Etherfunktionen (-O-) und gegebenenfalls 0 bis 12, insbesondere 1 bis 8, unter -OH (Hydroxyl), -OR' mit R' gleich C₁-C₆-Alkyl (Alkoxy), -CN, -X (Halogen, insbesondere Cl, F, Br oder I), -CO-, -SO3H, -COOH, -OCOO-, -COO-, -OCONR-, -OCONH-, -NH-CONH-, -NR-CONR-, -CF₃, -CN, Epoxid, -NHCO-, -N(R)CO- mit R = lineares oder verzweigtes C₁-C₂₂-Alkyl, das gegebenenfalls 1-12 Heteroatome umfaßt, ausgewählte Funktionen umfaßt, steht;
- (v) Vinylverbindungen der Formel CHR"5=CR5R'5, worin:
R"5 für H oder COOH steht und
R5 für H, CN oder COOH steht und
R'5 unter:
- einem Wasserstoffatom oder einer unter -OH, -CH=O, Halogen (insbesondere Cl, Br, I), -COOH, -CH₂COOH, -NHC(O)H, -N(CH₃)-C(O)H, -NHC(O)CH₃, -N(CH₃)-C(O)CH₃ ausgewählten Gruppe:
- einem Ring: 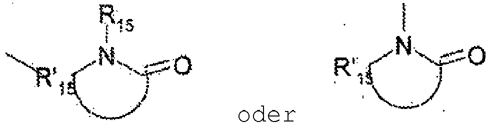 wobei R'15 und R15 unabhängig voneinander für H, eine lineare oder verzweigte, gesättigte oder ungesättigte, gegebenenfalls aromatische, cyclische oder nichtcyclische Alkylgruppe mit 1 bis 25 Kohlenstoffatomen, in die gegebenenfalls ein oder mehrere unter O, N, S und P ausgewählte Heteroatome eingeschoben sind, stehen; wobei die Alkylgruppe außerdem gegebenenfalls durch einen oder mehrere unter -OH und Halogenatomen (Cl, Br, I und F) ausgewählte Substituenten substituiert sein kann;
- einer linearen oder verzweigten Alkylgruppe mit 1 bis 25 Kohlenstoffatomen,
- einer linearen oder verzweigten Alkylgruppe mit 1 bis 25 Kohlenstoffatomen,
- einer C₃- bis C₈-Cycloalkylgruppe wie Cyclohexan,
- einer C₆- bis C₂₀-Arylgruppe wie Phenyl,
- einer C₇- bis C₃₀-Aralkylgruppe (C₁- bis C₄-Alkylgruppe) wie 2-Phenylethyl oder Benzyl,
- einer 4- bis 12-gliedrigen heterocyclischen Gruppe mit einem oder mehreren unter O, N und S ausgewählten Heteroatomen,
- einer Heterocycloalkylgruppe (Alkyl mit 1 bis 4 Kohlenstoffatomen) wie Furfuryl, Furfurylmethyl oder Tetrahydrofurfurylmethyl
ausgewählt ist, wobei die Alkylgruppen, Cycloalkylgruppen, Arylgruppen, Aralkylgruppen, heterocyclischen Gruppen oder Heterocycloalkylgruppen gegebenenfalls 0 bis 2 Etherfunktionen (-O-) und gegebenenfalls 0 bis 12, insbesondere 1 bis 8, unter -OH (Hydroxyl), -OR' mit R' gleich C₁-C₆-Alkyl (Alkoxy), -CN, -X (Halogen, insbesondere Cl, F, Br oder I), -CO-, -SO3H, -COOH, -OCOO-, -COO-, -OCONR-, -OCONH-, -NH-CONH-, -NR-CONR-, -CF₃, -CN, Epoxid, -NHCO-, -N(R)CO- mit R = lineares oder verzweigtes C₁-C₂₂-Alkyl, das gegebenenfalls 1-12 Heteroatome umfaßt, ausgewählte Funktionen umfassen können
und/oder gegebenenfalls durch eine oder mehrere lineare oder verzweigte C₁-C₄-Alkylgruppen substituiert sein können, die gegebenenfalls selbst 0 bis 2 Etherfunktionen (-O-) und gegebenenfalls 0 bis 12, insbesondere 1 bis 8, unter -OH (Hydroxyl), -OR' mit R' gleich C₁-C₆-Alkyl (Alkoxy), -CN, -X (Halogen, insbesondere Cl, F, Br oder I), -CO-, -SO3H, -COOH, -OCOO-, -COO-, -OCONH-, -NH-CONH-, -CF₃, -CN, Epoxid, -NHCO-, -N(R)CO- mit R = lineares oder verzweigtes C₁-C₂₂-Alkyl, das gegebenenfalls 1-12 Heteroatome umfaßt, ausgewählte Funktionen umfassen;
- (vii) den folgenden anionischen Monomeren und ihren Salzen:
Maleinsäureanhydrid, Acrylsäure, Methacrylsäure, Crotonsäure, Itaconsäure, Fumarsäure, Maleinsäure, 2-Carboxyethylacrylat (CH₂=CH-C(O)-O-(CH₂)₂-COOH), Styrolsulfonsäure, 2-Acrylamido-2-methylpropan-sulfonsäure, Vinylbenzoesäure, Vinylphosphonsäure, Sulfopropyl(meth)acrylat und unter den Salzen: Natrium- oder Kalium(meth)acrylat;
- (viii) den folgenden amphoteren Monomeren:
N,N-Dimethyl-N-(2-methacryloyloxyethyl)-N-(3-sulfopropyl)ammoniumbetain (insbesondere SPE von der Firma Raschig); N,N-Dimethyl-N-(3-methacrylamidopropyl)-N-(3-sulfopropyl)ammoniumbetain (SPP von Raschig) und 1-(3-Sulfopropyl)-2-vinylpyridiniumbetain (SPV von Raschig) sowie 2-(Methacryloyloxy)ethylphosphorylcholin;
- (ix) Monomeren der Formel: 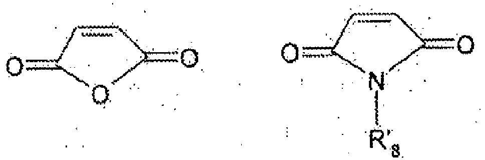 worin R'8 für H oder eine lineare oder verzweigte, gesättigte oder ungesättigte, gegebenenfalls aromatische, cyclische oder nichtcyclische Alkylgruppe mit 1 bis 25 Kohlenstoffatomen, in die gegebenenfalls ein oder mehrere unter O, N, S und P ausgewählte Heteroatome eingeschoben sind, steht; wobei die Alkylgruppe außerdem gegebenenfalls durch einen oder mehrere unter -OH und Halogenatomen (Cl, Br, I und F) ausgewählte Substituenten substituiert sein kann;
- (x) quaternisierten Monomeren der Formel (I), (II) und (III) und den quaternisierten Formen der obigen zusätzlichen Monomere;
- (xi) multivalenten Verbindungen mit mindestens zwei polymerisierbaren Funktionen vom Vinyl-, (Meth)acryl-, Allyl- oder (Meth)acrylamid-Typ.

45. Polymer nach Anspruch 44, wobei das zusätzliche Monomer unter Vinylneodecanoat, Vinyl-tert.-butylbenzoat; Vinylpyrrolidon; Vinylcaprolactam; N-Vinylformamid; N,N'-Dimethyldiallylammoniumchlorid; Triethylammoniumethylmethacrylatchlorid (MADQUAT); Ethyl(meth)acrylat, Methyl(meth)acrylat, *tert.-*Butyl(meth)acrylat oder Isobornyl(meth)acrylat; Vinylacetat, Crotonsäure; (Meth)acrylsäure, Methacryloylethylbetain, Octylacrylamid; N-Methyl-N-vinylimidazoliniumchlorid, 1-Eicosen, *tert.-*Butylacrylamid, Acrylamid, Hexadecen und Gemischen davon ausgewählt ist.

46. Polymer nach einem der Ansprüche 36 bis 45, ausgewählt unter:
- Poly(vinylpyrrolidon-co-dimethylaminoethylmethacrylat)-Copolymeren;
- Poly(vinylcaprolactam-co-vinylpyrrolidon-co-dimethylaminoethylmethacrylat)-Copolymeren;
- Poly(vinylpyrrolidon-co-dimethylaminopropylmethacrylamid)-Copolymeren;
- Poly(vinylpyrrolidon-co-polyvinylcaprolactam-co-dimethylaminopropylmethacrylamid)-Copolymeren;
- Poly(vinylamin), Poly(allylamin), Poly(diallylamin) und
- Poly(N-vinylformamid-co-vinylamin)-Copolymeren.

47. Polymer nach Anspruch 36, das mindestens 3 Wiederholungseinheiten umfaßt, die für sich alleine oder im Gemisch unter: 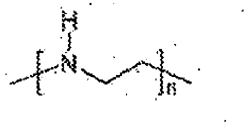 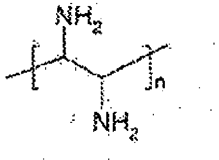 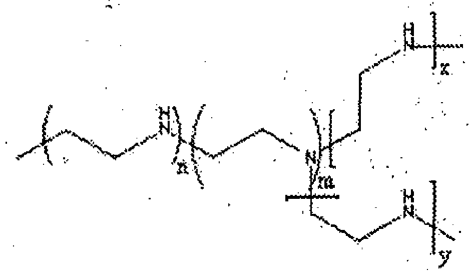 ausgewählt sind.

48. Polymer nach einem der Ansprüche 36 bis 47, wobei das Neutralisationsmittel in einer Menge von 0,01 bis 3 Moläquivalenten, insbesondere 0,05 bis 2,5 Moläquivalenten oder sogar 0,1 bis 2 Moläquivalenten, bezogen auf die gesamten Aminfunktionen des Polymers bzw. der Monomere, zugesetzt ist.

49. Polymer nach einem der Ansprüche 36 bis 48, wobei das Neutralisationsmittel unter:
- (i) Polymeren aus Dextran-Wiederholungseinheiten mit mindestens einer Säurefunktion, insbesondere Carbonsäurefunktion, am Kettenende;
- (ii) säuregruppenterminierten Poly(alkyloxazolinen), vorzugsweise Polymethyloxazolin und Polyethyloxazolin mit mindestens einer Säurefunktion;
- (iii) säurefunktionalisierten, insbesondere carbonsäurefunktionalisierten, Poly(N-methyl)-sarkosinen;
- (iv) Dendrimeren oder hyperverzweigten Molekülen, die durch Säuregruppen, insbesondere Carbonsäuregruppen, oberflächenfunktionalisiert sind, wie zum Beispiel Starburst-PAMAM von Dow Chemical;
- (v) hyperverzweigten Polymeren vom Polyester-Typ, die durch Umsetzung von Adipinsäure mit Glycerin erhalten werden;
- (vi) säuregruppenterminiertem, insbesondere carbonsäuregruppenterminiertem, Poly(glycerin), Poly-(hydroxyethylacrylat) und Poly(vinylpyrrolidon);
- (vii) Polymeren mit mindestens drei Wiederholungseinheiten vom Alkylenglykol-Typ mit 2 bis 4 Kohlenstoffatomen in der Alkylgruppe und Kombinationen davon; genannt seien Polyethylenglykol, Polypropylenglykol, Polyethylen-co-propylenglykol (PEG/PPO), Polytetramethylenoxid-co-polyethylenglykol (PTMO/PEG); mit der Maßgabe, daß sie außerdem mindestens eine Säurefunktion (insbesondere Carbonsäure-, Sulfonsäure- oder Phosphonsäurefunktion) umfassen;
ausgewählt ist.

50. Polymer nach einem der Ansprüche 36 bis 49, wobei es sich bei dem Neutralisationsmittel um ein Polymer handelt, das mindestens drei Wiederholungseinheiten vom Alkylenglykol-Typ, vorzugsweise vom Ethylenglykol-Typ: (-CH₂-CH₂-O-), umfaßt.

51. Polymer nach einem der Ansprüche 36 bis 50, wobei das Neutralisationsmittel unter Polymeren mit den folgenden Strukturen ausgewählt ist:
CH₃-O-(CH₂-CH₂-O-)ₙ-CO-(CH₂-CHR-O)ₘ-CH₂-R₁₀
CH₃-O-(CH₂-CH₂-O-)ₙ-b-(CH₂-CHR-O)ₘ-CH₂-R₁₀
CH₃-O-(CH₂-CH₂-O-)ₙ-CO-(CH₂-CHR-O)ₘ-CH₂-CH₂ R₁₀
CH₃-O-(CH₂-CH₂-O-)ₙ-b-(CH₂-CHR-O)ₘ-CH₂-CH₂R₁₀
CH₃-O-(CH₂-CH₂-O-)ₙ-CO-(CH₂-CHR-O)ₘ-CH₂-CH₂CH₂ R₁₀
CH₃-O-(CH₂-CH₂-O-)ₙ-b-(CH₂-CHR-O)ₘ-CH₂-CH₂CH₂ R₁₀
CH₃-O-(CH₂-CH₂-O-)ₙ-CO-(CH₂-CHR-O)ₘ-CH₂-CH₂OCO-CH₂-CH₂COOH
CH₃-O-(CH₂-CH₂-O-)ₙ-b-(CH₂-CHR-O)ₘ-CH₂-CH₂OCO-CH₂-CH₂COOH
R₁₀-(CH₂-CH₂-O-)ₙ-CO(CH₂-CHR-O)ₘ-CH₂-CH₂ R₁₀
R₁₀-(CH₂-CH₂-O-)ₙ-b-(CH₂-CHR-O)ₘ-CH₂-CH₂ R₁₀
R₁₀-CH₂O-(CH₂-CH₂-O-)ₙ-CO-(CH₂-CHR-O)ₘ-CH₂-R₁₀
R₁₀-CH₂O-(CH₂-CH₂-O-)ₙ-b-(CH₂-CHR-O)ₘ-CH₂-R₁₀
R₁₀-CH₂-CH₂-CH₂-O-(CH₂-CH₂-O-)ₙ-CO-(CH₂-CHR-O)ₘ-CH₂-CH₂-CH₂-R₁₀
R₁₀-CH₂-CH₂-CH₂-O-(CH₂-CH₂-O-)ₙ-b-(CH₂-CHR-O)ₘ-CH₂-CH₂-CH₂-R₁₀
R₁₀-CH₂-CH₂-COO-(CH₂-CH₂-O-)ₙ-CO-(CH₂-CHR-O)ₘ-CH₂-CH₂-O-CO-CH₂-CH₂-R₁₀
R₁₀-CH₂-CH₂-COO-(CH₂-CH₂-O-)ₙ-b-(CH₂-CHR-O)ₘ-CH₂-CH₂-O-CO-CH₂-CH₂-R₁₀
R₁₀-CH₂-CH₂-CONH-(CH₂-CH₂-O-)ₙ-CO-(CH₂-CHR-O)ₘ-CH₂-CH₂-NH-CO-CH₂-CH₂-R₁₀
R₁₀-CH₂-CH₂-CONH-(CH₂-CH₂-O-)ₙ-b-(CH₂-CHR-O)ₘ-CH₂-CH₂-NH-CO-CH₂-CH₂-R₁₀
worin:
- der Rest R gleich oder verschieden ist und für -CH₃, -(CH₂)ₓ-COOH; -(CH₂)ₓ-SO₃H oder (CH₂)ₓ-PO₃H₂ mit x = 1 bis 8 steht;
- der Rest R₁₀ gleich oder verschieden ist und für -COOH, -SO₃H oder -PO₃H₂ steht;
- n für eine ganze Zahl zwischen 3 und 1000 steht und
- m für eine ganze Zahl zwischen 0 und 200 steht;
mit der Maßgabe, daß das Verhältnis n:m größer 5 ist, wenn m nicht gleich null ist.

52. Polymer nach einem der Ansprüche 36 bis 51, wobei das Neutralisationsmittel unter:
CH₃-O-(CH₂-CH₂-O-)ₙ-CH₂-CH₂ R₁₀
CH₃-O-(CH₂-CH₂-O-)ₙ-CH₂-CH₂CH₂ R₁₀
R₁₀-(CH₂-CH₂-O-)ₙ-CH₂-CH₂ R₁₀
R₁₀-CH₂O-(CH₂-CH₂-O-)ₙ-CH₂-R₁₀
worin der Rest R₁₀ für COOH, SO₃H oder -PO₃H₂, vorzugsweise COOH, steht; und n für 3 bis 1000, insbesondere 4 bis 30 oder sogar 5 bis 15 steht; ausgewählt ist.

## Claims

1. Cosmetic or pharmaceutical composition comprising, in a physiologically acceptable, especially cosmetically or pharmaceutically acceptable, medium, a cationic ethylenic polymer comprising at least one primary, secondary or tertiary amine functional group that can be protonated, said functional group being at least partially neutralized by a neutralizing agent, **characterized in that** said neutralizing agent is a polymeric organic acid comprising at least one carboxylic, sulphonic and/or phosphonic acid functional group and is chosen from:
- polymers consisting of dextran repeating units comprising at least one acid, especially carboxylic acid, functional group as a chain end;
- dendrimers or hyperbranched molecules that are functionalized at the surface with acid, especially carboxylic acid, groups;
- polymers comprising at least three alkylene glycol type repeating units for which the alkyl group comprises 2 to 4 carbon atoms, and combinations thereof; being understood that they furthermore comprise at least one carboxylic, sulphonic and/or phosphonic acid functional group chosen from polymers having the following structures:
CH₃-O-(CH₂-CH₂-O-)ₙ-CO-(CH₂-CHR-O)ₘ-CH₂-R₁₀
CH₃-O-(CH₂-CH₂-O-)ₙ-b-(CH₂-CHR-O)ₘ-CH₂-R₁₀
CH₃-O-(CH₂-CH₂-O-)ₙ-CO-(CH₂-CHR-O)ₘ-CH₂-CH₂ R₁₀
CH₃-O-(CH₂-CH₂-O-)ₙ-b-(CH₂-CHR-O)ₘ-CH₂-CH₂ R₁₀
CH₃-O-(CH₂-CH₂-O-)ₙ-CO-(CH₂-CHR-O)ₘ-CH₂-CH₂CH₂ R₁₀
CH₃-O-(CH₂-CH₂-O-)ₙ-b-(CH₂-CHR-O)ₘ-CH₂-CH₂CH₂ R₁₀
CH₃-O-(CH₂-CH₂-O-)ₙ-CO-(CH₂-CHR-O)ₘ-CH₂-CH₂OCO-CH₂-CH₂COOH
CH₃-O-(CH₂-CH₂-O-)ₙ-b-(CH₂-CHR-O)ₘ-CH₂-CH₂OCO-CH₂-CH₂COOH
R₁₀-(CH₂-CH₂-O-)ₙ-CO-(CH₂-CHR-O)ₘ-CH₂-CH₂ R₁₀
R₁₀-(CH₂-CH₂-O-)ₙ-b-(CH₂-CHR-O)ₘ-CH₂-CH₂ R₁₀
R₁₀-CH₂O-(CH₂-CH₂-O-)ₙ-CO-(CH₂-CHR-O)ₘ-CH₂-R₁₀
R₁₀-CH₂O-(CH₂-CH₂-O-)ₙ-b-(CH₂-CHR-O)ₘ-CH₂-R₁₀
R₁₀-CH₂-CH₂-CH₂-O-(CH₂-CH₂-O-)ₙ-CO-(CH₂-CHR-O)ₘ-CH₂-CH₂-CH₂-R₁₀
R₁₀-CH₂-CH₂-CH₂-O-(CH₂-CH₂-O-)ₙ-b-(CH₂-CHR-O)ₘ-CH₂-CH₂-CH₂-R₁₀
R₁₀-CH₂-CH₂-COO-(CH₂-CH₂-O-)ₙ-CO-(CH₂-CHR-O)ₘ-CH₂-CH₂-O-CO-CH₂-CH₂-R₁₀
R₁₀-CH₂-CH₂-COO-(CH₂-CH₂-O-)ₙ-b-(CH₂-CHR-O)ₘ-CH₂-CH₂-O-CO-CH₂-CH₂-R₁₀
R₁₀-CH₂-CH₂-CONH-(CH₂-CH₂-O-)ₙ-CO-(CH₂-CHR-O)ₘ-CH₂-CH₂-NH-CO-CH₂-CH₂-R₁₀
R₁₀-CH₂-CH₂-CONH-(CH₂-CH₂-O-)ₙ-b-(CH₂-CHR-O)ₘ-CH₂-CH₂-NH-CO-CH₂-CH₂-R₁₀
in which:
- the R radical, being identical or different, represents -CH₃, -(CH₂)ₓ-COOH; -(CH₂)ₓ-SO₃H; (CH₂)ₓ-PO₃H₂; with x = 1 to 8;
- the R₁₀ radical, being identical or different, is -COOH, -SO₃H or -PO₃H₂;
- n is an integer between 3 and 1000;
- m is an integer between 0 and 200,
given that the n/m ratio is greater than 5, when m is non zero.

2. Composition according to Claim 1, in which the polymer comprises at least one monomer of formula (I), alone or as a mixture: 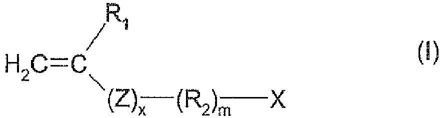 in which:
- R₁ is a hydrogen atom or a linear or branched, hydrocarbon-based radical of CₚH₂ₚ₊₁ type, with p being an integer between 1 and 12 inclusive;
- Z is a divalent group chosen from -COO-, -CONH-, -CONCH₃-, -OCO- or -O-, -SO₂-, -CO-O-CO- or -CO-CH₂-CO-; preferably, COO and CONH;
- x is 0 or 1, preferably 1;
- R₂ is a saturated or unsaturated, optionally aromatic, linear, branched or cyclic, carbon-based divalent radical having 1 to 30 carbon atoms, which may comprise 1 to 18 heteroatoms chosen from O, N, S, F, Cl, Br, Si and P;
- m is 0 or 1;
- X is
(a) a guanidino or amidino group, or else;
(b) a group of formula -N(R₆)(R₇) with R₆ and R₇ representing, independently of one another, (i) a hydrogen atom, (ii) a linear, branched or cyclic, saturated or unsaturated, optionally aromatic alkyl group comprising from 1 to 18 carbon atoms, which may comprise 1 to 10 heteroatoms chosen from O, N, S, F, Si and P; or (iii) R₆ and R₇ form, with the nitrogen atom, a ring of formula: 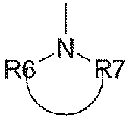 that is saturated or unsaturated, optionally aromatic, comprising in total 5, 6, 7, or 8 atoms, and especially 4, 5 or 6 carbon atoms and/or 2 to 4 heteroatoms chosen from O, S and N; said first ring possibly being fused with one or more other rings, which are saturated or unsaturated, optionally aromatic, each comprising 5, 6 or 7 atoms, and especially 4, 5, 6 or 7 carbon atoms and/or 2 to 4 heteroatoms chosen from O, S and N;
(c) a ring: 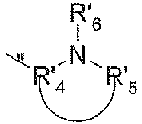
in which R'₄ and R'₅ form, with the nitrogen atom, a saturated or unsaturated, optionally aromatic, ring comprising in total 5, 6, 7 or 8 atoms, and especially 4, 5 or 6 carbon atoms and/or 2 to 4 heteroatoms chosen from O, S and N; said ring possibly being fused with one or more other rings, which are saturated or unsaturated, optionally aromatic, each comprising 5, 6 or 7 atoms, and especially 4, 5, 6, 7 or 8 carbon atoms and/or 2 to 4 heteroatoms chosen from O, S and N;
and R'₆ is chosen from H, -CH₃ and -C₂H₅;
being understood that said monomer of formula (I) comprises at least one primary, secondary or tertiary amine functional group which can be protonated at a pH chosen between pH 1 and pH 12; this functional group possibly being, or being borne by, the radicals R₂ and/or X.

3. Composition according to Claim 2, in which, in the monomer of formula (I), R₂ is or comprises:
- an alkylene radical such as methylene, ethylene, propylene, n-butylene, isobutylene, *tert*-butylene, n-hexylene, n-octylene, n-dodecylene, n-octadecylene, n-tetradecylene, n-docosanylene;
- a phenylene radical (ortho, meta or para)-C₆H₄- optionally substituted by a C₁-C₁₂ alkyl radical optionally comprising 1 to 25 heteroatoms chosen from N, O, S, F, Si and/or P; or else a benzylene radical -C₆H₄-CH₂-optionally substituted by a C₁-C₁₂ alkyl radical optionally comprising 1 to 25 heteroatoms chosen from O, N, S, F, Si and P;
- a radical of formula -CO-, -O-CO-O-, -CO-O-, -O-, -O-CO-NH-, -CO-NH-, -NHCO-, -N(R')CO-, -NH-CO-NH-, -NR'-, epoxy, -N-CO- with R' representing a C₁-C₂₂ linear or branched alkyl radical optionally comprising 1 to 12 heteroatoms chosen from O, N, S, F, Si and P; or
- a mixture of these radicals.

4. Composition according to one of Claims 2 to 3, in which, in the monomer of formula (I), X is an aromatic or non-aromatic heterocycle containing a secondary or tertiary nitrogen, especially a radical of indolyl, isoindolinyl, imidazolyl, imidazolinyl, piperidinyl, pyrazolynyl, pyrazolyl, quinoline, pyridinyl, piperazinyl, pyrrolidinyl, quinidinyl, thiazolinyl, morpholine, guanidino or amidino type, and mixtures thereof.

5. Composition according to one of Claims 2 to 4, in which the monomer of formula (I) is chosen from, alone or as a mixture, dimethylaminopropyl (meth)acrylamide, dimethylaminoethyl (meth)acrylamide, diethylaminoethyl (meth)acrylate, dimethylaminoethyl (meth)acrylate, tert-butylaminoethyl (meth)acrylate, morpholinoethyl (meth)acrylate, vinylimidazole, vinylpyridine, vinylamine, allylamine, and the monomers below in which R = H or methyl:

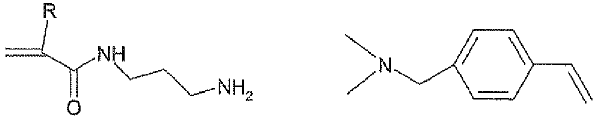

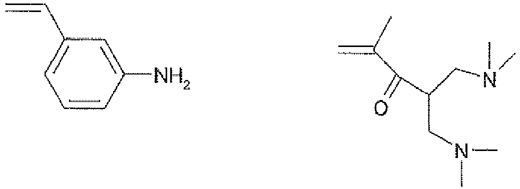

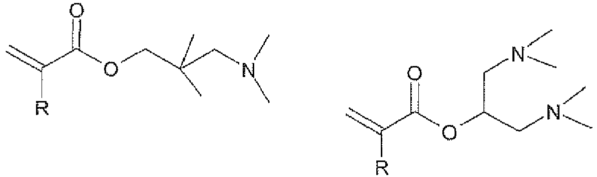

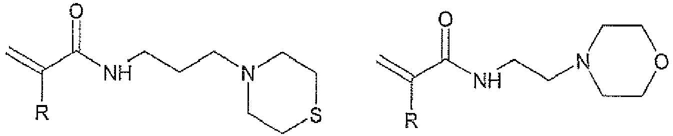

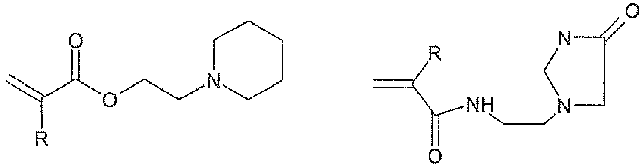

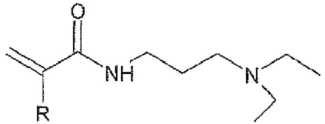

6. Composition according to Claim 1, in which the polymer comprises at least one monomer of formula (II), alone or as a mixture: 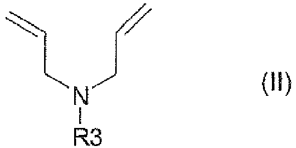 in which R₃ is hydrogen or a linear, branched or cyclic, saturated or unsaturated, optionally aromatic, carbon-based, especially hydrocarbon-based, radical having 1 to 30 carbon atoms, which may comprise 1 to 18 heteroatoms chosen from O, N, S, F, Cl, Br, Si and P,
being understood that the monomer of formula (II) comprises at least one primary, secondary or tertiary amine functional group which can be protonated at a pH chosen between pH 1 and pH 12.

7. Composition according to Claim 6, in which R₃ is chosen from:
- an alkyl radical such as methyl, ethyl, propyl, n-butyl, isobutyl, *tert*-butyl, n-hexyl, n-octyl, n-dodecyl, n-octadecyl, n-tetradecyl, n-docosanyl; optionally comprising 1 to 25 heteroatoms chosen from O, N, S, F, Si and P and/or optionally substituted by at least one of the aforementioned groups;
- a phenyl radical -C₆H₅ optionally substituted by a C₁-C₁₂ alkyl radical optionally comprising 1 to 25 heteroatoms chosen from N, O, S, F, Cl, Br, Si and/or P; and/or optionally substituted by at least one of the aforementioned groups;
- a benzyl radical -C₆H₄-CH₃ optionally substituted by a C₁-C₁₂ alkyl radical optionally comprising 1 to 25 heteroatoms chosen from O, N, S, F, Cl, Br, Si and P; and/or optionally substituted by at least one of the aforementioned groups; or
- a mixture of these radicals.

8. Composition according to one of Claims 6 to 7, in which the monomer of formula (II) is chosen from, alone or as a mixture, N-methyldiallylamine and diallylamine.

9. Composition according to Claim 1, in which the polymer comprises at least one monomer of formula (III), alone or as a mixture: 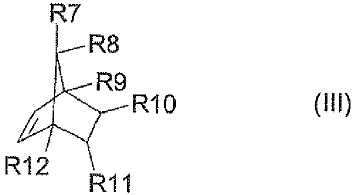 in which R₇ to R₁₂, independently of one another, represent:
- a hydrogen atom;
- an -NR₁₃R'₁₃ group with R₁₃ and R'₁₃, independently of one another, representing hydrogen or a linear, branched or cyclic, saturated or unsaturated, optionally aromatic alkyl comprising from 1 to 18 carbon atoms, which may comprise 1 to 10 heteroatoms chosen from O, N, S, F, Si and P; and in particular R₁₃ and R'₁₃ may be chosen from hydrogen, a methyl, ethyl, propyl, isopropyl, n-butyl, t-butyl, isobutyl, octyl, lauryl or stearyl group; and
- a linear, branched or cyclic, saturated or unsaturated, optionally aromatic, carbon-based, especially hydrocarbon-based, radical having 1 to 30 carbon atoms, possibly comprising 1 to 18 heteroatoms chosen from O, N, S, F, Si and P,
being understood that the monomer of formula (III) comprises at least one primary, secondary or tertiary amine functional group that can be protonated at a pH chosen between pH 1 and pH 12.

10. Composition according to Claim 9, in which R₇ to R₁₂ may be:
- H;
- NH₂;
- an alkyl radical such as methyl, ethyl, propyl, n-butyl, isobutyl, *tert*-butyl, n-hexyl, n-octyl, n-dodecyl, n-octadecyl, n-tetradecyl, n-docosanyl; optionally comprising 1 to 25 heteroatoms chosen from O, N, S, F, Si and P and/or optionally substituted by at least one of the aforementioned groups;
- a phenyl radical -C₆H₅ optionally substituted by a C₁-C₁₂ alkyl radical optionally comprising 1 to 25 heteroatoms chosen from O, N, S, F, Si and/or P; and/or optionally substituted by at least one of the aforementioned groups;
- a benzyl radical -C₆H₄-CH₃ optionally substituted by a C₁-C₁₂ alkyl radical optionally comprising 1 to 25 heteroatoms chosen from N, O, S, F, Si and P; and/or optionally substituted by at least one of the aforementioned groups; or
- a mixture of these radicals.

11. Composition according to one of Claims 9 to 10, in which the monomer of formula (III) is chosen from, alone or as a mixture:

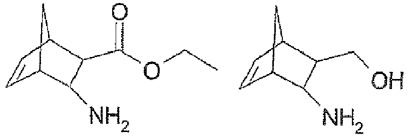

12. Composition according to one of the preceding claims, in which the monomers of formula (I), (II) and/or (III), represent 1 to 100% by weight of the weight of the final polymer, especially 1 to 99.9% by weight, in particular 30 to 95% by weight, preferably 35 to 90% by weight, or even 40 to 85% by weight.

13. Composition according to one of the preceding claims, in which the polymer moreover comprises 0.01 to 99% by weight relative to the weight of the final polymer, especially 5 to 70% by weight, preferably 10 to 65% by weight, or even 15 to 60% by weight, of additional monomers.

14. Composition according to Claim 13, in which the additional monomer is chosen from the following monomers, alone or as a mixture, and also their salts:
- (i) (meth)acrylic acid esters of formula CH₂=CHCOOR'1 or CH₂=C(CH₃)COOR'1 with R'₁ representing a linear, branched or cyclic, saturated or unsaturated, optionally aromatic (aryl, aralkyl or alkylaryl) carbon-based, especially hydrocarbon-based (alkyl) chain having 1 to 30 carbon atoms, optionally comprising 0 to 2 ether functional groups (-O-) and optionally 0 to 12, especially 1 to 8, functional groups chosen from -OH (hydroxyl), -OR' with R' a C₁-C₆ alkyl (alkoxy), -CN, -X (halogen, especially Cl, F, Br or I), -CO-, -SO₃H, -COOH, -OCOO-, -COO-, -OCONR-, -OCONH-, -NH-CONH-, -NR-CONR-, -CF₃, -CN, epoxy, -NHCO-, -N(R)CO- with R = linear or branched C₁-C₂₂ alkyl optionally comprising 1-12 heteroatoms;
- (ii) (meth)acrylic acid amides of formula CH₂=CHCONR'₂R"₂ or CH₂=C(CH₃)CONR'₂R"₂, with R'₂, R"₂, being identical or different, representing hydrogen or a linear, branched or cyclic, saturated or unsaturated, optionally aromatic, carbon-based, especially hydrocarbon-based (alkyl), chain having 1 to 30 carbon atoms, optionally comprising 0 to 2 ether functional groups (-O-) and optionally 0 to 12, especially 1 to 8, functional groups chosen from -OH (hydroxyl), -OR' with R' a C₁-C₆ alkyl (alkoxy), -CN, -X (halogen, especially Cl, F, Br or I), -CO-, -SO₃H, -COOH, -OCOO-, -COO-, -OCONR-, -OCONH-, -NH-CONH-, -NR-CONR-, -CF₃, -CN, epoxy, -NHCO-, -N(R)CO- with R = linear or branched C₁-C₂₂ alkyl optionally comprising 1-12 heteroatoms;
- (iii) vinyl esters of formula CH₂=CH-OCO-R'₃ or CH₂=C(CH₃)-OCO-R'₃ with R'₃ representing a linear, branched or cyclic, saturated or unsaturated, optionally aromatic, carbon-based, especially hydrocarbon-based, chain having 1 to 30 carbon atoms, optionally comprising 0 to 2 ether functional groups (-O-) and optionally 0 to 12, especially 1 to 8, functional groups chosen from -OH (hydroxyl), -OR' with R' a C₁-C₆ alkyl (alkoxy), -CN, -X (halogen, especially Cl, F, Br or I), -CO-, -SO₃H, -COOH, -OCOO-, -COO-, -OCONR-, -OCONH-, -NH-CONH-, -NR-CONR-, -CF₃, -CN, epoxy, -NHCO-, -N(R)CO- with R = linear or branched C₁-C₂₂ alkyl optionally comprising 1-12 heteroatoms;
- (iv) vinyl ethers of formula CH₂=CHOR'₄ or CH₂=C(CH₃)OR'₄ with R'₄ representing a linear, branched or cyclic, saturated or unsaturated, optionally aromatic, carbon-based, especially hydrocarbon-based, chain having 1 to 30 carbon atoms, optionally comprising 0 to 2 ether functional groups (-O-) and optionally 0 to 12, especially 1 to 8, functional groups chosen from -OH (hydroxyl), -OR' with R' a C₁-C₆ alkyl (alkoxy), -CN, -X (halogen, especially Cl, F, Br or I), -CO-, -SO₃H, -COOH, -OCOO-, -COO-, -OCONR-, -OCONH-, -NH-CONH-, -NR-CONR-, -CF₃, -CN, epoxy, -NHCO-, -N(R)CO- with R = linear or branched C₁-C₂₂ alkyl optionally comprising 1-12 heteroatoms;
- (v) vinyl compounds of formula CHR"₅=CR₅R'₅ in which:
R"₅ is H or COOH, and
R₅ is H, CN or COOH, and
R'₅ is chosen from:
- a hydrogen atom, or a group chosen from -OH, -CH=O, halogen (Cl, Br, I in particular), -COOH, -CH₂COOH, -NHC (O)H, -N(CH₃)-C(O)H, -NHC (O)CH₃, -N(CH₃)-C(O)CH₃;
- a ring: 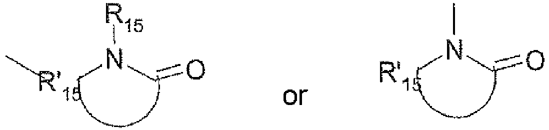 where R'₁₅ and R₁₅ represent, independently of one another, H, a linear or branched, saturated or unsaturated, optionally aromatic, cyclic or non-cyclic alkyl group, comprising 1 to 25 carbon atoms, optionally inserted into which are one or more heteroatoms chosen from O, N, S and P; said alkyl group possibly, moreover, being optionally substituted by one or more substituents chosen from -OH and halogen atoms (Cl, Br, I and F);
- a linear or branched alkyl group comprising 1 to 25 carbon atoms;
- a linear or branched alkyl group comprising 1 to 25 carbon atoms;
- a C₃ to C₈ cycloalkyl group such as cyclohexane;
- a C₆ to C₂₀ aryl group such as phenyl;
- a C₇ to C₃₀ aralkyl group (C₁ to C₄ alkyl group) such as 2-phenylethyl or benzyl;
- a heterocyclic group having 4 to 12 chain members containing one or more heteroatoms chosen from O, N and S; and
- a heterocycloalkyl group (alkyl having 1 to 4 carbons), such as furfuryl, furfurylmethyl or tetrahydrofurfurylmethyl,
said alkyl, cycloalkyl, aryl, aralkyl, heterocyclic or heterocycloalkyl groups possibly optionally comprising 0 to 2 ether functional groups (-O-) and optionally 0 to 12, especially 1 to 8, functional groups chosen from -OH (hydroxyl), -OR' with R' a C₁-C₆ alkyl (alkoxy), -CN, -X (halogen, especially Cl, F, Br or I), -CO-, -SO₃H, -COOH, -OCOO-, -COO-, - OCONR-, -OCONH-, -NH-CONH-, -NR-CONR-, -CF₃, -CN, epoxy, -NHCO-, -N(R)CO- with R = linear or branched C₁-C₂₂ alkyl optionally comprising 1-12 heteroatoms;
and/or possibly being optionally substituted by one or more linear or branched, C₁-C₄ alkyl groups, themselves optionally comprising 0 to 2 ether functional groups (-O-) and optionally 0 to 12, especially 1 to 8, functional groups chosen from -OH (hydroxyl), -OR' with R' a C₁-C₆ alkyl (alkoxy), -CN, -X (halogen, especially Cl, F, Br or I), -CO-, -SO₃H,-COOH, -OCOO-, -COO-, -OCONH-, -NH-CONH-, -CF₃, -CN, epoxy, -NHCO-, -N(R)CO- with R = linear or branched C₁-C₂₂ alkyl optionally comprising 1-12 heteroatoms,
- (vii) the following anionic monomers, and salts thereof:
maleic anhydride, acrylic acid, methacrylic acid, crotonic acid, itaconic acid, fumaric acid, maleic acid, 2-carboxyethyl acrylate (CH₂=CH-C(O)-O-(CH₂)₂-COOH), styrenesulphonic acid, 2-acrylamido-2-methylpropanesulphonic acid, vinylbenzoic acid, vinylphosphonic acid, sulphopropyl (meth)acrylate, and among the salts: sodium or potassium (meth)acrylate;
- (viii) the following amphoteric monomers:
N,N-dimethyl-N-(2-methacryloyloxyethyl)-N-(3-sulphopropyl)ammonium betaine (especially SPE from Raschig); N,N-dimethyl-N-(3-methacrylamidopropyl)-N-(3-sulphopropyl)ammonium betaine (SPP from Raschig), and 1-(3-sulphopropyl)-2-vinylpyridinium betaine (SPV from Raschig), and also 2-(methacryloyloxy)ethyl phosphorylcholine;
- (ix) monomers of formula: 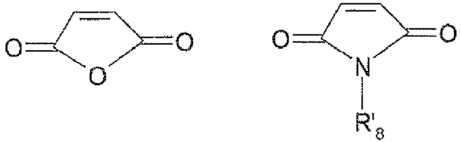 in which R'₈ is H or a linear or branched, saturated or unsaturated, optionally aromatic, cyclic or non-cyclic alkyl group comprising 1 to 25 carbon atoms, optionally inserted into which are one or more heteroatoms chosen from O, N, S and P; said alkyl group possibly, moreover, being optionally substituted by one or more substituents chosen from -OH and halogen atoms (Cl, Br, I and F);
- (x) quaternized monomers of formula (I), (II) and (III), and the quaternized forms of the additional monomers above;
- (xi) multivalent compounds comprising at least two polymerizable functional groups of the vinyl, (meth)acrylic, allyl or (meth)acrylamide type.

15. Composition according to Claim 14, in which the additional monomer is chosen from, alone or as a mixture: methyl, ethyl, propyl, isopropyl, n-butyl, isobutyl, *tert*-butyl, hexyl, cyclohexyl, 2-ethylhexyl, octyl, isooctyl, isodecyl, decyl, dodecyl, myristyl, cetyl, palmityl, stearyl, behenyl, oleyl, tridecyl, hexadecyl, isobornyl, hydroxyethyl, hydroxypropyl, phenyl, benzyl, furfuryl; tetrahydrofurfuryl, ethoxyethyl, methoxyethyl; glycerol, 2,2,2,-trifluoroethyl and poly(ethylene-isobutylene) (meth)acrylates; (meth)acrylonitriles; (meth)acrylamide, N-methyl(meth)acrylamide, N-isopropyl(meth)acrylamide, N-tert-butyl(meth)acrylamide, N-octyl(meth)acrylamide, N-undecyl(meth)acrylamide, N,N-dimethyl(meth)acrylamide, N,N-dibutyl(meth)acrylamide; vinyl acetate, vinyl propionate, vinyl butyrate (or butanoate), vinyl ethylhexanoate, vinyl neononanoate and vinyl neododecanoate, vinyl neodecanoate, vinyl pivalate, vinyl cyclohexanoate, vinyl benzoate, vinyl 4-*tert-*butylbenzoate, vinyl trifluoroacetate; methyl vinyl ether, ethyl vinyl ether, ethylhexyl vinyl ether and butyl vinyl ether, cyclohexyl vinyl ether, isobutyl vinyl ether; vinyl alcohol, vinylcyclohexane; vinylpyrrolidone, vinylcaprolactam, N-vinylacetamide, N-vinylformamide, N-methyl-N-vinylformamide, N-vinylacetamide; N-methyl-N-vinylacetamide; styrene, methylstyrene, 4-*tert*-butylstyrene, 4-acetoxystyrene, 4-methoxystyrene, 3-methylstyrene, 4-methylstyrene, 2-chlorostyrene, 3-chlorostyrene, 4-chlorostyrene, dimethylstyrene, 2,6-dichlorostyrene, 2,4-dimethylstyrene; 2,5-dimethylstyrene, 3,5-ethoxystyrene, 2,4-ethoxystyrene, 4-fluorostyrene; vinyl butyral; vinylcarbazole; vinylchloride; vinyl formal; vinylidene chloride, vinylidene fluoride, 2-vinylnaphthalene; N-methylmaleimide; 1-octene, 1-butene, cis-chlorobutadiene, trans-chlorobutadiene, chlorotrifluoroethylene; cis-isoprene, trans-isoprene, 1-octadecene, butadiene, hexadecene and eicosene; maleic anhydride and N-methylmaleimide; 1,3-butanediol di(meth)acrylate; 1,6-hexanediol di(meth)acrylate, N,N'-dimethyldiallylammonium chloride; triethylammonium ethyl methacrylate chloride (MADQUAT), 4-methylvinylpyridinium chloride, N-methyl-N-vinylimidazolinium chloride, trimethylammonium propyl (meth)acrylamide chloride.

16. Composition according to one of Claims 14 to 15, in which the additional monomer is chosen from vinyl neodecanoate, vinyl *tert*-butylbenzoate; vinylpyrrolidone; vinylcaprolactam; N-vinylformamide; N,N'-dimethyldiallylammonium chloride; triethylammonium ethylmethacrylate chloride; ethyl, methyl, *tert*-butyl or isobornyl (meth)acrylate; vinyl acetate, crotonic acid; (meth)acrylic acid, methacryloyl ethyl betaine, octylacrylamide; N-methyl-N-vinylimidazolinium chloride, 1-eicosene, *tert*-butylacrylamide, acrylamide, hexadecene, and mixtures thereof.

17. Composition according to one of the preceding claims, in which the polymer is in the form of a homopolymer or a copolymer which may be linear or branched, crosslinked or uncrosslinked; random, alternating block or gradient, even star-shaped; preferably linear, random or block.

18. Composition according to one of the preceding claims, in which the polymer is chosen from:
- poly(vinylpyrrolidone-co-dimethylaminoethyl methacrylate) copolymers;
- poly(vinylcaprolactam-co-vinylpyrrolidone-co-dimethylaminoethyl methacrylate) copolymers;
- poly(vinylpyrrolidone-co-dimethylaminopropyl methacrylamide) copolymers;
- poly(vinylpyrrolidone-co-polyvinylcaprolactam-co-dimethylaminopropyl methacrylamide) copolymers;
- poly(vinylamine), poly(allylamine), poly-(diallylamine); and
- poly(N-vinylformamide-co-vinylamine) copolymers.

19. Composition according to one of the preceding claims, in which the polymer has a weight-average molecular weight (M_{w}) between 1000 and 3,000,000, especially between 1500 and 1,000,000 and more preferably between 2000 and 800,000, and even better between 2500 and 500,000.

20. Composition according to Claim 1, in which the polymer comprises at least 3 repeating units chosen, alone or as mixtures, from:

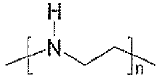

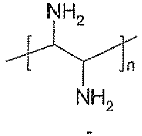

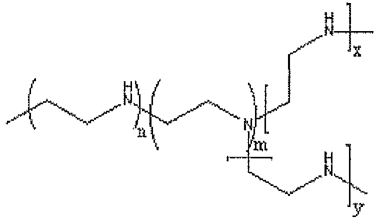

21. Composition according to one of the preceding claims, in which the neutralizing agent is added in an amount of 0.01 to 3 molar equivalents, especially 0.05 to 2.5, or even 0.1 to 2 molar equivalents, relative to the total amine functional groups of the polymer or of the monomers.

22. Composition according to one of the preceding claims, in which the neutralizing agent is added in a stoichiometric amount relative to the total amine functional groups of the polymer or of the monomers.

23. Composition according to one of the preceding claims, in which the neutralizing agent has a number-average molecular weight (Mn) between 300 and 50,000, especially between 350 and 20,000, and more preferably between 400 and 10,000, or even 450 and 5000.

24. Composition according to one of the preceding claims, in which the neutralizing agent is linear and comprises two acid functional groups, one at each end of the chain.

25. Composition according to one of the preceding claims, in which the neutralizing agent is chosen from:
polymers comprising at least three alkylene glycol type repeating units for which the alkyl group comprises 2 to 4 carbon atoms, and combinations thereof; chosen from polymers having the following structures:
CH₃-O-(CH₂-CH₂-O-)ₙ-CO-(CH₂-CHR-O)ₘ-CH₂-R₁₀
CH₃-O-(CH₂-CH₂-O-)ₙ-b-(CH₂-CHR-O)ₘ-CH₂-R₁₀
CH₃-O-(CH₂-CH₂-O-)ₙ-CO-(CH₂-CHR-O)ₘ-CH₂-CH₂ R₁₀
CH₃-O-(CH₂-CH₂-O-)ₙ-b-(CH₂-CHR-O)ₘ-CH₂-CH₂ R₁₀
CH₃-O-(CH₂-CH₂-O-)ₙ-CO-(CH₂-CHR-O)ₘ-CH₂-CH₂CH₂ R₁₀
CH₃-O-(CH₂-CH₂-O-)ₙ-b-(CH₂-CHR-O)ₘ-CH₂-CH₂CH₂ R₁₀
CH₃-O-(CH₂-CH₂-O-)ₙ-CO-(CH₂-CHR-O)ₘ-CH₂-CH₂OCO-CH₂-CH₂COOH
CH₃-O-(CH₂-CH₂-O-)ₙ-b-(CH₂-CHR-O)ₘ-CH₂-CH₂OC-CH₂-CH₂COOH
R₁₀-(CH₂-CH₂-O-)ₙ-CO-(CH₂-CHR-O)ₘ-CH₂-CH₂ R₁₀
R₁₀-(CH₂-CH₂-O-)ₙ-b-(CH₂-CHR-O)ₘ-CH₂-CH₂ R₁₀
R₁₀-CH₂O-(CH₂-CH₂-O-)ₙ-CO-(CH₂-CHR-O)ₘ-CH₂-R₁₀
R₁₀-CH₂O-(CH₂-CH₂-O-)ₙ-b-(CH₂-CHR-O)ₘ-CH₂-R₁₀
R₁₀-CH₂-CH₂-CH₂-O-(CH₂-CH₂-O-)ₙ-CO-(CH₂-CHR-O)ₘ-CH₂-CH₂-CH₂-R₁₀
R₁₀-CH₂-CH₂-CH₂-O-(CH₂-CH₂-O-)ₙ-b-(CH₂-CHR-O)ₘ-CH₂-CH₂-CH₂-R₁₀
R₁₀-CH₂-CH₂-COO-(CH₂-CH₂-O-)ₙ-CO-(CH₂-CHR-O)ₘ-CH₂-CH₂-O-CO-CH₂-CH₂-R₁₀
R₁₀-CH₂-CH₂-COO-(CH₂-CH₂-O-)ₙ-b-(CH₂-CHR-O)ₘ-CH₂-CH₂-O-CO-CH₂-CH₂-R₁₀
R₁₀-CH₂-CH₂-CONH-(CH₂-CH₂-O-)ₙ-CO-(CH₂-CHR-O)ₘ-CH₂-CH₂-NH-CO-CH₂-CH₂-R₁₀
R₁₀-CH₂-CH₂-CONH-(CH₂-CH₂-O-)ₙ-b-(CH₂-CHR-O)ₘ-CH₂-CH₂-NH-CO-CH₂-CH₂-R₁₀
in which:
- the R radical, being identical or different, represents -CH₃, -(CH₂)ₓ-COOH; -(CH₂)ₓ-SO₃H; (CH₂)ₓ-PO₃H₂; with x = 1 to 8;
- the R₁₀ radical, being identical or different, is -COOH, -SO₃H or -PO₃H₂;
- n is an integer between 3 and 1000;
- m is an integer between 0 and 200,
- given that the n/m ratio is greater than 5, when m is non zero.

26. Composition according to Claim 25, in which the neutralizing agent is chosen from the polymers for which:
- R represents methyl, or -(CH₂)ₓ-COOH; -(CH₂)ₓ-SO₃H; -(CH₂)ₓ-PO₃H₂ with x = 2 to 3; and/or
- n represents 4 to 30, especially 5 to 15.

27. Composition according to one of the preceding claims, in which the neutralizing agent is chosen from:
CH₃-O-(CH₂-CH₂-O-)ₙ-CO-(CH₂-CHR-O)ₘ-CH₂-CH₂ R₁₀
CH₃-O-(CH₂-CH₂-O-)ₙ-CO-(CH₂-CHR-O)ₘ-CH₂-CH₂CH₂ R₁₀
R₁₀-(CH₂-CH₂-O-)ₙ-CO-(CH₂-CHR-O)ₘ-CH₂-CH₂ R₁₀
R₁₀-CH₂O-(CH₂-CH₂-O-)ₙ-CO-(CH₂-CHR-O)ₘ-CH₂-R₁₀
in which the R₁₀ radical is -COOH, -SO₃H or -PO₃H₂; preferably COOH; m is 0 to 200 and n is 3 to 1000, especially 4 to 30, or even 5 to 15.

28. Composition according to one of the preceding claims, in which the neutralizing agent is chosen from:
CH₃-O-(CH₂-CH₂-O-)ₙ-CH₂-CH₂ R₁₀
CH₃-O-(CH₂-CH₂-O-)ₙ-CH₂-CH₂CH₂ R₁₀
R₁₀-(CH₂-CH₂-O-)ₙ-CH₂-CH₂ R₁₀
R₁₀-CH₂O-(CH₂-CH₂-O-)ₙ-CH₂-R₁₀
in which the R₁₀ radical is -COOH, -SO₃H or -PO₃H₂; preferably COOH; and n is 3 to 1000, especially 4 to 30, or even 5 to 15.

29. Composition according to one of the preceding claims, in which the neutralizing agent is chosen from:
- CH₃-O-(CH₂-CH₂-O-)ₙ-CH₂-CH₂-SO₃H;
- CH₃-O-(CH₂-CH₂-O-)ₙ-CH₂-CH₂-CH₂COOH;
- HOCO-(CH₂-CH₂-O-)ₙ-CH₂-CH₂COOH; and
- HOCO-CH₂O-(CH₂-CH₂-O-)ₙ-CH₂-COOH;
with n = 3 to 1000, especially 4 to 30, or even 5 to 15.

30. Composition according to one of Claims 1 to 29, in which the polymer is present in an amount of 0.01 to 50% by weight of dry matter, especially 0.1 to 30% by weight, or even 0.3 to 10% by weight, even better from 1 to 3% by weight, relative to the total weight of the cosmetic or pharmaceutical composition.

31. Composition according to Claim 30, furthermore comprising at least one constituent chosen from: water, hydrophilic organic solvents such as alcohols and especially linear or branched C₁-C₆ monoalcohols, such as ethanol, *tert*-butanol, n-butanol, isopropanol or n-propanol, and polyols such as glycerol, diglycerol, propylene glycol, sorbitol, pentylene glycol, and polyethylene glycols; glycol ethers especially C₂ glycol ethers and hydrophilic C₂-C₄ aldehydes; waxes, pasty fatty substances, gums; lipophilic organic solvents; oils and especially hydrocarbon-based oils of animal origin such as perhydrosqualene; hydrocarbon-based vegetable oils such as the liquid triglycerides of fatty acids having 4 to 10 carbon atoms such as heptanoic or octanoic acid triglycerides, or else sunflower, maize, soya bean, grape seed, sesame, apricot, macadamia, castor, or avocado oils, caprylic/capric acid triglycerides, jojoba oil or shea butter oil; linear or branched hydrocarbons of mineral or synthetic origin such as paraffin oils and derivatives thereof, vaseline, polydecenes, hydrogenated polyisobutene such as parleam; synthetic esters and ethers especially of fatty acids such as for example Purcellin oil, isopropyl myristate, 2-ethylhexyl palmitate, 2-octyldodecyl stearate, 2-octyldodecyl erucate, isostearyl isostearate; hydroxylated esters such as isostearyl lactate, octyl hydroxystearate, octyldodecyl hydroxystearate, diisostearyl malate, triisocetyl citrate, and heptanoates, octanoates and decanoates of fatty alcohols; polyol esters such as propylene glycol dioctanoate, neopentyl glycol diheptanoate, diethylene glycol diisononanoate; and pentaerythritol esters; fatty alcohols having 12 to 26 carbon atoms such as octyldodecanol, 2-butyloctanol, 2-hexyldecanol, 2-undecylpentadecanol, oleyl alcohol; partially hydrocarbon-based and/or silicone-based fluoro oils; silicone oils such as volatile or non-volatile, linear or cyclic polymethylsiloxanes (PDMS) that are liquid or pasty at ambient temperature such as cyclomethicones or dimethicones, optionally comprising a phenyl group, such as phenyltrimethicones, phenyltrimethylsiloxydiphenylsiloxanes, diphenylmethyldimethyltrisiloxanes, diphenyldimethicones, phenyldimethicones, polymethylphenylsiloxanes; ketones that are liquid at ambient temperature; polypropylene glycol ethers that are liquid at ambient temperature; short-chain esters (having from 3 to 8 carbon atoms in total); ethers that are liquid at 25°C; alkanes that are liquid at 25°C; cyclic aromatic compounds that are liquid at 25°C; aldehydes that are liquid at 25°C; pigments, nacres, fillers; water-soluble dyes; liposoluble dyes; polymers, especially film-forming polymers; anionic, amphoteric, non-ionic or cationic surfactants or mixtures thereof; vitamins, fragrances, nacres, thickeners, gelling agents, trace elements, demulcent agents, sequestering agents, fragrances, basifying or acidifying agents, preservatives, sunscreens, surfactants, antioxidants, agents for combating hair loss, antidandruff agents, propellants or ceramides; or mixtures of these constituents.

32. Composition according to one of Claims 1 to 31, being in the form of a product for caring for and/or making up the skin of the body or face, the lips and hair, a suntan or self-tanning product, a hair product, especially for form retention of the hairstyle or hair shaping.

33. Composition according to one of Claims 1 to 32, being in the form of shampoos, gels, hair setting lotions, blow-drying lotions, fixing and styling compositions such as lacquers or sprays, shower gels, foam baths; rinse-out or leave-in conditioner, perming, hair straightening, dyeing or bleaching compositions, or else in the form of rinse-out compositions, to be applied before or after a dyeing, bleaching, perming or hair straightening operation or else between the two steps of a perming or a hair straightening operation; washing compositions for the skin, and in particular in the form of solutions or gels for the bath or shower or makeup removers; aqueous or aqueous/alcoholic lotions for caring for the skin and/or hair.

34. Method for the cosmetic treatment of keratinous substances such as the skin of the body or face, the nails, hair, eyelashes and/or eyebrows, **characterized in that** it consists in applying, to said keratinous substances, a cosmetic composition as defined in one of Claims 1 to 33.

35. Method for the form retention of the hairstyle, cosmetic treatment, care, washing, makeup removal and/or making up, keratinous substances, especially the skin of the body or face, nails, hair, eyelashes, eyebrows, **characterized in that** it consists in applying, to said keratinous substances, a cosmetic composition as defined in one of Claims 1 to 37 or 41 to 44, then in optionally rinsing with water.

36. Cationic ethylenic polymer comprising at least one primary, secondary or tertiary amine functional group that can be protonated, said functional group being at least partially neutralized by a neutralizing agent chosen from polymeric organic acids comprising at least one carboxylic, sulphonic and/or phosphonic acid functional group, said at least partially neutralized polymer being able to be conveyed in an aqueous medium, that is to say being water-soluble or water-dispersible.

37. Polymer according to Claim 36, comprising at least one monomer of formula (I), alone or as a mixture: 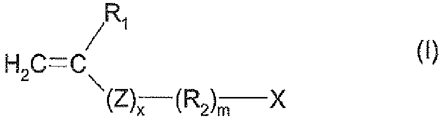 in which:
- R₁ is a hydrogen atom or a linear or branched, hydrocarbon-based radical of CₚH₂ₚ₊₁ type, with p being an integer between 1 and 12 inclusive;
- Z is a divalent group chosen from -COO-, -CONH-, -CONCH₃-, -OCO- or -O-, -SO₂-, -CO-O-CO-or -CO-CH₂-CO-; preferably, COO and CONH;
- x is 0 or 1, preferably 1;
- R₂ is a saturated or unsaturated, optionally aromatic, linear, branched or cyclic, carbon-based divalent radical having 1 to 30 carbon atoms, which may comprise 1 to 18 heteroatoms chosen from O, N, S, F, Cl, Br, Si and P;
- m is 0 or 1;
- X is
(a) a guanidino or amidino group, or else;
(b) a group of formula -N(R₆)(R₇) with R₆ and R₇ representing, independently of one another, (i) a hydrogen atom, (ii) a linear, branched or cyclic, saturated or unsaturated, optionally aromatic alkyl group comprising from 1 to 18 carbon atoms, which may comprise 1 to 10 heteroatoms chosen from O, N, S, F, Si and P; or (iii) R₆ and R₇ form, with the nitrogen atom, a ring of formula: 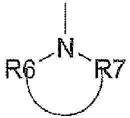 that is saturated or unsaturated, optionally aromatic, comprising in total 5, 6, 7, or 8 atoms, and especially 4, 5 or 6 carbon atoms and/or 2 to 4 heteroatoms chosen from O, S and N; said first ring possibly being fused with one or more other rings, which are saturated or unsaturated, optionally aromatic, each comprising 5, 6 or 7 atoms, and especially 4, 5, 6 or 7 carbon atoms and/or 2 to 4 heteroatoms chosen from O, S and N;
(c) a ring: 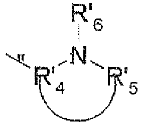
in which R'₄ and R'₅ form, with the nitrogen atom, a saturated or unsaturated, optionally aromatic, ring comprising in total 5, 6, 7 or 8 atoms, and especially 4, 5 or 6 carbon atoms and/or 2 to 4 heteroatoms chosen from O, S and N; said ring possibly being fused with one or more other rings, which are saturated or unsaturated, optionally aromatic, each comprising 5, 6 or 7 atoms, and especially 4, 5, 6, 7 or 8 carbon atoms and/or 2 to 4 heteroatoms chosen from O, S and N;
and R'₆ is chosen from H, -CH₃ and -C₂H₅;
being understood that said monomer of formula (I) comprises at least one primary, secondary or tertiary amine functional group which can be protonated at a pH chosen between pH 1 and pH 12; this functional group possibly being, or being borne by, the radicals R₂ and/or X.

38. Polymer according to one of Claims 36 to 37, in which the monomer of formula (I) is chosen from, alone or as a mixture, dimethylaminopropyl (meth)acrylamide, dimethylaminoethyl (meth)acrylamide, diethylaminoethyl (meth)acrylate, dimethylaminoethyl (meth)acrylate, tert-butylaminoethyl (meth)acrylate, morpholinoethyl (meth)acrylate, vinylimidazole, vinylpyridine, vinylamine, allylamine, and the monomers below in which R = H or methyl:

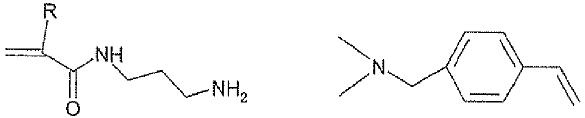

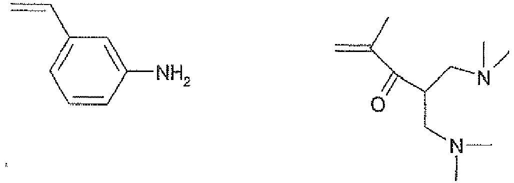

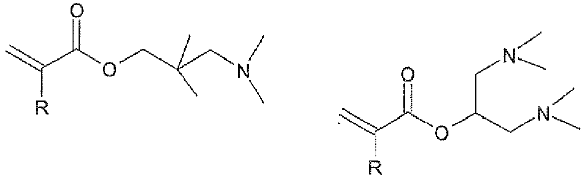

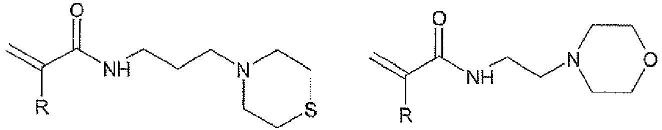

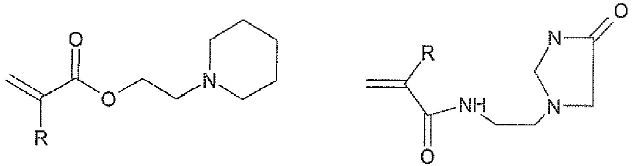

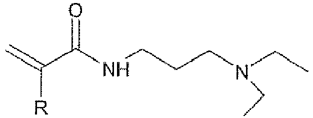

39. Polymer according to Claim 36, comprising at least one monomer of formula (II), alone or as a mixture: 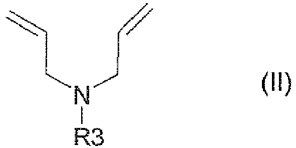
in which R₃ is hydrogen or a linear, branched or cyclic, saturated or unsaturated, optionally aromatic, carbon-based, especially hydrocarbon-based, radical having 1 to 30 carbon atoms, which may comprise 1 to 18 heteroatoms chosen from O, N, S, F, Cl, Br, Si and P,
being understood that the monomer of formula (II) comprises at least one primary, secondary or tertiary amine functional group which can be protonated at a pH chosen between pH 1 and pH 12.

40. Polymer according to Claim 39, in which the monomer of formula (II) is chosen from, alone or as a mixture, N-methyldiallylamine and diallylamine.

41. Polymer according to Claim 36, comprising at least one monomer of formula (III), alone or as a mixture: 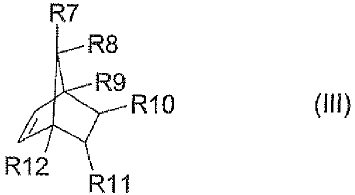 in which R₇ to R₁₂, independently of one another, represent:
- a hydrogen atom;
- an -NR₁₃R'₁₃ group with R₁₃ and R'₁₃, independently of one another, representing hydrogen or a linear, branched or cyclic, saturated or unsaturated, optionally aromatic alkyl comprising from 1 to 18 carbon atoms, which may comprise 1 to 10 heteroatoms chosen from O, N, S, F, Si and P; and in particular R₁₃ and R'₁₃ may be chosen from hydrogen, a methyl, ethyl, propyl, isopropyl, n-butyl, t-butyl, isobutyl, octyl, lauryl or stearyl group; and
- a linear, branched or cyclic, saturated or unsaturated, optionally aromatic, carbon-based, especially hydrocarbon-based, radical having 1 to 30 carbon atoms, possibly comprising 1 to 18 heteroatoms chosen from O, N, S, F, Si and P, being understood that the monomer of formula (III) comprises at least one primary, secondary or tertiary amine functional group that can be protonated at a pH chosen between pH 1 and pH 12.

42. Polymer according to Claim 41, in which the monomer of formula (III) is chosen from, alone or as a mixture:

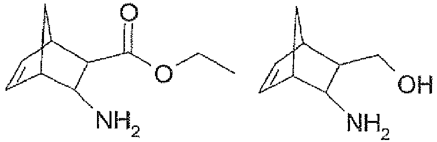

43. Polymer according to one of Claims 36 to 42 53, moreover comprising 0.01 to 99% by weight relative to the weight of the final polymer, especially 5 to 70% by weight, preferably 10 to 65% by weight, or even in an amount of 15 to 60% by weight, of additional monomers.

44. Polymer according to Claim 43, in which the additional monomer is chosen from the following monomers, alone or as a mixture, and also their salts:
- (i) (meth)acrylic acid esters of formula CH₂=CHCOOR'₁ or CH₂=C(CH₃)COOR'₁ with R'₁ representing a linear, branched or cyclic, saturated or unsaturated, optionally aromatic (aryl, aralkyl or alkylaryl) carbon-based, especially hydrocarbon-based (alkyl), chain having 1 to 30 carbon atoms, optionally comprising 0 to 2 ether functional groups (-O-) and optionally 0 to 12, especially 1 to 8, functional groups chosen from -OH (hydroxyl), -OR' with R' a C₁-C₆ alkyl (alkoxy), -CN, -X (halogen, especially Cl, F, Br or I), -CO-, -SO₃H, -COOH, -OCOO-, -COO-, -OCONR-, -OCONH-, -NH-CONH-, -NR-CONR-, -CF₃, -CN, epoxy, -NHCO-, -N(R)CO- with R = linear or branched C₁-C₂₂ alkyl optionally comprising 1-12 heteroatoms;
- (ii) (meth)acrylic acid amides of formula CH₂=CHCONR'₂R"₂ or CH₂=C(CH₃)CONR'₂R"₂, with R'₂, R"₂, being identical or different, representing hydrogen or a linear, branched or cyclic, saturated or unsaturated, optionally aromatic, carbon-based, especially hydrocarbon-based (alkyl), chain having 1 to 30 carbon atoms, optionally comprising 0 to 2 ether functional groups (-O-) and optionally 0 to 12, especially 1 to 8, functional groups chosen from -OH (hydroxyl), -OR' with R' a C₁-C₆ alkyl (alkoxy), -CN, -X (halogen, especially Cl, F, Br or I), -CO-, -SO₃H, -COOH, -OCOO-, -COO-, -OCONR-, -OCONH-, -NH-CONH-, -NR-CONR-, -CF₃, -CN, epoxy, -NHCO-, -N(R)CO- with R = linear or branched C₁-C₂₂ alkyl optionally comprising 1-12 heteroatoms;
- (iii) vinyl esters of formula CH₂=CH-OCO-R'₃ or CH₂=C(CH₃)-OCO-R'₃ with R'₃ representing a linear, branched or cyclic, saturated or unsaturated, optionally aromatic, carbon-based, especially hydrocarbon-based chain having 1 to 30 carbon atoms, optionally comprising 0 to 2 ether functional groups (-O-) and optionally 0 to 12, especially 1 to 8, functional groups chosen from -OH (hydroxyl), -OR' with R' a C₁-C₆ alkyl (alkoxy), -CN, -X (halogen, especially Cl, F, Br or I), -CO-, -SO₃H, -COOH, -OCOO-, -COO-, -OCONR-, -OCONH-, -NH-CONH-, -NR-CONR-, -CF₃, -CN, epoxy, -NHCO-, -N(R)CO- with R = linear or branched C₁-C₂₂ alkyl optionally comprising 1-12 heteroatoms;
- (iv) vinyl ethers of formula CH₂=CHOR'₄ or CH₂=C(CH₃)OR'₄ with R'₄ representing a linear, branched or cyclic, saturated or unsaturated, optionally aromatic, carbon-based, especially hydrocarbon-based, chain having 1 to 30 carbon atoms, optionally comprising 0 to 2 ether functional groups (-O-) and optionally 0 to 12, especially 1 to 8, functional groups chosen from -OH (hydroxyl), -OR' with R' a C₁-C₆ alkyl (alkoxy), -CN, -X (halogen, especially Cl, F, Br or I), -CO-, -SO₃H, -COOH, -OCOO-, -COO-, -OCONR-, -OCONH-, -NH-CONH-, -NR-CONR-, -CF₃, -CN, epoxy, -NHCO-, -N(R)CO- with R = linear or branched C₁-C₂₂ alkyl optionally comprising 1-12 heteroatoms;
- (v) vinyl compounds of formula CHR"₅=CR₅R'₅ in which:
R"₅ is H or COOH, and
R₅ is H, CN or COOH, and
R'₅ is chosen from:
- a hydrogen atom, or a group chosen from -OH, -CH=O, halogen (Cl, Br, I in particular), -COOH, -CH₂COOH, -NHC(O)H, -N(CH₃)-C(O)H, -NHC(O)CH₃, -N(CH₃)-C(O)CH₃;
- a ring: 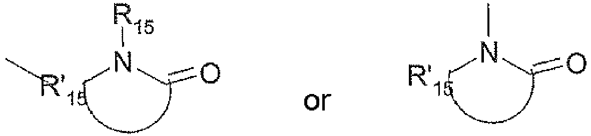 where R'₁₅ and R₁₅ represent, independently of one another, H, a linear or branched, saturated or unsaturated, optionally aromatic, cyclic or non-cyclic alkyl group, comprising 1 to 25 carbon atoms, optionally inserted into which are one or more heteroatoms chosen from O, N, S and P; said alkyl group possibly, moreover, being optionally substituted by one or more substituents chosen from -OH and halogen atoms (Cl, Br, I and F);
- a linear or branched alkyl group comprising 1 to 25 carbon atoms;
- a linear or branched alkyl group comprising 1 to 25 carbon atoms;
- a C₃ to C₈ cycloalkyl group such as cyclohexane;
- a C₆ to C₂₀ aryl group such as phenyl;
- a C₇ to C₃₀ aralkyl group (C₁ to C₄ alkyl group) such as 2-phenylethyl or benzyl;
- a heterocyclic group having 4 to 12 chain members containing one or more heteroatoms chosen from O, N and S; and
- a heterocycloalkyl group (alkyl having 1 to 4 carbons), such as furfuryl, furfurylmethyl or tetrahydrofurfurylmethyl,
said alkyl, cycloalkyl, aryl, aralkyl, heterocyclic or heterocycloalkyl groups possibly optionally comprising 0 to 2 ether functional groups (-O-) and optionally 0 to 12, especially 1 to 8, functional groups chosen from -OH (hydroxyl), -OR' with R' a C₁-C₆ alkyl (alkoxy), -CN, -X (halogen, especially Cl, F, Br or I), -CO-, -SO₃H, -COOH, -OCOO-, -COO-,-OCONR-, -OCONH-, -NH-CONH-, -NR-CONR-, -CF₃, -CN, epoxy, -NHCO-, -N(R)CO- with R = linear or branched C₁-C₂₂ alkyl optionally comprising 1-12 heteroatoms;
and/or possibly being optionally substituted by one or more linear or branched, C₁-C₄ alkyl groups, themselves optionally comprising 0 to 2 ether functional groups (-O-) and optionally 0 to 12, especially 1 to 8, functional groups chosen from -OH (hydroxyl), -OR' with R' a C₁-C₆ alkyl (alkoxy), -CN, -X (halogen, especially Cl, F, Br or I), -CO-, -SO₃H,-COOH, -OCOO-, -COO-, -OCONH-, -NH-CONH-, -CF₃, -CN, epoxy, -NHCO-, -N(R)CO- with R = linear or branched C₁-C₂₂ alkyl optionally comprising 1-12 heteroatoms,
- (vii) the following anionic monomers, and salts thereof:
maleic anhydride, acrylic acid, methacrylic acid, crotonic acid, itaconic acid, fumaric acid, maleic acid, 2-carboxyethyl acrylate (CH₂=CH-C(O)-O-(CH₂)₂-COOH); styrenesulphonic acid, 2-acrylamido-2-methylpropanesulphonic acid, vinylbenzoic acid, vinylphosphonic acid, sulphopropyl (meth)acrylate, and among the salts: sodium or potassium (meth)acrylate;
- (viii) the following amphoteric monomers:
N,N-dimethyl-N-(2-methacryloyloxyethyl)-N-(3-sulphopropyl)ammonium betaine (especially SPE from Raschig); N,N-dimethyl-N-(3-methacrylamidopropyl)-N-(3-sulphopropyl)ammonium betaine (SPP from Raschig), and 1-(3-sulphopropyl)-2-vinylpyridinium betaine (SPV from Raschig), and also 2-(methacryloyloxy)ethyl phosphorylcholine;
- (ix) monomers of formula: 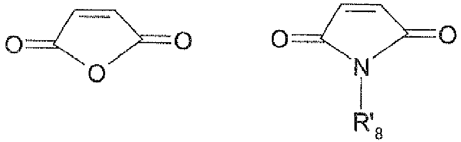 in which R'₈ is H or a linear or branched, saturated or unsaturated, optionally aromatic, cyclic or non-cyclic alkyl group comprising 1 to 25 carbon atoms, optionally inserted into which are one or more heteroatoms chosen from O, N, S and P; said alkyl group possibly, moreover, being optionally substituted by one or more substituents chosen from -OH and halogen atoms (Cl, Br, I and F);
- (x) quaternized monomers of formula (I), (II) and (III), and the quaternized forms of the additional monomers above;
- (xi) multivalent compounds comprising at least two polymerizable functional groups of the vinyl, (meth)acrylic, allyl or (meth)acrylamide type.

45. Polymer according to Claim 44, in which the additional monomer is chosen from vinyl neodecanoate, vinyl *tert*-butylbenzoate; vinylpyrrolidone; vinylcaprolactam; N-vinylformamide; N,N'-dimethyldiallylammonium chloride; triethylammonium ethylmethacrylate chloride (MADQUAT); ethyl, methyl, *tert*-butyl or isobornyl (meth)acrylates; vinyl acetate, crotonic acid; (meth)acrylic acid, methacryloyl ethyl betaine, octylacrylamide; N-methyl-N-vinylimidazolinium chloride, 1-eicosene, tert-butylacrylamide, acrylamide, hexadecene, and mixtures thereof.

46. Polymer according to one of Claims 36 to 45, chosen from:
- poly(vinylpyrrolidone-co-dimethylaminoethyl methacrylate) copolymers;
- poly(vinylcaprolactam-co-vinylpyrrolidone-co-dimethylaminoethyl methacrylate) copolymers;
- poly(vinylpyrrolidone-co-dimethylaminopropyl methacrylamide) copolymers;
- poly(vinylpyrrolidone-co-polyvinylcaprolactam-co-dimethylaminopropyl methacrylamide) copolymers;
- poly(vinylamine), poly(allylamine), poly-(diallylamine); and
- poly(N-vinylformamide-co-vinylamine) copolymers.

47. Polymer according to Claim 36, comprising at least 3 repeating units chosen, alone or as mixtures, from:

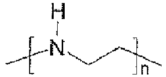

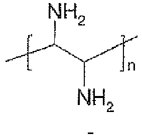

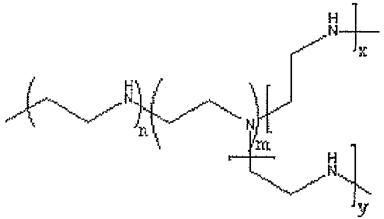

48. Polymer according to one of Claims 36 to 47, in which the neutralizing agent is added in an amount of 0.01 to 3 molar equivalents, especially 0.05 to 2.5, or even 0.1 to 2 molar equivalents, relative to the total amine functional groups of the polymer or of the monomers.

49. Polymer according to one of Claims 36 to 48, in which the neutralizing agent is chosen from:
- (i) polymers consisting of dextran repeating units comprising at least one acid, especially carboxylic acid, functional group as a chain end;
- (ii) acid-terminated poly(alkyloxazolines), preferably poly(methyloxazoline) and poly(ethyl-oxazoline) comprising at least one acid functional group;
- (iii) acid, especially carboxylic acid, functionalized poly(N-methylsarcosines);
- (iv) dendrimers or hyperbranched molecules that are functionalized at the surface with acid, especially carboxylic acid, groups; such as for example the starburst PAMAM dendrimers from Dow Chemical;
- (v) hyperbranched polymers of the polyester type obtained by reaction between adipic acid and glycerol;
- (vi) acid, especially carboxylic acid, terminated poly(glycerol), poly(hydroxyethyl acrylate) and poly(vinylpyrrolidone);
- (vii) polymers comprising at least three alkylene glycol type repeating units for which the alkyl group comprises 2 to 4 carbon atoms, and combinations thereof; mention may be made of polyethylene glycol, polypropylene glycol, polyethylene-co-propylene glycol (PEG/PPO), polytetramethylene oxide-co-polyethylene glycol (PTMO/PEG); being understood that they furthermore comprise at least one acid (especially carboxylic, sulphonic and/or phosphonic acid) functional group.

50. Polymer according to one of Claims 36 to 49, in which the neutralizing agent is a polymer which comprises at least three alkylene glycol type repeating units, preferably of ethylene glycol type: (-CH₂-CH₂-O-).

51. Polymer according to one of Claims 36 to 50, in which the neutralizing agent is chosen from polymers having the following structures:
CH₃-O-(CH₂-CH₂-O-)ₙ-CO-(CH₂-CHR-O)ₘ-CH₂-R₁₀
CH₃-O-(CH₂-CH₂-O-)ₙ-b-(CH₂-CHR-O)ₘ-CH₂-R₁₀
CH₃-O-(CH₂-CH₂-O-)ₙ-CO-(CH₂-CHR-O)ₘ-CH₂-CH₂ R₁₀
CH₃-O-(CH₂-CH₂-O-)ₙ-b-(CH₂-CHR-O)ₘ-CH₂-CH₂ R₁₀
CH₃-O-(CH₂-CH₂-O-)ₙ-CO-(CH₂-CHR-O)ₘ-CH₂-CH₂CH₂ R₁₀
CH₃-O-(CH₂-CH₂-O-)ₙ-b-(CH₂-CHR-O)ₘ-CH₂-CH₂CH₂ R₁₀
CH₃-O-(CH₂-CH₂-O-)ₙ-CO-(CH₂-CHR-O)ₘ-CH₂-CH₂OCO-CH₂-CH₂COOH
CH₃-O-(CH₂-CH₂-O-)ₙ-b-(CH₂-CHR-O)ₘ-CH₂-CH₂OCO-CH₂-CH₂COOH
R₁₀-(CH₂-CH₂-O-)ₙ-CO-(CH₂-CHR-O)ₘ-CH₂-CH₂ R₁₀
R₁₀-(CH₂-CH₂-O-)ₙ-b-(CH₂-CHR-O)ₘ-CH₂-CH₂ R₁₀
R₁₀-CH₂O-(CH₂-CH₂-O-)ₙ-CO-(CH₂-CHR-O)ₘ-CH₂-R₁₀
R₁₀-CH₂O-(CH₂-CH₂-O-)ₙ-b-(CH₂-CHR-O)ₘ-CH₂-R₁₀
R₁₀-CH₂-CH₂-CH₂-O-(CH₂-CH₂-O-)ₙ-CO-(CH₂-CHR-O)ₘ-CH₂-CH₂-CH₂-R₁₀
R₁₀-CH₂-CH₂-CH₂-O-(CH₂-CH₂-O-)ₙ-b-(CH₂-CHR-O)ₘ-CH₂-CH₂-CH₂-R₁₀
R₁₀-CH₂-CH₂-COO-(CH₂-CH₂-O-)ₙ-CO-(CH₂-CHR-O)ₘ-CH₂-CH₂-O-CO-CH₂-CH₂-R₁₀
R₁₀-CH₂-CH₂-COO-(CH₂-CH₂-O-)ₙ-b-(CH₂-CHR-O)ₘ-CH₂-CH₂-O-CO-CH₂-CH₂-R₁₀
R₁₀-CH₂-CH₂-CONH-(CH₂-CH₂-O-)ₙ-CO-(CH₂-CHR-O)ₘ-CH₂-CH₂-NH-CO-CH₂-CH₂-R₁₀
R₁₀-CH₂-CH₂-CONH-(CH₂-CH₂-O-)ₙ-b-(CH₂-CHR-O)ₘ-CH₂-CH₂-NH-CO-CH₂-CH₂-R₁₀
in which:
- the R radical, being identical or different, represents -CH₃, -(CH₂)ₓ-COOH; -(CH₂)ₓ-SO₃H; (CH₂)ₓ-PO₃H₂; with x = 1 to 8;
- the R₁₀ radical, being identical or different, is -COOH, -SO₃H or -PO₃H₂;
- n is an integer between 3 and 1000;
- m is an integer between 0 and 200,
given that the n/m ratio is greater than 5, when m is non zero.

52. Polymer according to one of Claims 36 to 51, in which the neutralizing agent is chosen from:
CH₃-O-(CH₂-CH₂-O-)ₙ-CH₂-CH₂ R₁₀
CH₃-O-(CH₂-CH₂-O-)ₙ-CH₂-CH₂CH₂ R₁₀
R₁₀-(CH₂-CH₂-O-)ₙ-CH₂-CH₂ R₁₀
R₁₀-CH₂O-(CH₂-CH₂-O-)n-CH₂-R₁₀
in which the R₁₀ radical is -COOH, -SO₃H or -PO₃H₂; preferably COOH; and n is 3 to 1000, especially 4 to 30, or even 5 to 15.
